# EUROPEAN PATENT SPECIFICATION

(11) **EP 3 426 241 B1**
(45) Date of publication and mention of the grant of the patent: **12.06.2024**
(21) Application number: 17763668.5
(22) Date of filing: 08.03.2017
(51) Int. Cl.: A61K 31/216, G01N 33/574, G01N 33/68, A23L 33/175, A61P 35/00, A61K 31/195, A61K 31/198, A61P 3/00

(54) **METHODS OF DIAGNOSING CANCER**
VERFAHREN ZUR DIAGNOSE VON KREBS
PROCÉDÉS DE DIAGNOSTIC DU CANCER

(30) Priority: 08.03.2016 SG 10201601791W
(43) Date of publication of application: 16.01.2019
(60) Divisional application: 24174387.1
(73) Proprietor: Agency for Science, Technology and Research, Singapore 138632 (SG)
(72) Inventor: HAN, Weiping, Singapore 138667 (SG); ERICKSEN, Russell E., Singapore 138667 (SG)
(74) Representative: Regimbeau
(86) International application number: PCT/SG2017/050112
(87) International publication number: WO 2017/155467

(56) References cited:
- EP-A1- 2 249 161
- WO-A1-2016/201380
- US-A1- 2008 274 556
- JOON-HO SHEEN ET AL: "Defective Regulation of Autophagy upon Leucine Deprivation Reveals a Targetable Liability of Human Melanoma Cells In?Vitro and In?Vivo", CANCER CELL, vol. 19, no. 5, 1 May 2011 (2011-05-01), pages 613-628, XP055238570, US ISSN: 1535-6108, DOI: 10.1016/j.ccr.2011.03.012
- TETSURO NISHIHIRA ET AL: "Anti-cancer therapy with valine-depleted amino acid imbalance solution.", TOHOKU JOURNAL OF EXPERIMENTAL MEDICINE., vol. 156, no. 3, 1 January 1988 (1988-01-01), pages 259-270, XP055623153, JP ISSN: 0040-8727, DOI: 10.1620/tjem.156.259
- XUEYING SUN ET AL: "Branched chain amino acid imbalance selectively inhibits the growth of gastric carcinoma cells in vitro", NUTRITION RESEARCH., vol. 23, no. 9, 1 September 2003 (2003-09-01), pages 1279-1290, XP055623088, XX ISSN: 0271-5317, DOI: 10.1016/S0271-5317(03)00128-3
- BARACOS V E ET AL: "Investigations Of Branched-Chain Amino Acids And Their Metabolites In Animal Models Of Cancer", THE JOURNAL OF NUTRITION, AMERICAN SOCIETY FOR NUTRITION, US, vol. 136, no. 1, 1 January 2006 (2006-01-01), pages 237S-247S, XP003031221, ISSN: 0022-3166
- LIAN K. et al.: "Impaired Adiponectin Signaling Contributes to Disturbed Catabolism of Branched-Chain Amino Acids in Diabetic Mice", Diabetes, vol. 64, no. 1 28 July 2014 (2014-07-28), pages 49-59, XP055414676, [retrieved on 2017-05-30]
- ROSENSON R.S: "Effect of Fenofibrate on Adiponectin and Inflammatory Biomarkers in Metabolic Syndrome Patients", Obesity, vol. 17, no. 3 20 November 2008 (2008-11-20), pages 504-509, XP055414677, [retrieved on 2017-05-30]
- TSO S-C. et al.: "Structure-based design and mechanisms of allosteric inhibitors for mitochondrial branched-chain a-ketoacid dehydrogenase kinase", Proc Natl Acad Sci, vol. 110, no. 24 28 May 2013 (2013-05-28), pages 9728-9733, XP055414680, [retrieved on 2017-05-30]
- BURRAGE L.C. et al.: "Branched-chain amino acid metabolism: from rare Mendelian diseases to more common disorders", Human Molecular Genetics, vol. 23, no. R1 20 March 2014 (2014-03-20), pages R1-R8, XP055304583, [retrieved on 2017-05-30]
- AHMED K. et al.: "Structural and functional studies of novel small-molecule inhibitors to mitochondrial branched-chain a-ketoacid dehydrogenase kinase", Journal of Investigative Medicine, vol. 60, no. 1 January 2012 (2012-01), pages 265-311, ISSN: 1081-5589 [retrieved on 2017-05-30]
- WILK A. et al.: "Molecular Mechanisms of Fenofibrate-Induced Metabolic Catastrophe and Glioblastoma Cell death", Mol Cell Bio, vol. 35, no. 1 20 October 2014 (2014-10-20), pages 182-198, XP055414691, [retrieved on 2017-05-30]
- PANIGRAHY D. et al.: "PPARa agonist fenofibrate suppresses tumor growth through direct and indirect angiogenesis inhibition", Proc Natl Acad Sci, vol. 105, no. 3 16 January 2008 (2008-01-16), pages 985-990, XP055274295, [retrieved on 2017-05-30]
- KIM E.-S. et al.: "Effect of oncogene activating mutations and kinase inhibitors on amino acid metabolism of human isogenic breast cancer cells", Mol. BioSyst., vol. 11, no. 12 7 October 2015 (2015-10-07), pages 3378-3386, XP055414693, [retrieved on 2017-05-30]
- GRABACKA M. et al.: "Peroxisome Proliferator Activated Receptor A Ligands as Anticancer Drugs Targeting Mitochondrial Metabolism", Curr Pharm Biotechnol, vol. 14, no. 3 1 March 2013 (2013-03-01), pages 342-356, XP055414694, Retrieved from the Internet: URL:https://www.ncbi.nlm.nih.gov/pmc/artic les/PMC3631438 [retrieved on 2017-05-30]
- CHEN J. et al.: "Effects of different activators of peroxisome roliferator- activated receptors alpha on the expressions of plasminogen activator inhibitor- 1 in human hepatoma cells", Chinese Journal of Clinical Rehabilitation, vol. 9, no. 31 21 August 2005 (2005-08-21), pages 112-115, XP009512525, ISSN: 1671-5926 [retrieved on 2017-05-30]
- LACHAL S. et al.: "PPARa agonists stimulate progastrin production in human colorectal carcinoma cells", Regulatory Peptides, vol. 120, no. 1-3 15 August 2004 (2004-08-15), pages 243-251, XP055414697, [retrieved on 2017-05-30]
- CHANG H. et al.: "Anti-proliferative mechanism of fenofibrate against human gastric tumor MGC803 cells", Chin Hosp Pharm J., vol. 35, no. 8 April 2015 (2015-04), pages 730-762, XP009512466, ISSN: 1001-5213 [retrieved on 2017-05-30]
- ERICKSEN RUSSELL E. ET AL: "Loss of BCAA Catabolism during Carcinogenesis Enhances mTORC1 Activity and Promotes Tumor Development and Progression", CELL METABOLISM, vol. 29, no. 5, 1 May 2019 (2019-05-01), pages 1151-1165.e6, XP055797611, United States ISSN: 1550-4131, DOI: 10.1016/j.cmet.2018.12.020 Retrieved from the Internet: URL:https://www.ncbi.nlm.nih.gov/pmc/artic les/PMC6506390/pdf/nihms-1517776.pdf>

## Description

### CROSS-REFERENCE TO RELATED APPLICATIONS

This application claims the benefit of priority of Singapore provisional application No. 10201601791W, filed 8 March 2016, the contents of it being hereby incorporated by reference in its entirety for all purposes.

### FIELD OF THE INVENTION

The present invention relates to biochemistry in particular biomarkers. In particular, the present invention relates to biomarkers associated with cancer and methods of using the biomarkers to determine the likelihood that a patient suffers from proliferative diseases.

### BACKGROUND OF THE INVENTION

Proliferative diseases can develop in any tissue of any organ at any age. Proliferative diseases are cellular malignancies whose unique trait-loss of normal control mechanisms-results in unregulated growth, lack of differentiation, and ability to invade local tissues and metastasize. Thus cells afflicted with proliferative diseases are unlike normal cells, and are potentially identifiable by not only their phenotypic traits, but also by their biochemical and molecular biological characteristics. In particular, the altered phenotype of cells afflicted with proliferative diseases indicates altered gene activity, either unusual gene expression, or gene regulation. Identification of gene expression products or proteins associated with cells afflicted with proliferative diseases will allow for the molecular characterization of malignancies. The ability to specifically characterize suspected proliferative diseases, and to potentially identify not only cell type, but also predisposition for metastasis and any sensitivity to particular anti-cancer therapy, is most useful for determining not only the course of treatment, but also the likelihood of success. Additionally, most proliferative diseases that are detected at an early stage are potentially curable. Whilst there are multiple different methods available in the market for the diagnosis of proliferative diseases, some proliferative diseases are still not detected and thus may progress to an incurable state. Therefore, there is a need to provide an alternative method to diagnose, to make a prognosis, and/or to treat proliferative diseases. Nishihira, T. et al. (1988), TOHOKU JOURNAL OF EXPERIMENTAL MEDICINE., vol. 156, no. 3, 259-270 discloses an anti-cancer therapy with valine-depleted amino acid imbalance solution.

Baracos, V.E. et al. (2006), The Journal of Nutrition, vol. 136, no. 1, p. 2375-2475 investigates branched-chain amino acids and their metabolites in animal models of cancer.

### SUMMARY OF THE INVENTION

The invention in its broadest form is as defined in independent claim 1. Preferred embodiments are defined in claims 2-4.

### Summary of the disclosure

In one aspect, the present disclosure refers to a method of treating or preventing a proliferative disease in a subject in need thereof, wherein the proliferative disease is characterized and/or diagnosed by at least one selected from the group consisting of an accumulation of at least one branched-chain amino acid (BCAA); a suppression of enzyme activity involved in the catabolism of at least one branched-chain amino acid (BCAA); a suppression of transcripts-level of enzymes involved in the catabolism of at least one branched-chain amino acid (BCAA); and a decrease in a level of an acylcarnitine (C5:1); wherein said method comprises administering a branched-chain amino acid catabolism enhancer and/or a branched-chain α-ketoacid dehydrogenase complex (BCKDC) kinase inhibitor (BDK inhibitor).

In another aspect, the present disclosure refers to a method of treating or preventing a proliferative disease in a subject in need thereof, wherein the proliferative disease is characterized and/or diagnosed by at least one selected from the group consisting of an accumulation of at least one branched-chain amino acid (BCAA); a suppression of enzyme activity involved in the catabolism of at least one branched-chain amino acid (BCAA), a suppression of transcripts involved in the catabolism of at least one branched-chain amino acid (BCAA); and a decrease in a level of an acylcarnitine (C5:1); wherein said method comprises administering a meal replacement comprising low level of branched-chain amino acid (BCAA) into the subject in need thereof.

In yet another aspect, the present disclosure refers to a method of determining or predicting whether a subject is having or likely to have a proliferative disease, the method comprising a. measuring a level of at least one branched-chain amino acid (BCAA) of the subject; and b. comparing the branched-chain amino acid level of the subject to the branched-chain amino acid level of a control subject or subjects not having said proliferative disease, wherein the branched-chain amino acid level in excess of the branched-chain amino acid level of the control subject indicates the subject is having or is likely to have the proliferative disease.

In a further aspect, the present disclosure refers to a method of predicting the likelihood of a subject surviving proliferative disease comprising a. measuring a level of branched amino acids (BCAA) of the subject; b. comparing and/or correlating the level measured in (a) to a standard level of branched amino acids, wherein the degree of deviation above the level of the standard indicates the degree of severity of the outcome.

In another aspect, the present disclosure refers to a method of determining or predicting whether a subject is having or likely to have a proliferative disease, the method comprising a. measuring a level of branched amino acids (BCAA) catabolic enzymes of the subject; and b. comparing the level measured in (a) to the level of a branched amino acids (BCAA) catabolic enzymes of a control subject (or subjects) not having said proliferative disease, wherein a decreased branched amino acids (BCAA) catabolic enzymes level as compared to the branched amino acids (BCAA) catabolic enzymes level of the control subject indicates the subject is having or likely to have the proliferative disease.

In a further aspect, the present disclosure refers to a method of predicting the likelihood of a subject surviving proliferative disease comprising a. measuring a level of branched amino acids (BCAA) catabolic enzymes of the subject; b. comparing and/or correlating the level measured in (a) to a standard level of branched amino acids catabolic enzymes, wherein the degree of deviation above the level of the standard indicates the degree of severity of the outcome.

In another aspect, the present disclosure refers to a method of determining or predicting whether a subject is having or likely to have a proliferative disease comprising a. measuring a level of an acylcarnitine (C5:1) of the subject; and b. comparing the acylcarnitine (C5:1) level of the subject as compared to the acylcarnitine (C5:1) level of a control subject or subjects not having said proliferative disease, wherein a decrease in the acylcarnitine (C5:1) level as compared to the level of the control subject indicates the subject is having or is likely to have the proliferative disease.

In a further aspect, the present disclosure refers to a kit or microarray chip for use in any of the methods as defined above, the kit or microarray chip comprising a. a reagent or a group of reagents for measuring a level of at least one selected from the group consisting of acylcarnitine (C5:1), branched-chain amino acid (BCAA), and a level of at least one branched-chain amino acid (BCAA) catabolic enzyme in the subject; b. a reagent or a group of reagents comprising a pre-determined level of at least one selected from the group consisting of acylcarnitine (C5:1), branched-chain amino acid (BCAA), and branched-chain amino acid (BCAA) catabolic enzyme, c. optionally instructions for using the reagent in (a) and (b) to determine or predict whether a subject has or likely to have proliferative disease, wherein the pre-determined level is determined by measured level of at least one acylcarnitine (C5:1) and/or branched-chain amino acid and/or branched-chain amino acid (BCAA) catabolic enzyme in a control subject(s) not having the proliferative disease, and/or to determine the prognosis of the subject.

### BRIEF DESCRIPTION OF THE DRAWINGS

The invention will be better understood with reference to the detailed description when considered in conjunction with the non-limiting examples and the accompanying drawings, in which:

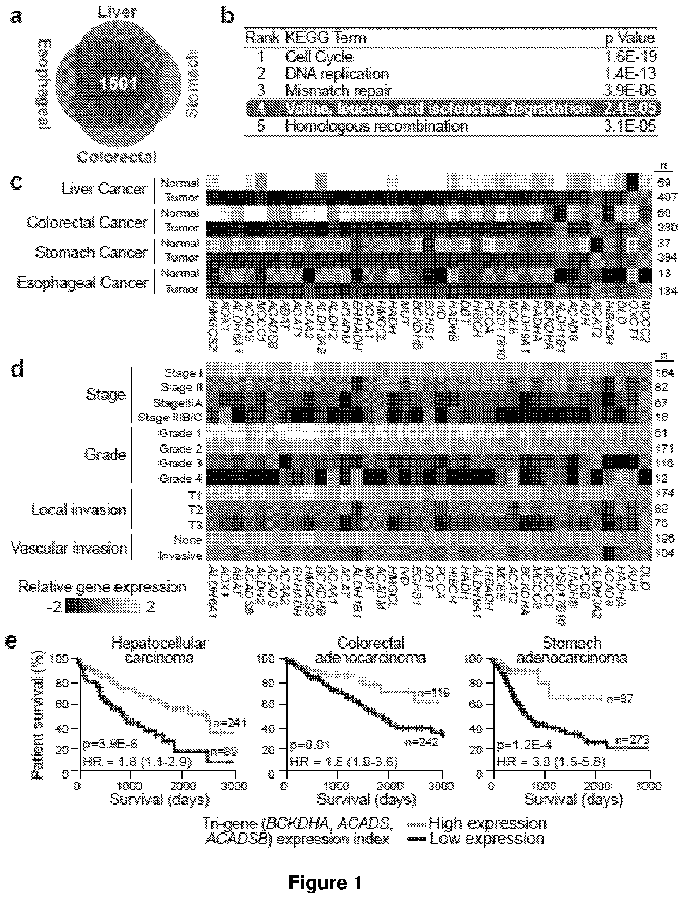

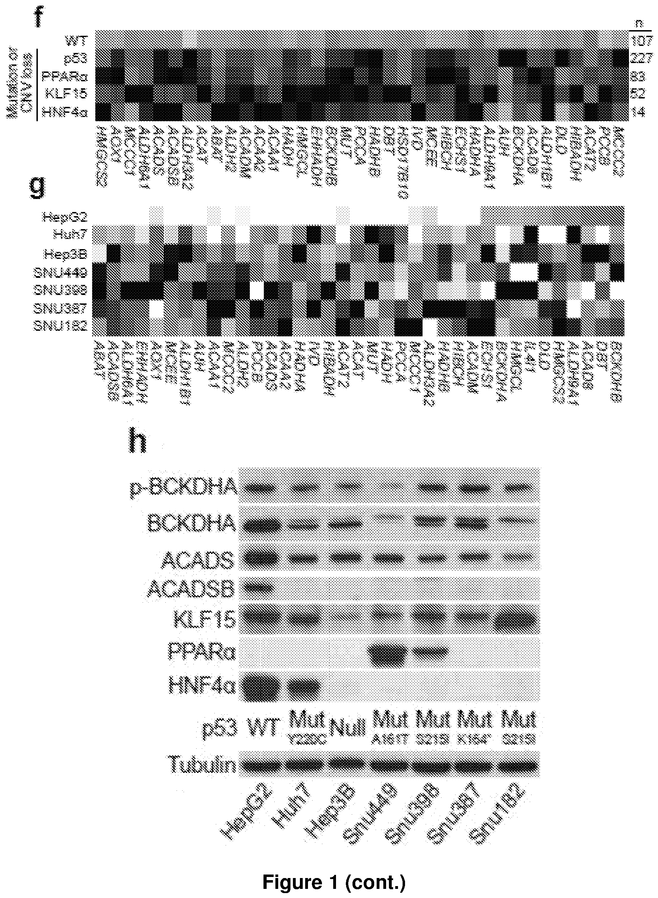

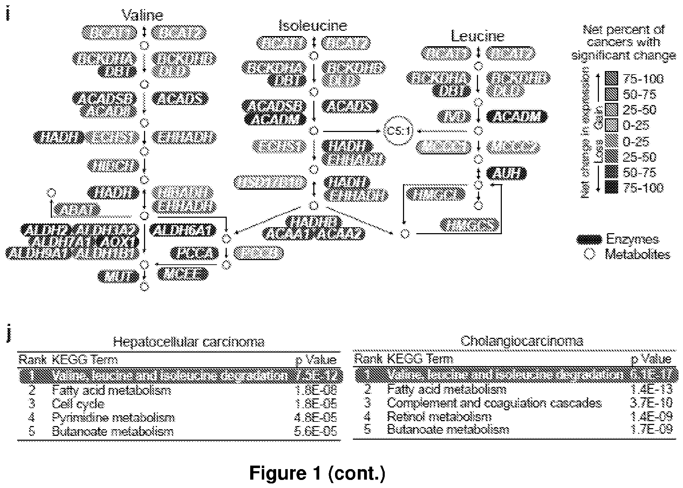

**Figure 1** shows a set of images detailing the comprehensive transcriptomic analyses of human gastrointestinal cancers and cell lines identifying loss of branched-chain amino acid (BCAA) catabolism in tumor development and progression. **Figure 1a** shows a Venn diagram depicting the summary of genes differentially expressed in primary tumors compared to solid normal tissues, including 1082 significantly upregulated and 419 significantly downregulated in all four gastrointestinal cancers. **Figure 1b** shows a table listing the result of Kyoto Encyclopedia of Genes and Genomes (KEGG) pathway analysis of the 1501 gene set. **Figure 1c** shows a diagram depicting the summary of branched-chain amino acid catabolic enzyme transcript levels across the four gastrointestinal cancers and solid normal tissues. The results show that tumors across all four cancers have significantly lower expression levels of all the indicated genes encoding enzymes in the branched-chain amino acid (BCAA) degradation pathways. **Figure 1d** shows a diagram depicting the summary of branched-chain amino acid (BCAA) catabolic enzyme transcript levels in hepatocellular carcinoma tumors sorted by stage, grade, vascular invasion, primary tumor size and local invasion (T score). The results show that more aggressive tumors, i.e. those of higher grades, higher stages, with more significant local invasions or with vascular invasions, have significantly lower expression levels of all the indicated genes encoding enzymes in the branched-chain amino acid (BCAA) degradation pathways. **Figure 1e** shows a line graph depicting Kaplan-Meier survival estimate curves for patients ranked by a combined index of tumor *BCKDHA*, *ACADS*, and *ACADSB* expression. P-values for log-rank tests and cox proportional hazard ratios (HR, with 95% percent confidence intervals) adjusted for age, sex, tumor stage and grade, and radiation, prescription and additional therapies shown. **Figure 1f** shows a diagram depicting the summary of branched-chain amino acid (BCAA) catabolic enzyme transcript levels in tumors with indicated transcription factor mutation or copy number variation (CNV) loss. The results show that the expression levels of almost all the indicated genes encoding enzymes in the branched-chain amino acid (BCAA) degradation pathways are lower in tumors with mutation or CNV loss of p53, PPARα, KLF15 or HNF4α. **Figure 1g** shows a diagram depicting the summary of branched-chain amino acid (BCAA) enzyme expression in nontumorigenic (HepG2) and tumorigenic (remaining) liver cell lines. The results show that the expression levels of almost all the indicated genes encoding enzymes in the branched-chain amino acid (BCAA) degradation pathways are lower across a range of cancer cell lines. **Figure 1h** shows a set of Western blots depicting protein expression of selected catabolic enzymes and transcription factors across the liver cell lines. **Figure 1i** shows a flowchart depicting summary of changes in branched-chain amino acid (BCAA) catabolic enzyme transcript levels across all cancers profiled by The Cancer Genome Atlas (TCGA) with at least 5 solid normal tissue samples. **Figure 1j** shows a table depicting KEGG pathway analysis of all significant differentially expressed genes (Bonferroni corrected) in human hepatocellular carcinoma (HCC) and cholangiocarcinoma compared to respective solid normal tissues. Thus, **Figure 1** shows cells afflicted by proliferative diseases have lower expression/levels of BCAA catabolic enzymes, and that the degree of expression loss is positively correlated with diseases severity and poor prognosis.

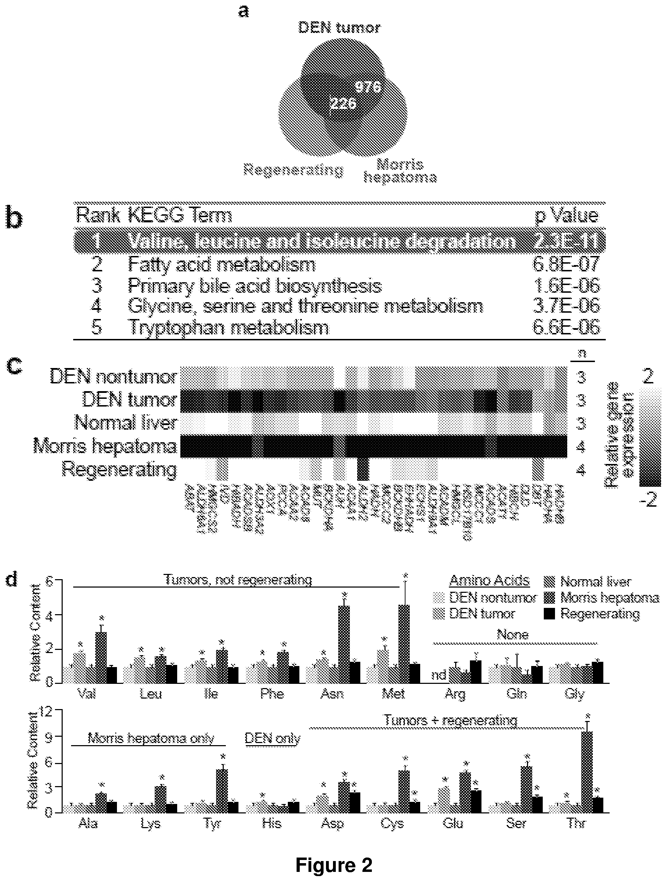

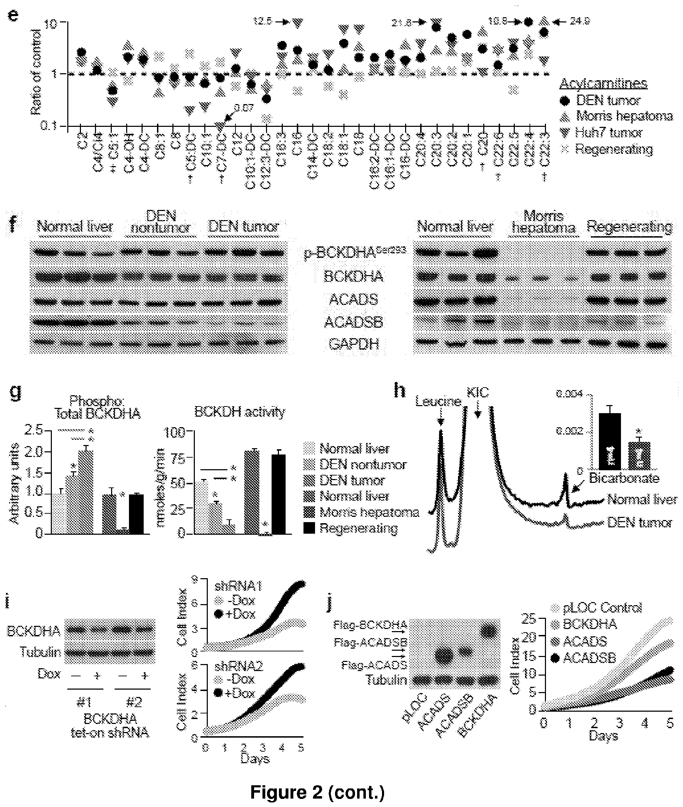

**Figure 2** shows a set of images depicting that tumors but not regenerating tissues, accumulate branched-chain amino acids (BCAA) due to loss of catabolic enzyme activity based on characterizations of animal tumor models. DEN-induced tumors are compared to DEN nontumor tissue, and orthotopic Morris Hepatoma tumors and regenerating liver tissue after partial hepatectomy are compared to normal liver tissue. **Figure 2a** shows a Venn diagram depicting the summary of transcriptomic analyses. **Figure 2b** shows a table listing KEGG pathway analysis of the 976 genes differentially expressed in tumors but not regenerating tissues. **Figure 2c** shows a diagram depicting summary of branched-chain amino acid (BCAA) catabolic enzyme transcript levels across normal, tumor, and regenerating tissues. The expression levels of all the indicated genes encoding enzymes in the BCAA degradation pathways are lower in DEN tumors when compared with DEN nontumor, in Morris hepatoma when compared with normal liver, but they are not reduced in regenerating liver samples. **Figure 2d** shows a bar graph depicting the quantification of tissue amino acid content, normalized to respective controls. **Figure 2e** shows a scatter plot depicting quantification of tissue acylcarnitine content, normalized to respective control. Huh7 tumors (n=3) are compared to normal mouse liver tissue (n=3). Only acylcarnitines that trended in the same direction in all 3 tumor models are shown. **Figure 2f** shows a set of Western blots of enzymes involved in branched-chain amino acid catabolism in mouse (left) and rat (right) liver tissues. **Figure 2g** shows a set of bar graphs depicting quantification of phospho to total BCKDHA ratio and corresponding tissue BCKDH complex enzymatic activity as assessed by ex vivo α-ketoisovalerate catabolism. **Figure 2h** shows a set of spectra depicting representative liver MRS spectra after intravenous injection of hyperpolarized [1-C¹³]α-ketoisocaproate (KIC) and a bar graph depicting quantification of the bicarbonate peak of over 30 seconds. Baselines are shifted to display the difference in bicarbonate peak. Quantification of relative bicarbonate levels over a 30 second interval is shown in the inset. **Figure 2i** shows a set of photographs and a set of line graphs depicting characterization of AML12 cells expressing tet-inducible BCKDHA shRNAs, including immunoblots confirming knockdown, and real-time proliferation curves. **Figure 2j** shows a set of photographs and a line graph depicting characterization of Hep3B cells overexpressing Flag-tagged BCKDHA, ACADS, or ACADSB, including immunoblots confirming overexpression, and real-time proliferation curves. Student's t-test, *P<0.05, compared to respective controls; †P<0.05, in 2 of 3 tumor models, ‡P<0.05 in all 3 tumor models, compared to respective controls, while not significantly different in regenerating tissue; nd=not detected. Data are shown as mean ± s.e. Thus, **Figure 2** shows that suppression of catabolic enzymes to enhance branched-chain amino acid (BCAA) accumulation is observed in cells afflicted by proliferative diseases but not in regenerating normal tissue.

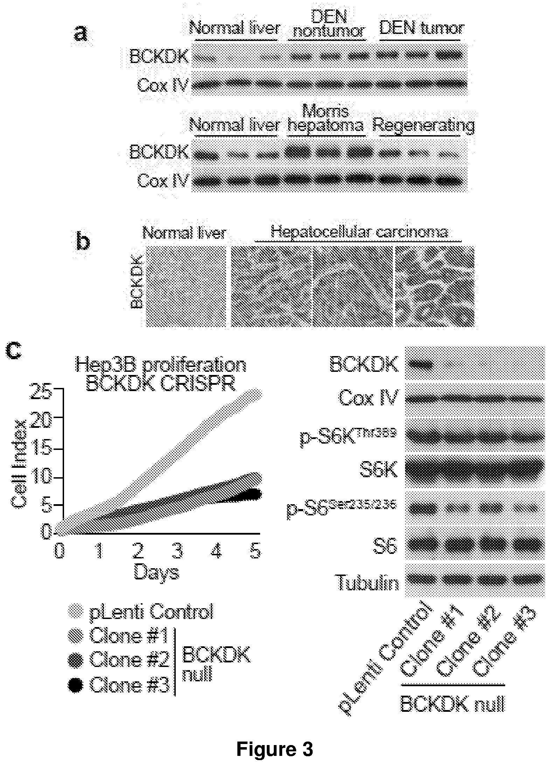

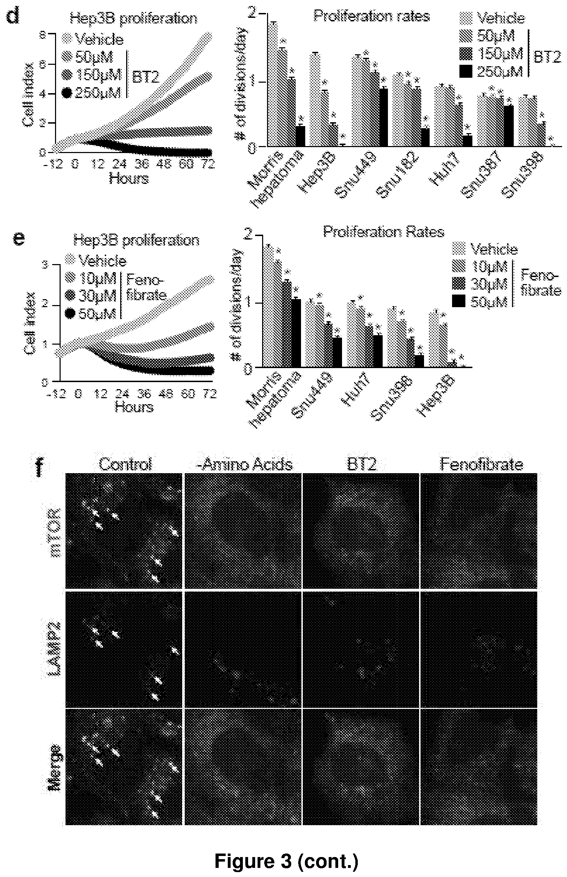

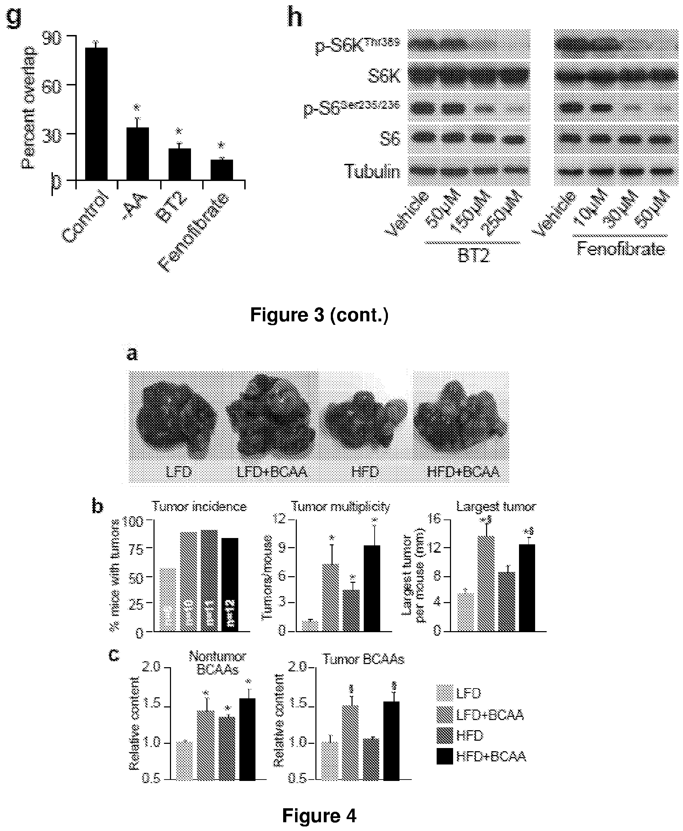

**Figure 3** shows a set of images depicting that BCKDK is a targetable kinase regulating BCAA catabolism, mTOR activity and cancer cell proliferation *in vitro.* **Figure 3a** shows a set of photographs of immunoblots of animal tumor and regenerating model mitochondrial fractions showing higher protein expression of BCKDK in tumors. **Figure 3b** shows a set of photographs depicting representative BCKDK immunohistochemical micrographs from normal liver tissue and hepatocellular carcinoma (HCC) tumor biopsies, as profiled by The Human Protein Atlas. The immunohistochemistry (IHC) micrographs show that the protein levels of BCKDK as detected in the HCC samples are higher than the level of BCKDK in the normal liver. **Figure 3c** shows a line graph and a set of photographs depicting characterization of Hep3B control and CRISPR-Cas9-mediated BCKDK null clones, including real-time proliferation curves and immunoblots of mitochondrial fractions (BCKDK and CoxIV) confirming knockout, and whole cell lysates (remaining) detailing the mTOR pathway activity. **Figure 3d** shows a line graph and a bar graph depicting real-time proliferation curves of Hep3B cells, and calculation of proliferation rates (number of divisions per day) for liver cancer cell lines treated with BT2. **Figure 3e** shows a line graph and a bar graph depicting real-time proliferation curves of Hep3B cells, and calculation of proliferation rates (number of divisions per day) for liver cancer cell lines treated with Fenofibrate. **Figure 3f** shows a set of photographs depicting representative immunofluorescent pictures of Hep3B cells grown in standard conditions, deprived of all amino acids for 60 minutes, or treated with the BCKDK inhibitors BT2 or Fenofibrate for 2 hours. Arrows highlight the co-localization of mTOR and lysosomal marker LAMP2. The immunofluorescence images show that under the three treatment conditions (-amino acids, BT2 and Fenofibrate), no significant co-localization was observed, in contrast to the control condition, where clear and significant co-localization of LAMP2 and mTOR was observed, consistent with mTOR activation under the control condition, but lack of mTOR activation under the treatment conditions. **Figure 3g** shows a bar graph depicting the quantification of average mTOR/LAMP2 percent co-localization in at least 10 random cells in each group. **Figure 3h** shows a set of photographs depicting immunoblots detailing mTOR pathway activity in Hep3B cells after 2 hours of BT2 or Fenofibrate treatment. Student's t-test, *P<0.05, compared to respective controls. Data are shown as mean ± standard error (s.e.) Thus, **Figure 3** shows that in a cell afflicted by proliferative diseases, mTOR activation and cell proliferation are sustained due to reduced activity of the BCAA catabolic enzymes.

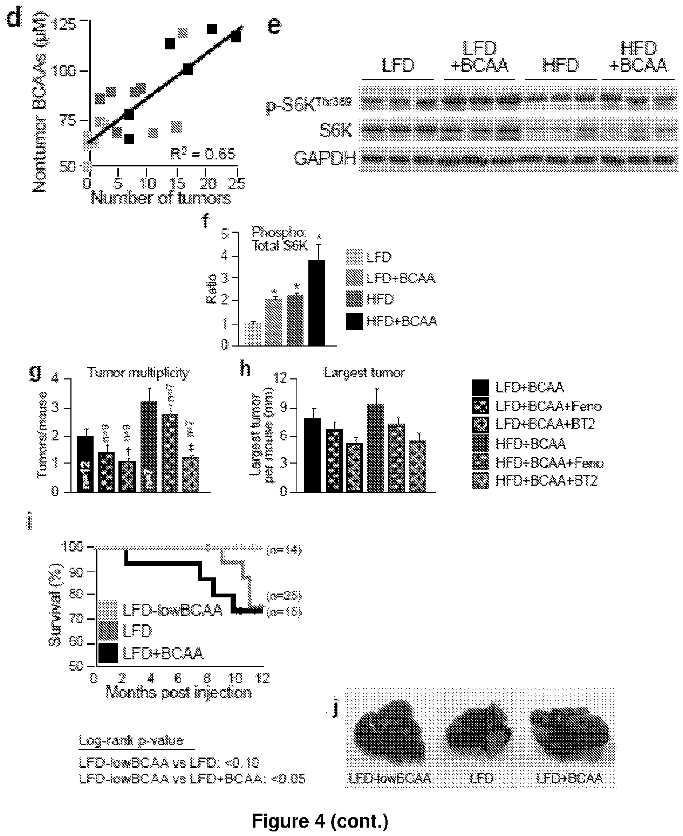

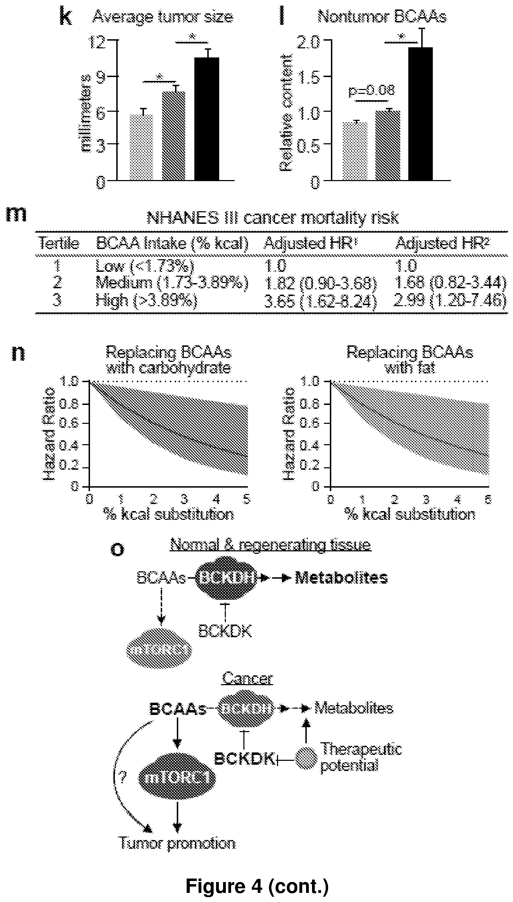

**Figure 4** shows a set of images depicting that dietary branched-chain amino acids modulate tumor development and growth *in vivo.* Analysis of mice 8 months after DEN injection, fed either a low fat diet (LFD, 10% kcal from fat) or high fat diet (HFD, 45% kcal from fat) with normal or high (+150%) levels of BCAAs. **Figure 4a** shows a set of photographs depicting representative livers of each cohort group. The photographs show significantly more or apparent tumors in LFD+BCAA, HFD and HFD+BCAA conditions when compared with LFD condition. These results were quantified and presented in Figure 4b. **Figure 4b** shows a set of bar graphs depicting tumor incidence of each cohort group, as well as quantification of the number of tumors (≥3mm) and size of the largest tumor per mouse. **Figure 4c** shows a set of bar graphs depicting quantification of BCAA content in nontumor and tumor liver tissues. **Figure 4d** shows a scatter plot depicting association of tumor multiplicity and nontumor liver BCAA content. **Figure 4e** shows a set of photographs depicting immunoblots detailing the mTOR pathway activity in nontumor liver tissues. **Figure 4f** shows a bar graph depicting quantification of phospho:total p70S6K ratios. **Figure 4g** shows a bar graph depicting quantification of the number of tumors (≥3mm) obtained from the analysis of mice 8 months after DEN injection, fed either LFD+BCAA or HFD+BCAA with 0.05% Fenofibrate or 0.02% BT2. **Figure 4h** shows a bar graph depicting size of the largest tumor per mouse obtained from the analysis of mice 8 months after DEN injection, fed either LFD+BCAA or HFD+BCAA with 0.05% Fenofibrate or 0.02% BT2. **Figure 4i** shows a line graph depicting Kaplan-Meier survival curves obtained from analysis of DEN-injected mice fed low fat diets with low (-50%), normal, or high (+150%) levels of BCAAs. **Figure 4j** shows a set of photographs depicting representative livers obtained from analysis of DEN-injected mice fed low fat diets with low (-50%), normal, or high (+150%) levels of BCAAs. The photographs show that LFD-lowBCAA has much significantly fewer or smaller tumors compared with LFD and LFD+BCAA groups. These were quantified and presented in Figure 4k. **Figure 4k** shows a bar graph depicting average tumor sizes 12 months after DEN injection obtained from analysis of DEN-injected mice fed low fat diets with low (-50%), normal, or high (+150%) levels of BCAAs. Figure 4l shows a bar graph depicting quantification of nontumor liver tissue BCAA content obtained from analysis of DEN-injected mice fed low fat diets with low (-50%), normal, or high (+150%) levels of BCAAs. **Figure 4m** shows a table showing the analysis of individuals 50-66 years-old in the NHANES III dataset. Hazard ratios (with 95% confidence intervals) based on BCAA intake, adjusted for age, sex, race, total kcal, usual dietary intake, diet change, physical activity, intentional weight loss, waist circumference, smoking, education, and prior diagnosis of cancer, diabetes and cardiovascular disease, additionally adjusted for % kcal from other macronutrients. **Figure 4n** shows a table showing the analysis of individuals 50-66 years-old in the NHANES III dataset. Substitution analysis comparing change in risk when replacing BCAAs with carbohydrate or fat, with the same adjustments as HR2 except total kcal and % kcal from nonBCAA protein. Data are presented as hazard ratio (solid line) with 95% confidence interval (shaded area). **Figure 4o** shows a flowchart depicting summary of BCAA catabolism in normal, regenerating, and cancerous tissues. Student's t-test, *P<0.05 vs. LFD, §P<0.05 vs HFD. †P<0.05 vs LFD+BCAA. ‡P<0.05 vs HFD+BCAA. Data are shown as mean ± s.e. Thus, **Figure 4** shows that diets with high levels of branched-chain amino acids (BCAAs) may lead to accumulation of BCAA in cells and lead to proliferative diseases.

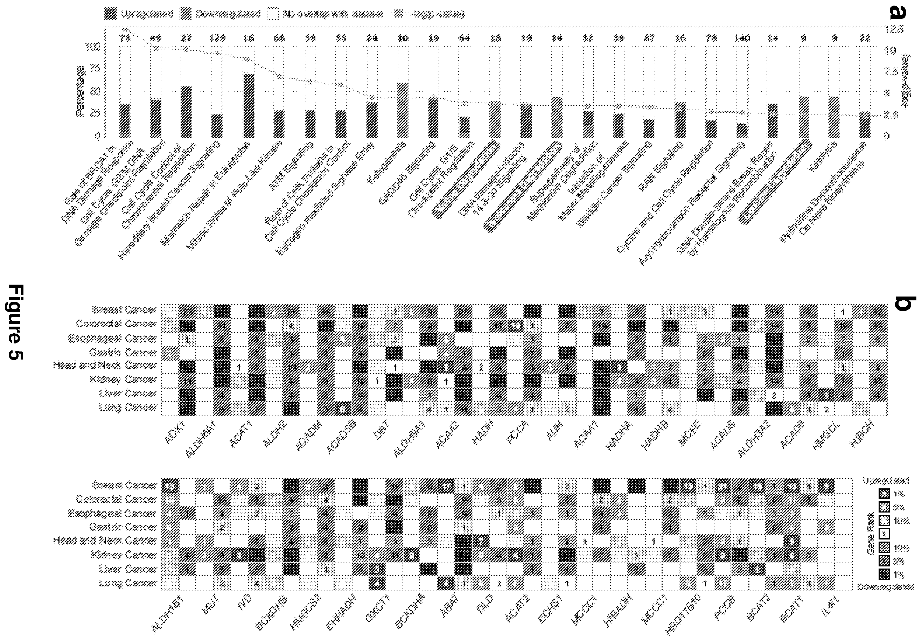

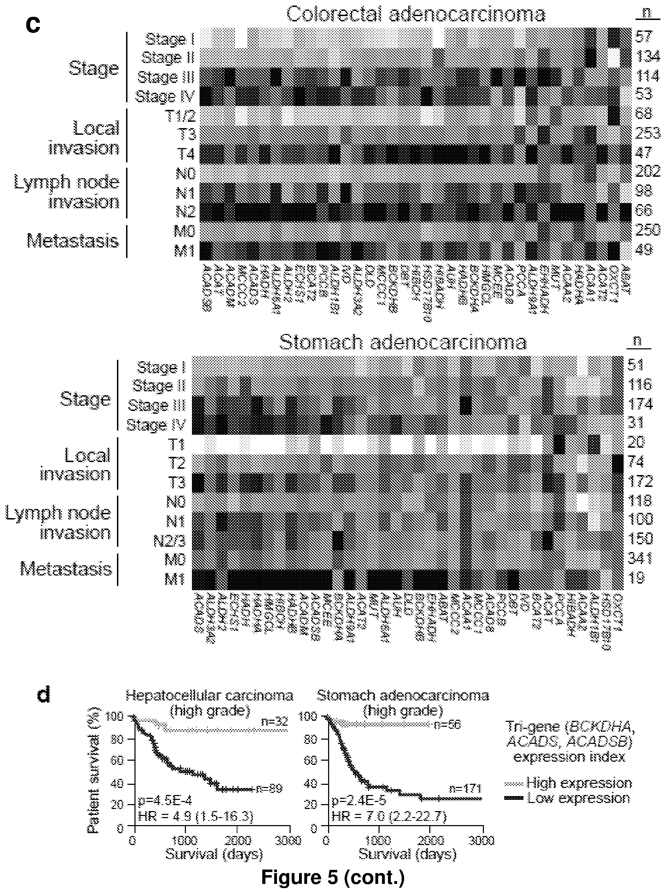

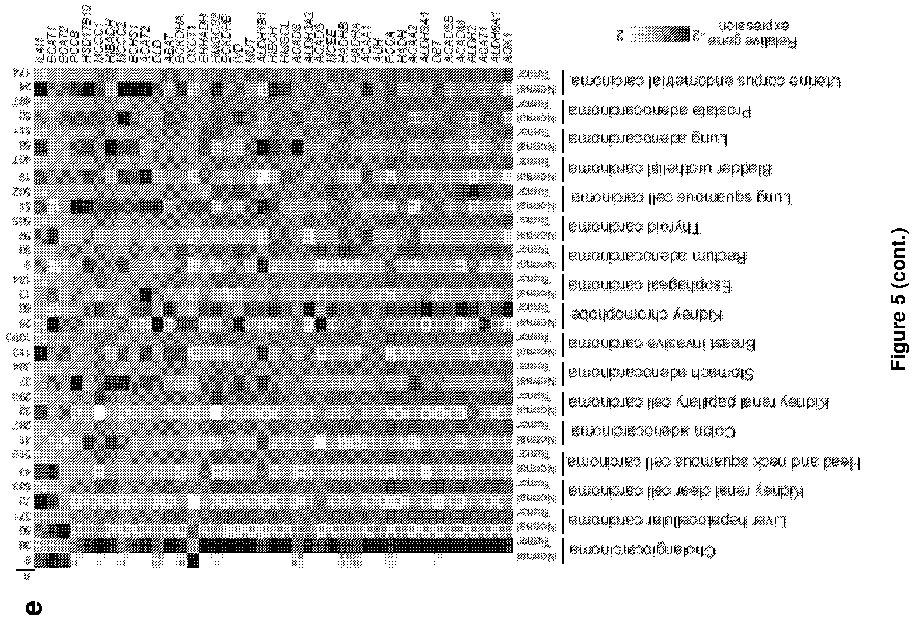

**Figure 5** shows a set of images depicting that suppression of branched-chain amino acid catabolic enzyme expression is consistent across multiple cancers and cohorts. **Figure 5a** shows a combination of bar graph and line graph depicting Ingenuity Pathway Analysis of the 1501 genes identified as differentially expressed in gastrointestinal tumors. **Figure 5b** shows a diagram depicting Oncomine analysis of mRNA datasets profiling various cancers compared to normal controls. Colors reflect the degree of over- or under-expression, and numbers reflect the number of studies included in the analysis. **Figure 5c** shows a diagram depicting the summary of branched-chain amino acid catabolic enzyme transcript levels in colorectal adenocarcinoma and stomach adenocarcinoma tumors sorted by stage, grade, primary tumor size and local invasion (T score), lymph node invasion (N score), and distant metastases (M score). Figure 5c is similar to figure 1d. However instead of depicting the summary of branched-chain amino acid catabolic enzyme transcript levels in HCC, figure 5c depicts the summary of branched-chain amino acid catabolic enzyme transcript levels in colorectal adenocarcinoma or stomach adenocarcinoma. **Figure 5d** shows a set of line graphs depicting Kaplan-Meier survival estimate curves for patients with high grade hepatocellular carcinoma and stomach adenocarcinoma tumors ranked by a combined index of tumor *BCKDHA*, *ACADS*, and *ACADSB* expression. P-values for log-rank test and cox proportional hazard ratios (HR, with 95% percent confidence intervals) adjusted for age, sex, tumor stage and grade, and radiation, prescription and additional therapies shown. **Figure 5e** shows a diagram depicting the summary of branched-chain amino acid catabolic enzyme transcript levels for all cancers profiled by The Cancer Genome Atlas with at least 5 solid normal tissue samples. Cancers are arranged from those with the greatest number of enzymes suppressed (top) to the least (bottom) and genes are arranged from those suppressed in the greatest number of cancers (left) to the least (right). Thus, **Figure 5** shows that in addition to the suppression of BCAA catabolic enzymes activity, in cells afflicted with proliferative diseases, the expression of BCAA catabolic enzymes are also suppressed.

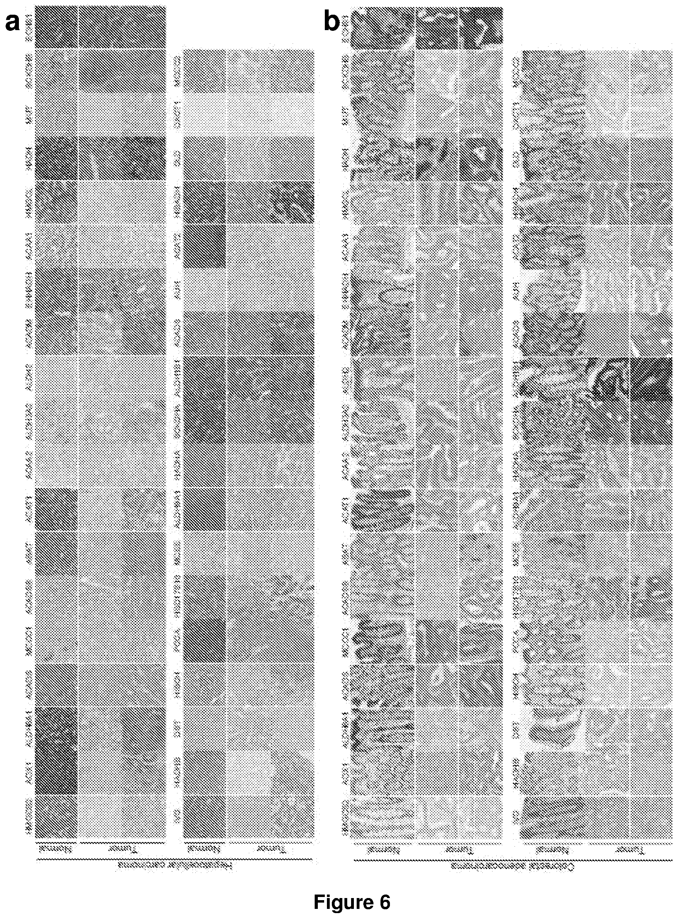

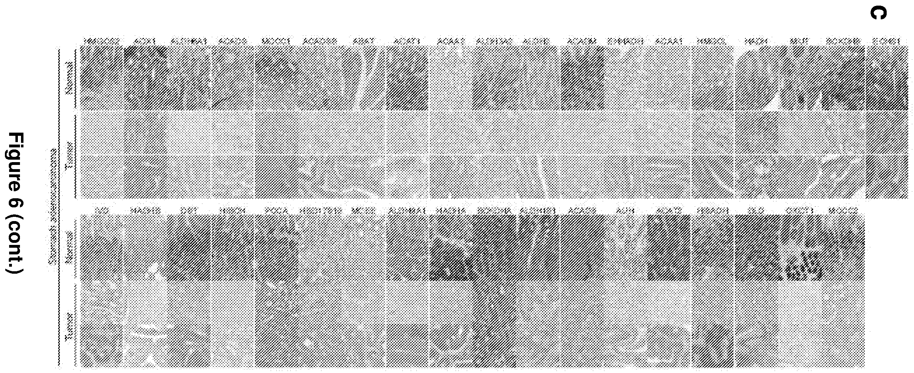

**Figure 6** shows a set of images depicting that protein levels of branched-chain amino acid catabolic enzymes are reduced in human cancers. **Figure 6a** shows a set of images depicting the representative immunohistochemical micrographs from normal tissue and tumor biopsies for hepatocellular carcinoma, as profiled by The Human Protein Atlas. **Figure 6b** shows a set of images depicting the representative immunohistochemical micrographs from normal tissue and tumor biopsies for colorectal adenocarcinoma, as profiled by The Human Protein Atlas. **Figure 6c** shows a set of images depicting the representative immunohistochemical micrographs from normal tissue and tumor biopsies for stomach adenocarcinoma, as profiled by The Human Protein Atlas. Figure 6 shows the result of immunohistochemical micrographs in three different cancers. In Figure 6, it can be observed that the levels of the indicated genes, which encode enzymes in the BCAA degradation pathways, appear to be reduced in the tumors compared to normal samples. That is, the downregulation of all the catabolic enzymes can be seen. Thus, **Figure 6** shows that comparative immunohistochemical micrographs from normal tissue and tumor biopsies indicates that there is a reduction in the protein levels of branched-chain amino acid catabolic enzymes in tissues from tumor biopsies.

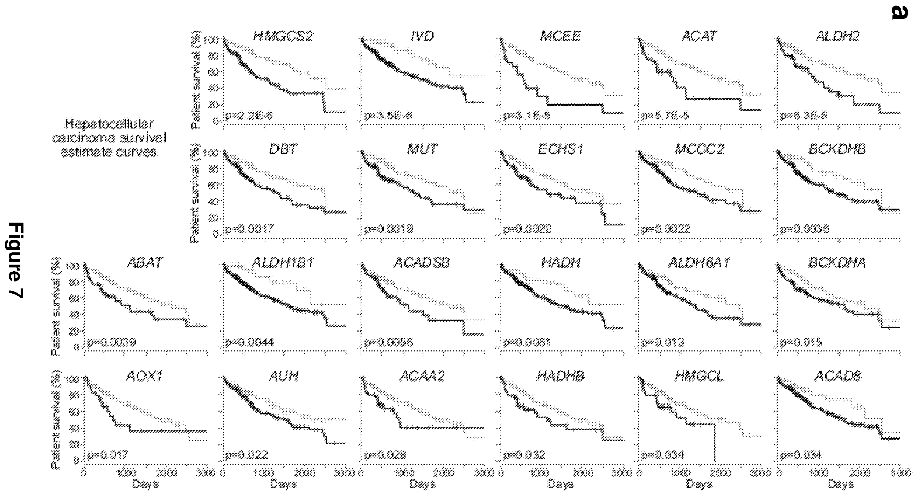

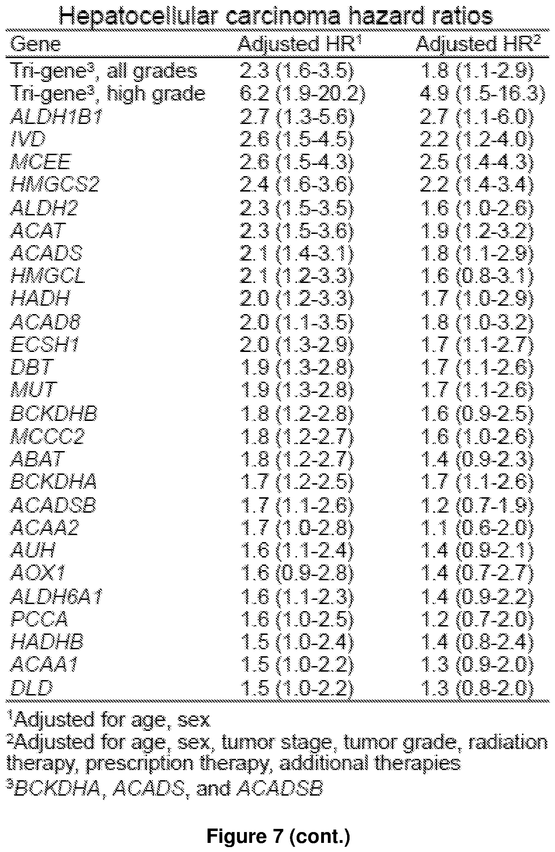

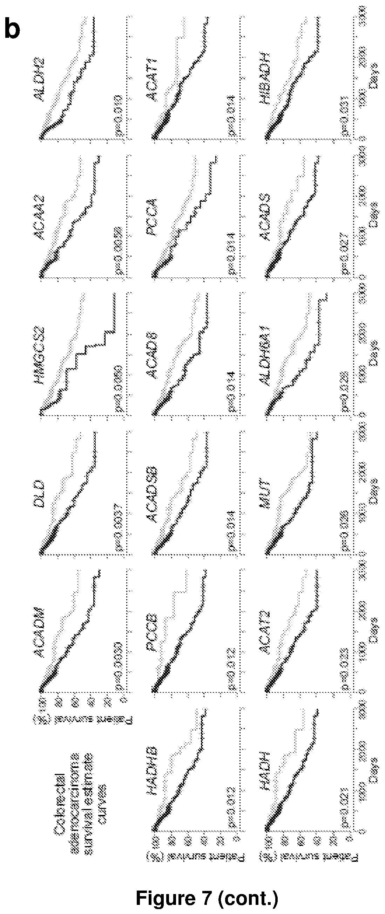

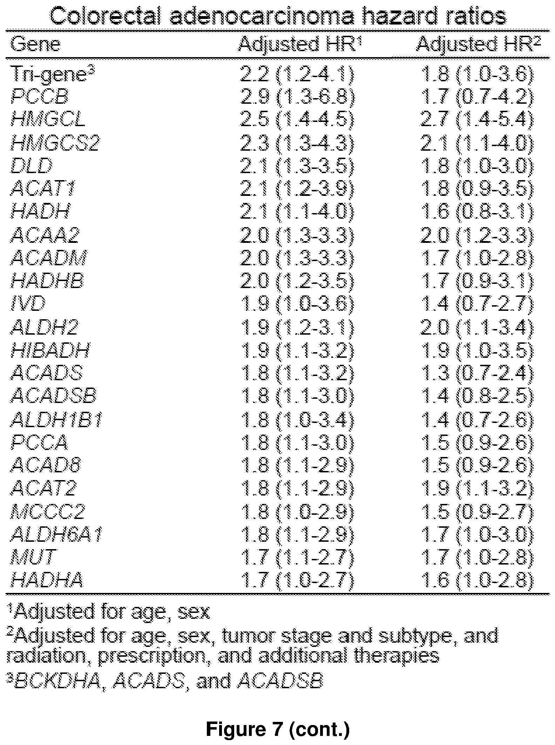

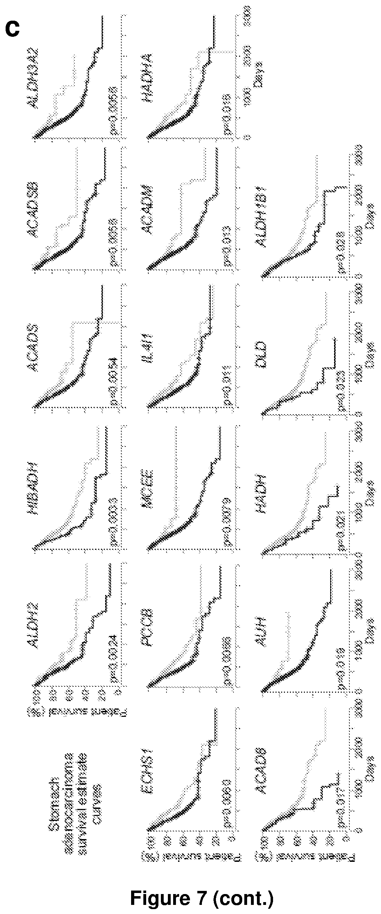

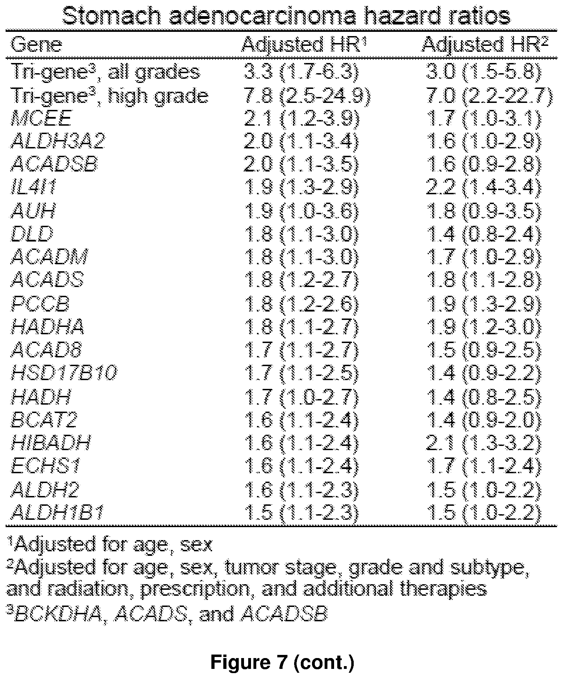

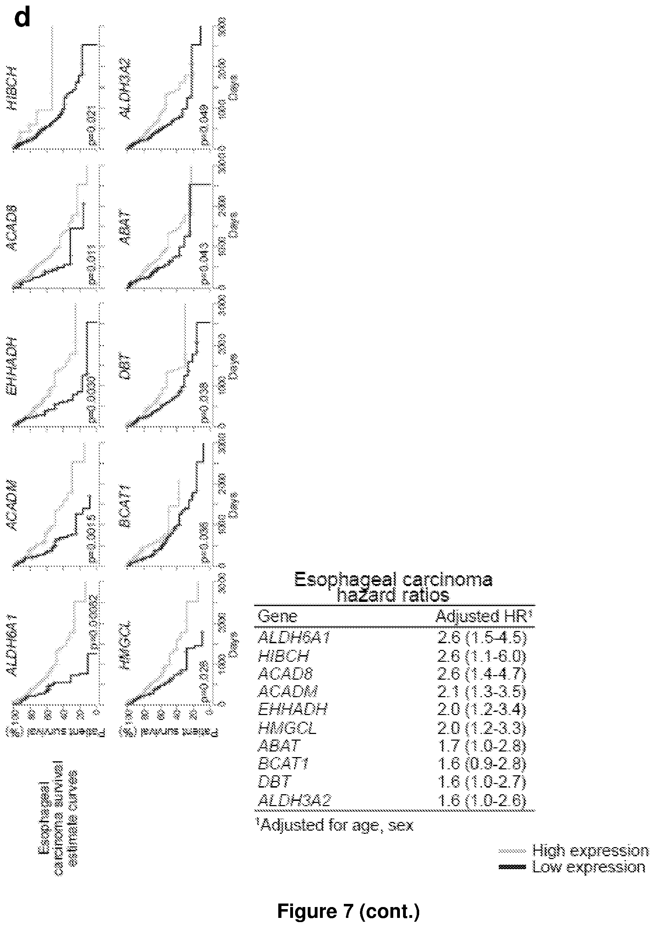

**Figure 7** shows a set of images depicting that the expression of branched-chain amino acid catabolic enzymes are independent predictors of clinical outcome. **Figure 7a** shows a table and a set of line graphs depicting Kaplan-Meier survival estimate curves of hepatocellular carcinoma (n=331) patients with high or low tumor expression of enzymes involved in BCAA catabolism. P-values for log-rank test and adjusted cox proportional hazard ratios with 95% confidence intervals for low expression group shown. **Figure 7b** shows a table and a set of line graphs depicting Kaplan-Meier survival estimate curves of colorectal adenocarcinoma (n=361) patients with high or low tumor expression of enzymes involved in BCAA catabolism. P-values for log-rank test and adjusted cox proportional hazard ratios with 95% confidence intervals for low expression group shown. **Figure 7c** shows a table and a set of line graphs depicting Kaplan-Meier survival estimate curves of stomach adenocarcinoma (n=360) patients with high or low tumor expression of enzymes involved in BCAA catabolism. P-values for log-rank test and adjusted cox proportional hazard ratios with 95% confidence intervals for low expression group shown. **Figure 7d** shows a table and a set of line graphs depicting Kaplan-Meier survival estimate curves of esophageal carcinoma (n=170) patients with high or low tumor expression of enzymes involved in BCAA catabolism. P-values for log-rank test and adjusted cox proportional hazard ratios with 95% confidence intervals for low expression group shown. Thus, **Figure 7** shows that expression level of BCAA catabolic enzymes in cells afflicted with proliferative diseases can be used to predict likelihood of patient survival.

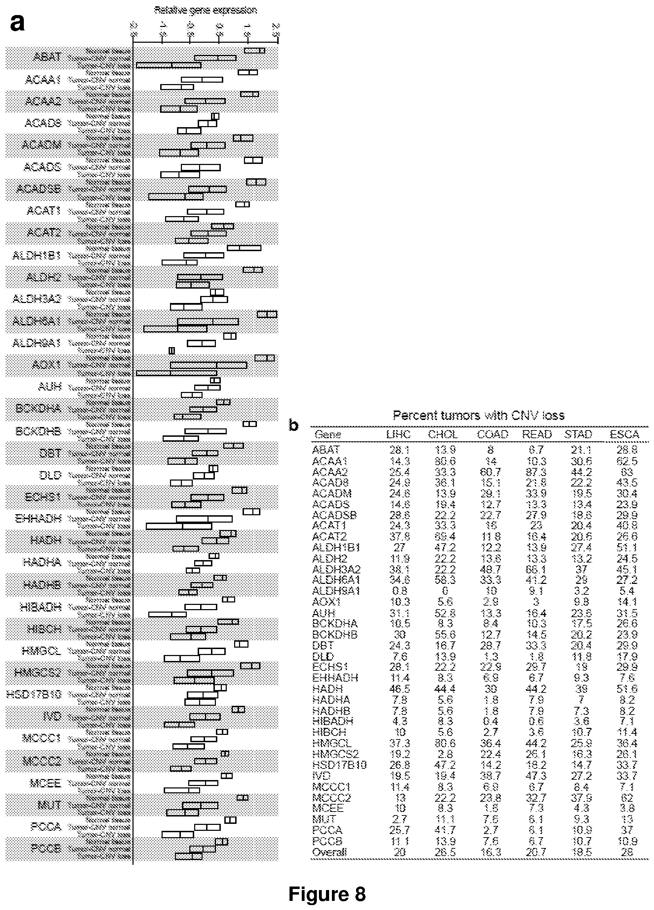

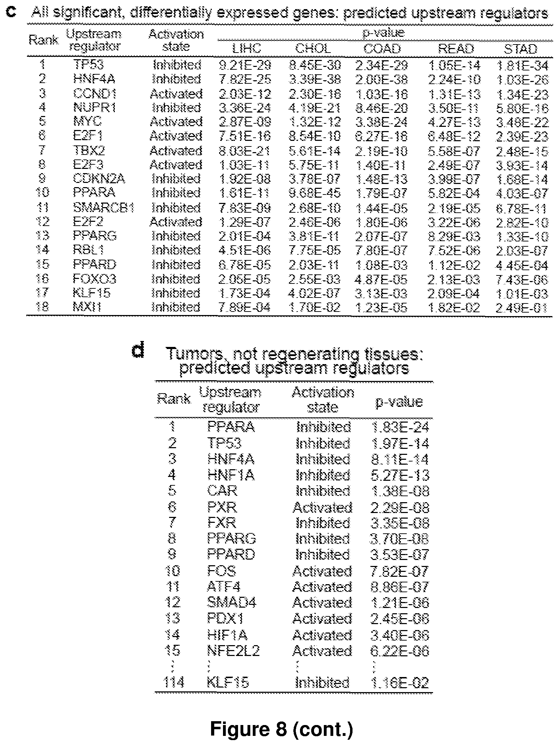

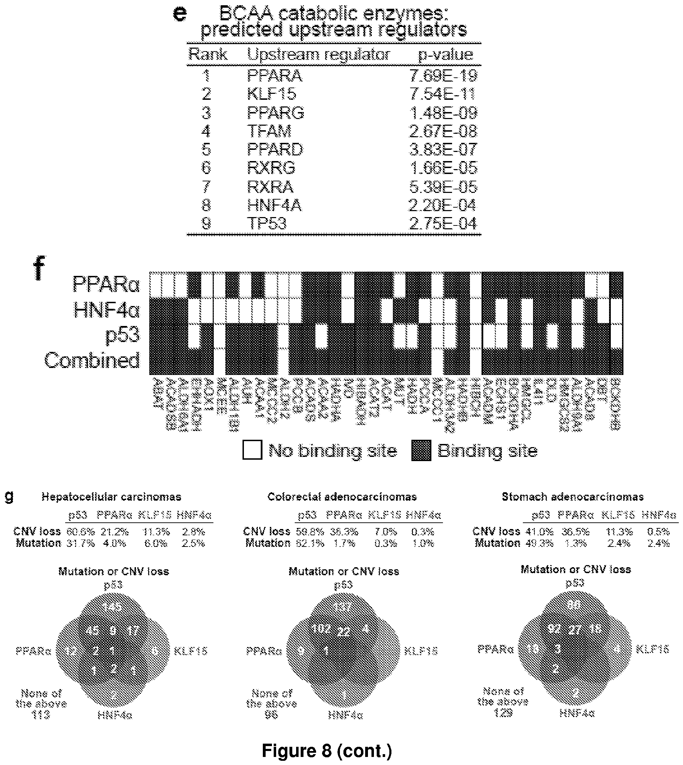

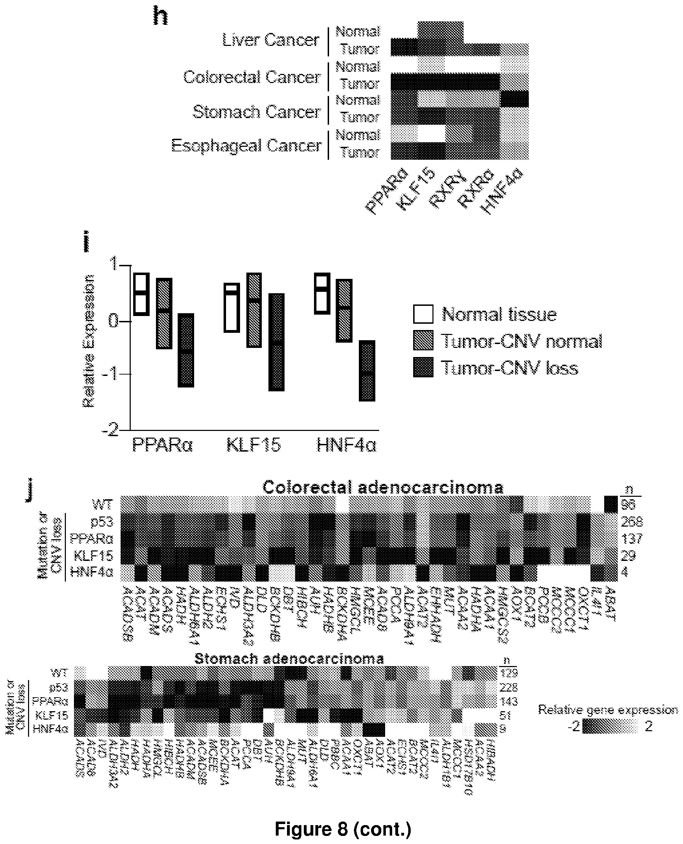

**Figure 8** shows a set of images depicting that BCAA catabolism enzyme expression is lost due to copy number variation (CNV) loss and changes in regulatory transcription factors. **Figure 8a** shows a graph depicting summary of BCAA catabolic enzyme expression in normal liver tissues and hepatocellular carcinomas with or without CNV loss of indicated enzymes. **Figure 8b** shows a table depicting frequency of CNV loss for BCAA catabolic enzymes across the gastrointestinal cancers. **Figure 8c** shows a table depicting Ingenuity Pathway Analysis, predicted upstream regulators of all significant, differentially expressed genes across the gastrointestinal cancers profiled by The Cancer Genome Atlas. **Figure 8d** shows a table depicting Ingenuity Pathway Analysis, predicted upstream regulators of significant, differentially expressed genes in tumor models but not regenerating tissues. **Figure 8e** shows a table depicting Ingenuity Pathway Analysis, predicted upstream regulators of BCAA catabolic enzymes. **Figure 8f** shows a diagram depicting transcription factors with confirmed binding sites on the promoters of BCAA catabolic enzymes. **Figure 8g** shows a set of tables and a set of Venn diagrams depicting frequency of transcription factor mutation or CNV loss across the gastrointestinal cancers. **Figure 8h** shows a diagram depicting summary of transcription factor expression across gastrointestinal cancers (those not shown are significantly downregulated in less than 3 cancers, and of these, only p53 is mutated in a significant number of tumors). The results show that in the four indicated cancers, the expression levels of the five genes (PPARa, KLF15, RXRg, RXRa and HNF4a) are lower in tumor samples when compared with normal samples. **Figure 8i** shows a bar graph depicting summary of transcription factor expression in normal liver tissues and hepatocellular carcinomas with or without CNV loss of indicated transcription factors. **Figure 8j** shows a diagram depicting summary of branched-chain amino acid catabolic enzyme transcript levels in tumors with indicated transcription factor mutation or CNV loss. Figure 8j is similar to Figure 1f. However, instead of branched-chain amino acid catabolic enzyme transcript levels in HCC, Figure 8j showed that in colorectal adenocarcinoma or stomach adenocarcinoma, the expression levels of the indicated genes encoding BCAA degradation enzymes are lower in those with mutation or CNV loss of p53, PPAR1, KLF15 or HFN4a when compared with wildtype samples. Student's t-test, *P<0.05, compared to respective controls. Data are shown as mean ± s.e. Thus, **Figure 8** shows that CNV loss and changes in regulatory transcription factors can affect the BCAA catabolism enzyme expression.

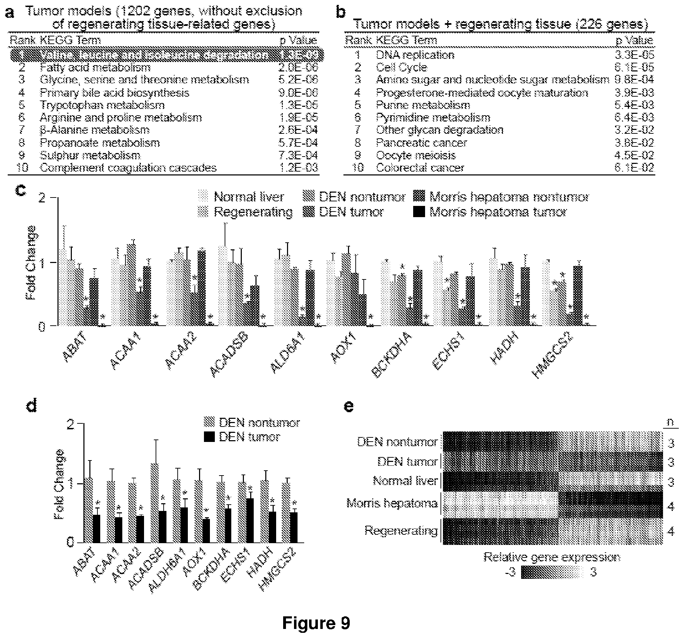

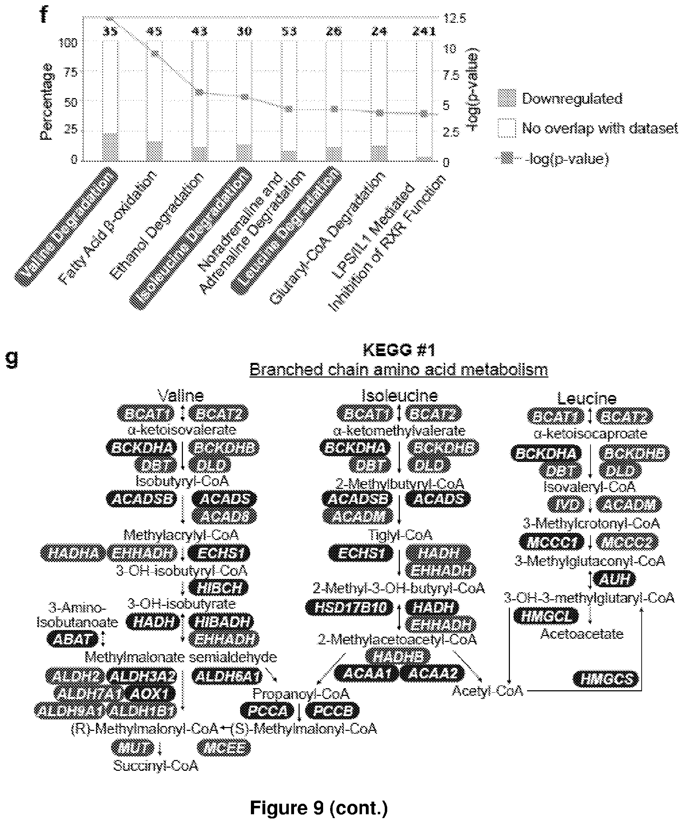

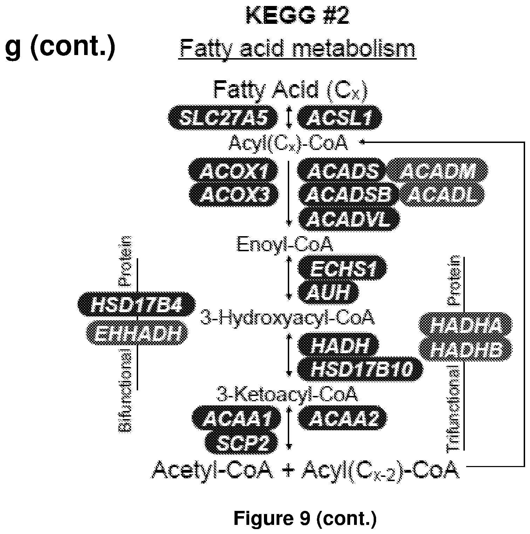

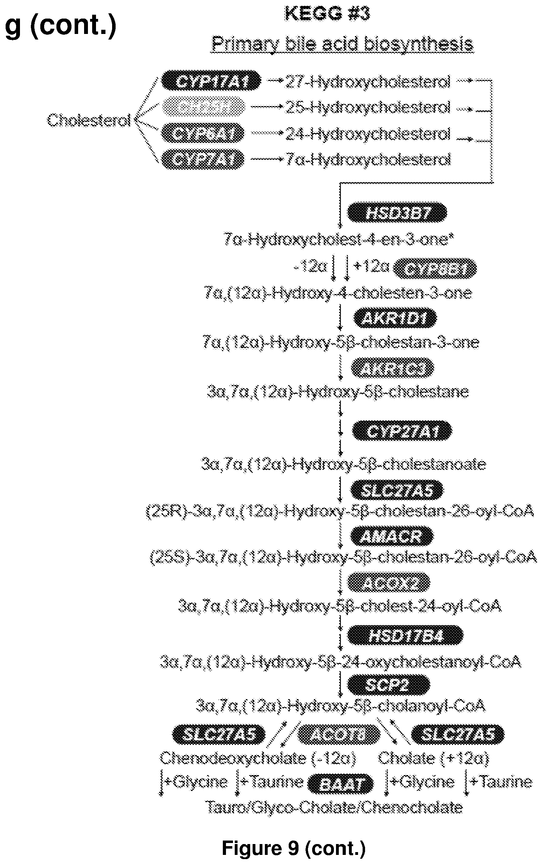

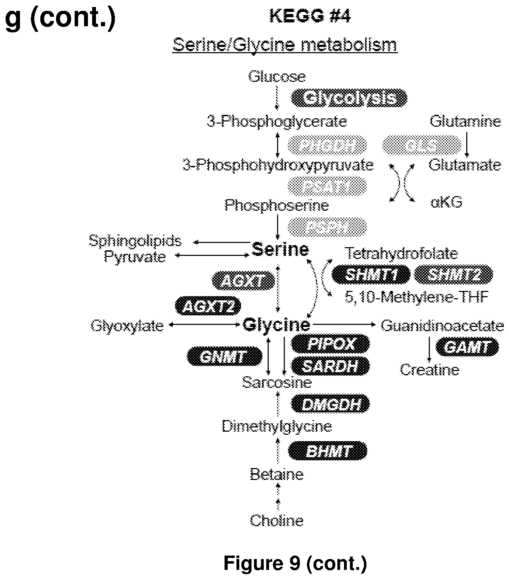

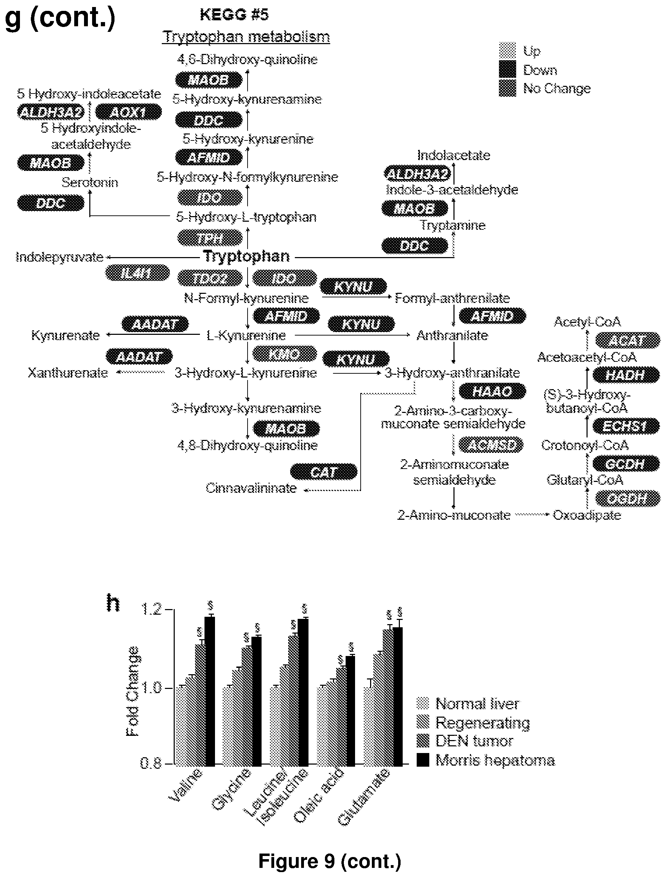

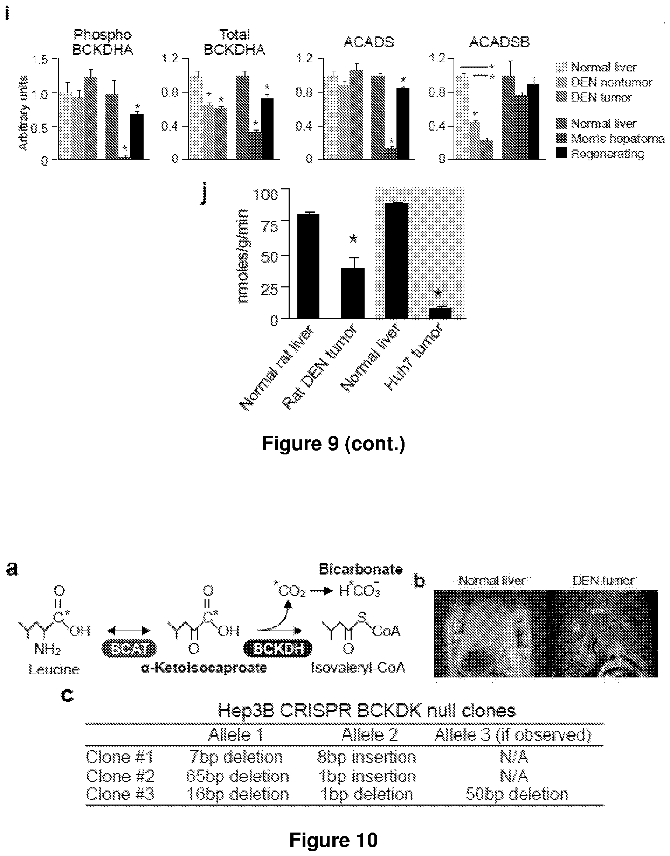

**Figure 9** shows a set of images depicting that liver cancer models match the transcriptomic profiles of human liver cancers and display loss of BCAA catabolic activity.. **Figure 9a** shows a table depicting the KEGG pathway analysis inclusive of all 1202 genes shared by the two tumor models. **Figure 9b** shows a table depicting the KEGG pathway analysis of the 226 genes shared by regenerating tissues and DEN and Morris hepatoma tumor models. **Figure 9c** shows a bar graph depicting RT-PCR analysis of BCAA catabolic enzyme genes from rat tumor and regenerating tissues, normalized to normal liver tissues. **Figure 9d** shows a bar graph depicting RT-PCR analysis of BCAA catabolic enzyme genes from mouse tumor tissues, normalized to nontumor liver tissue. **Figure 9e** shows a diagram depicting expression summary of the 976 genes identified in the transcriptomic analysis. Mouse DEN tumor tissues are compared to mouse DEN nontumor tissues, and Morris Hepatoma and regenerating liver tissues are compared to rat normal liver tissue. **Figure 9f** shows a combination of line graph and bar graph depicting the canonical pathways identified by Ingenuity Pathway Analysis for the 976 genes identified in the transcriptomic analysis. **Figure 9g** shows a flowchart depicting summary of gene expression changes in the top KEGG pathways. **Figure 9h** shows a bar graph depicting non-targeted metabolomics analysis of rat DEN-induced tumors, Morris hepatoma tumors, and rat regenerating tissues, normalized to normal liver tissues. **Figure 9i** shows a set of bar graphs depicting quantification of immunoblots presented in Figure 2f, normalized to respective normal liver tissue controls. Figure 9j shows a bar graph depicting quantification of BCKDH complex activity in two additional tumor models, DEN-induced tumors in rats and Huh7 tumors. Student's t-test, *P<0.05, §P<0.01, compared to respective controls. Data are shown as mean ± s.e Thus, **Figure 9** shows that rodent (e.g. mouse and rat) liver cancer models may be used as a model to further study human liver cancer.

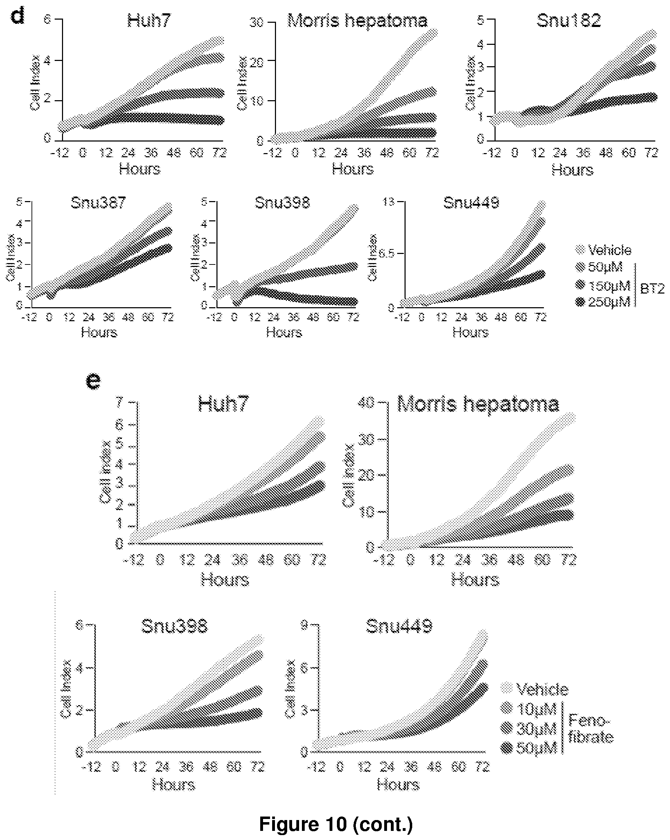

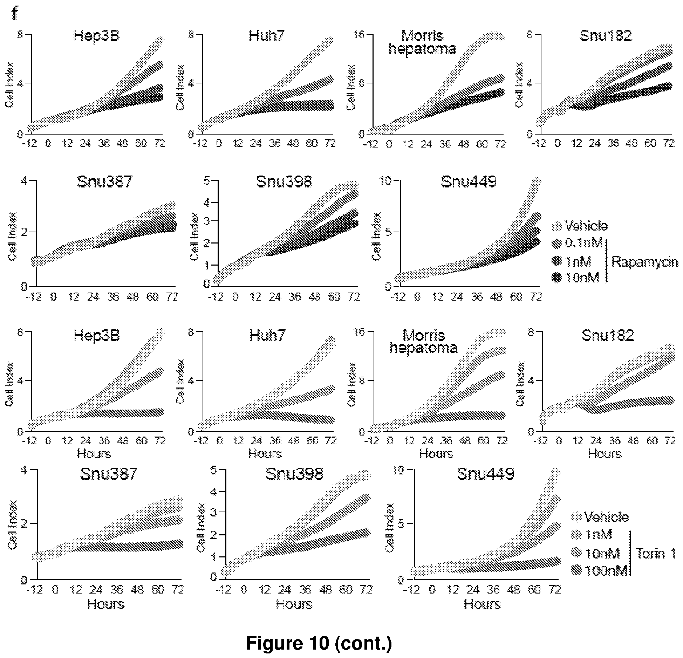

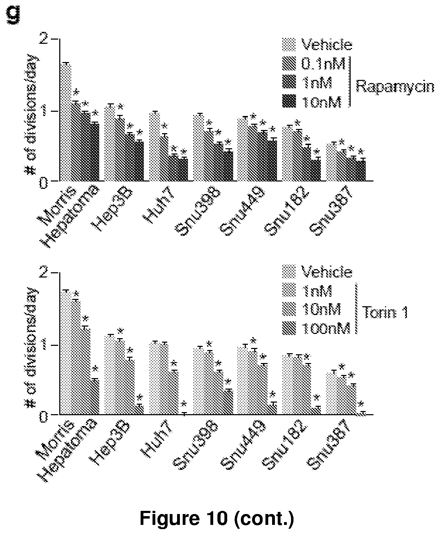

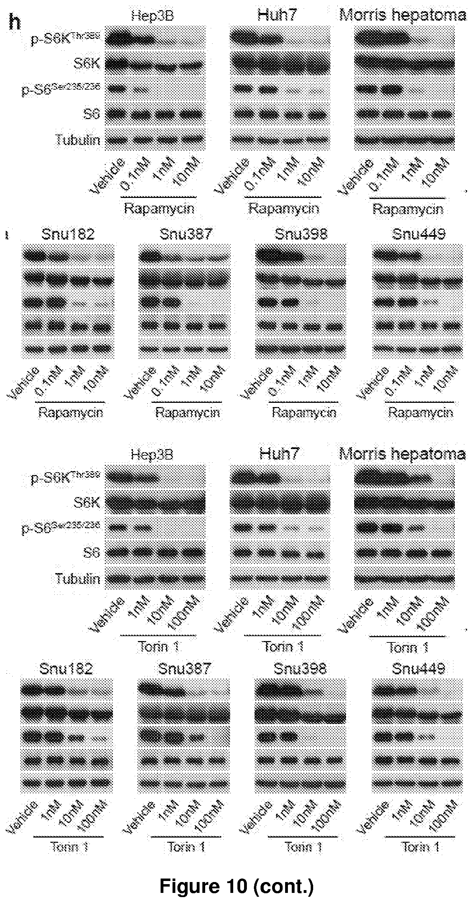

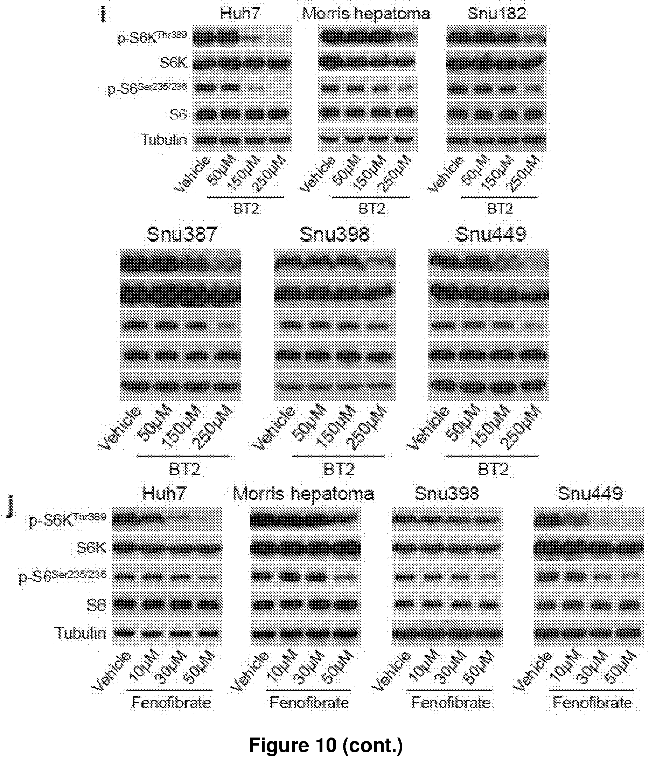

**Figure 10** shows a set of images depicting that BCKDK is a targetable kinase regulating branched-chain amino acid catabolism and cell proliferation. **Figure 10a** shows a reaction scheme depicting schematic of magnetic resonance spectroscopy (MRS) BCKDH complex activity assay. Hyperpolarized [1-C¹³] α-ketoisocaproate was injected by tail vein, and enzyme activity was assessed by detection of labeled bicarbonate in the liver. **Figure 10b** shows a set of photographs depicting representative coronal FLASH MRI images of DEN-induced liver tumors and normal liver from rats used in the MRS analyses. The MRI images show representative abdominal scans of normal and liver tumors (arrows) from the rats in the MRS studies. **Figure 10c** shows a table depicting summary of frame-shift mutations caused by CRISPR-Cas9-mediated insertions and/or deletions in the BCKDK gene of Hep3B clones. **Figure 10d** shows a set of line graphs depicting the representative real-time proliferation curves of the liver cancer cell lines treated with the BCKDK inhibitor, BT2. **Figure 10e** shows a set of line graphs depicting the representative real-time proliferation curves of the liver cancer cell lines treated with the BCKDK inhibitor, Fenofibrate. **Figure 10f** shows a set of line graphs depicting representative real-time proliferation curves of the characterization of liver cancer cell lines treated with the mTOR inhibitors Rapamycin or Torin 1. **Figure 10g** shows a set of bar graphs depicting number of divisions per day for the characterization of liver cancer cell lines treated with the mTOR inhibitors Rapamycin or Torin 1. **Figure 10h** shows a set of photographs depicting immunoblots detailing the mTOR pathway activity after 2 hours of treatment for the characterization of liver cancer cell lines treated with the mTOR inhibitors Rapamycin or Torin 1. **Figure 10i** shows a set of photographs depicting immunoblots detailing the mTOR pathway activity after 2 hours of BT2 treatment for the characterization of liver cancer cell lines treated with the mTOR inhibitors Rapamycin or Torin 1. **Figure 10j** shows a set of photographs depicting immunoblots detailing the mTOR pathway activity after 2 hours of Fenofibrate treatment for the characterization of liver cancer cell lines treated with the mTOR inhibitors Rapamycin or Torin 1.. Student's t-test, *P<0.05, compared to respective controls. Data are shown as mean ± s.e. Thus, **Figure 10** shows that targeting BCKDK kinases in subjects or cells afflicted proliferative diseases may regulate branched-chain amino acid catabolism and cell proliferation.

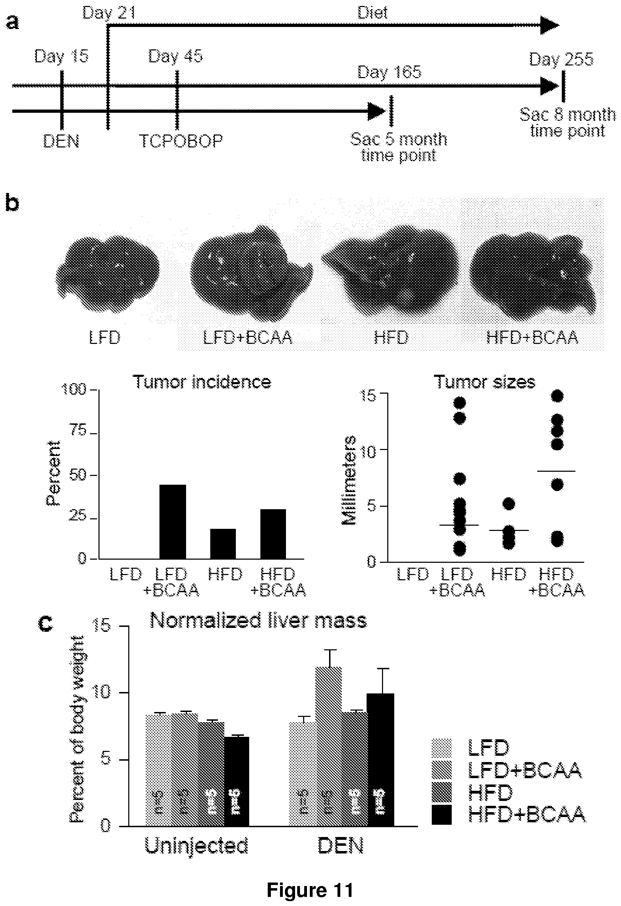

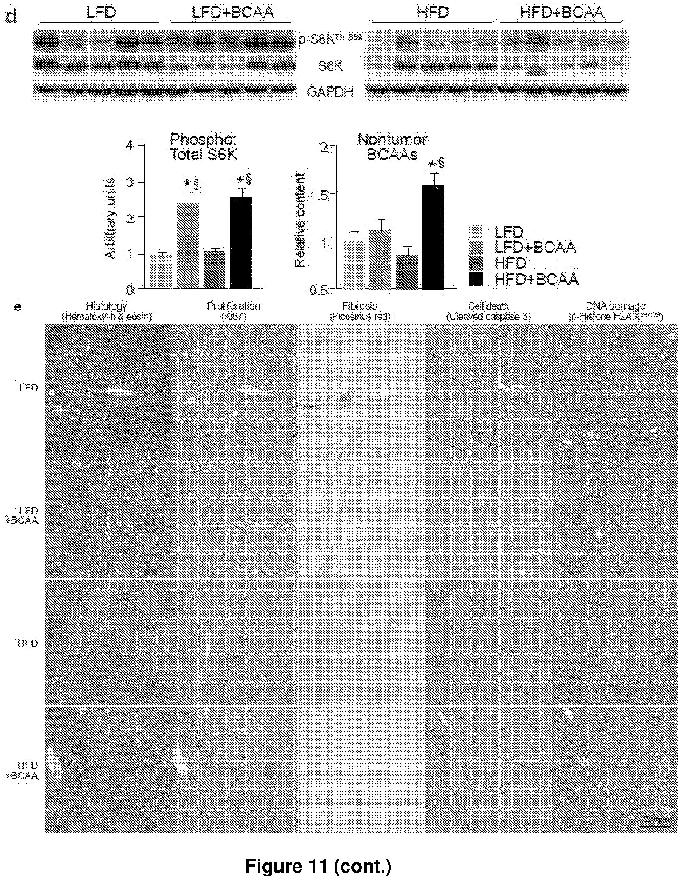

**Figure 11** shows a set of images depicting the characterization of mice on BCAA-supplemented diets 5 months post-DEN injection. **Figure 11a** shows a diagram of model timeline. This diagram indicates the feeding timeline of the mice. **Figure 11b** shows a set of photographs, a bar graph, and a scatter plot depicting representative livers and quantification of tumor incidence and tumor sizes from DEN-injected mice fed indicated diets. The photographs show that mice treated with LFD+BCAA, HFD and HFD+BCAA conditions have more obvious tumors. The analysis and quantification are presented as a bar graph and a scatter plot below the photographs. **Figure 11c** shows a bar graph depicting the liver masses of DEN-injected and uninjected mice fed indicated diets, normalized by body weight. Student's t-test, *P<0.05 compared to LFD, §P<0.05 compared to HFD. Data are shown as mean ± s.e. **Figure 11d** shows a set of photographs and a set of bar graphs depicting immunoblots of nontumor liver tissues with quantification of the phosphop70S6K^{Thr389} to total p70S6K ratio and nontumor BCAA content, normalized by the LFD group. Student's t-test, *P<0.05 compared to LFD, §P<0.05 compared to HFD. Data are shown as mean ± s.e. **Figure 11e** shows a set of photographs depicting representative histological analyses of DEN-injected mice fed indicated diets. The photographs show that BCAA supplementation did not lead to enhanced DNA damage, fibrosis or cell death. Thus, **Figure 11** shows that diet comprising high amount of BCAA may increase tumor size and tumor incidence in mice liver.

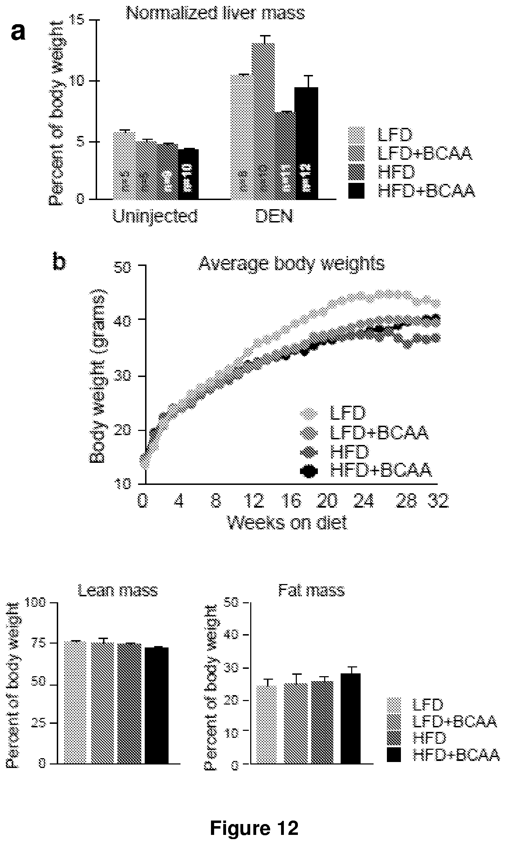

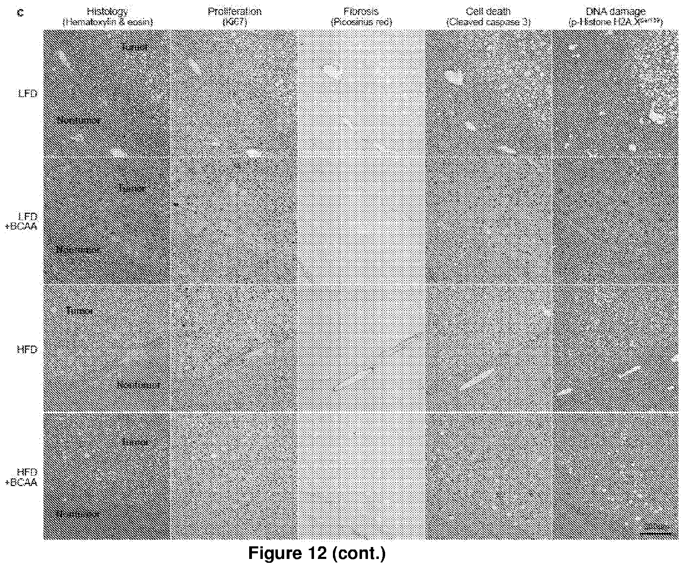

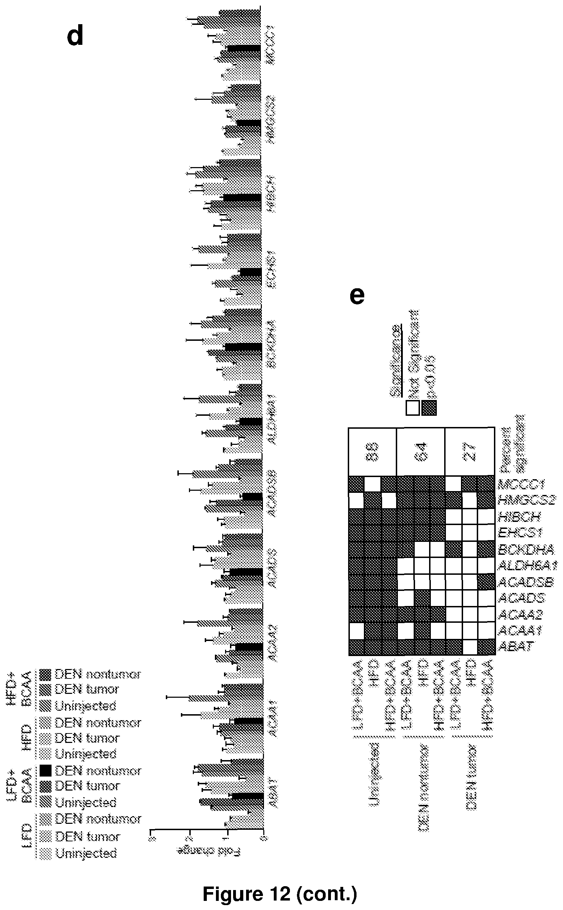

**Figure 12** shows a set of images depicting characterization of mice on BCAA-supplemented diets 8 months post-DEN injection. **Figure 12a** shows a bar graph depicting liver masses of DEN-injected and control uninjected mice fed indicated diets, normalized by body weight. **Figure 12b** shows a scatter plot and a set of bar graphs depicting weekly body weights, and day of sacrifice body composition of DEN-injected mice fed indicated diets. **Figure 12c** shows a set of photograph depicting the representative histological analyses of DEN-injected mice fed indicated diets. **Figure 12d** shows a bar graph depicting RT-PCR analysis of BCAA catabolic enzymes from nontumor and tumor liver tissues of DEN-injected mice and normal liver tissue of uninjected mice fed indicated diets, normalized to normal liver tissue of uninjected LFD-fed mice. Order of experimental groups in bar graph detailed below. **Figure 12e** shows a diagram depicting summary of RT-PCR statistical analyses, comparing tissues of LFD+BCAA, HFD, and HFD+BCAA groups to corresponding tissues of the LFD group. Data are shown as mean ± s.e. Thus, **Figure 12** shows that BCAAs (e.g. BCAAs obtained from diet) may specifically influence precancerous tissues and thus increase the liver size of mice.

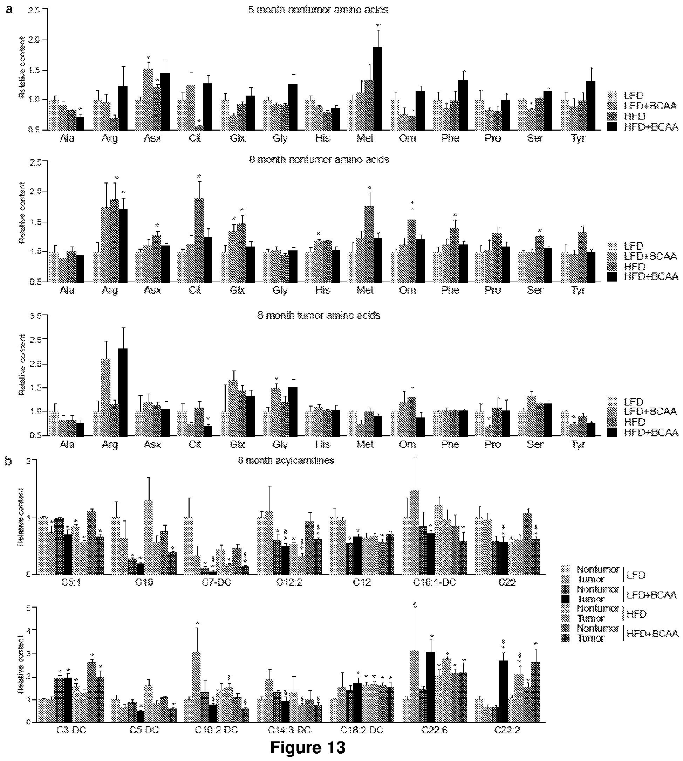

**Figure 13** shows a set of images depicting the metabolomic analyses of DEN-injected mice fed BCAA-supplemented diets. **Figure 13a** shows a set of bar graphs depicting the amino acid content of tumor and nontumor liver tissues of DEN-injected mice at 5 and 8 months post-injection, normalized to respective LFD groups. No tumors developed in LFD-fed mice at 5 months, and due to the lack of appropriate controls, these tissues were not included in analyses. Student's t-test, *P<0.05 compared to LFD group of respective cohort. Student's t-test, *P<0.05 compared to tissues of respective LFD group. **Figure 13b** shows a set of bar graphs depicting the acylcarnitine analysis of tumor and nontumor liver tissues of DEN-injected mice at 8 months post-injection, normalized to nontumor tissue of the LFD group. Order of experimental groups in bar graph detailed to right. Student's t-test, *P<0.05 compared to nontumor LFD, §P<0.05 compared to tumor HFD. Data shown as mean ± s.e. Thus, **Figure 13** shows that BCAA catabolism enhancers and BDK inhibitors may be utilized to treat subjects afflicted with proliferative diseases.

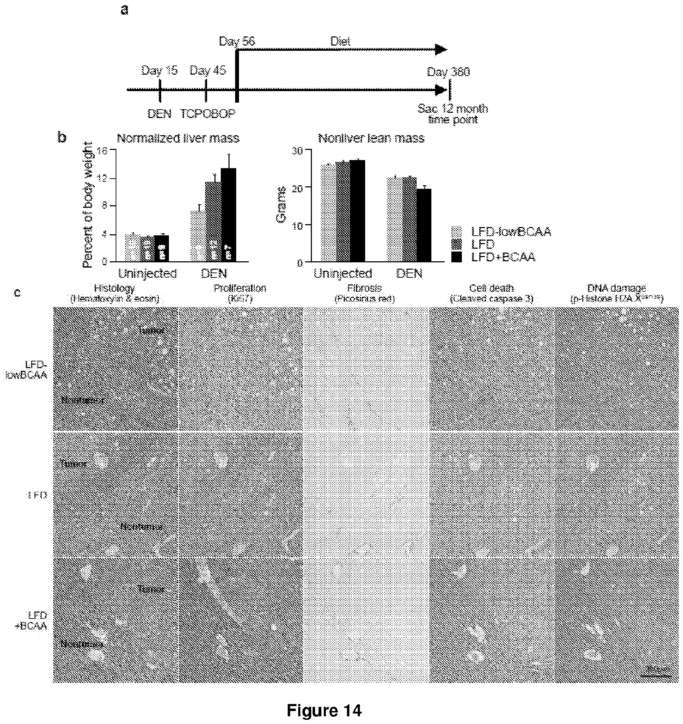

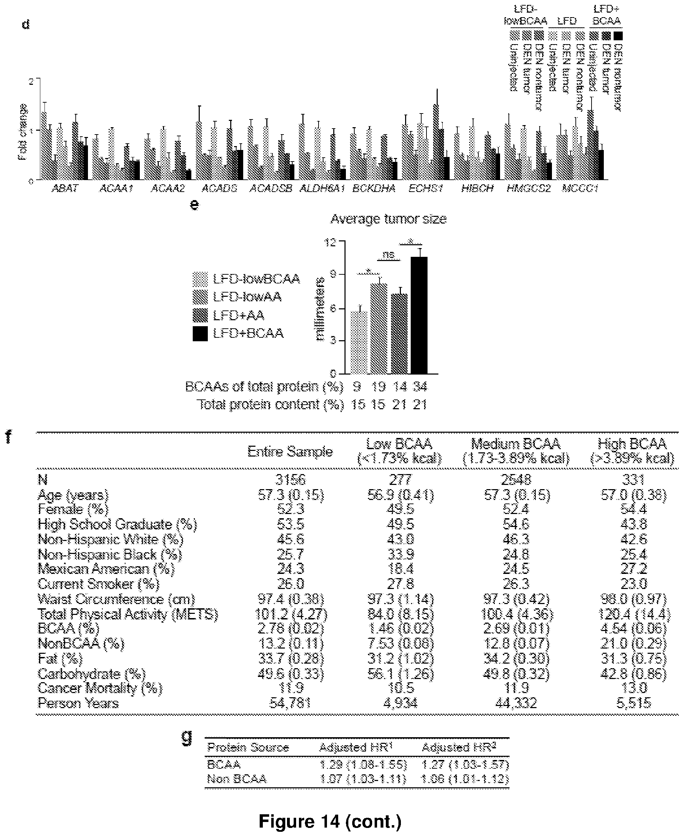

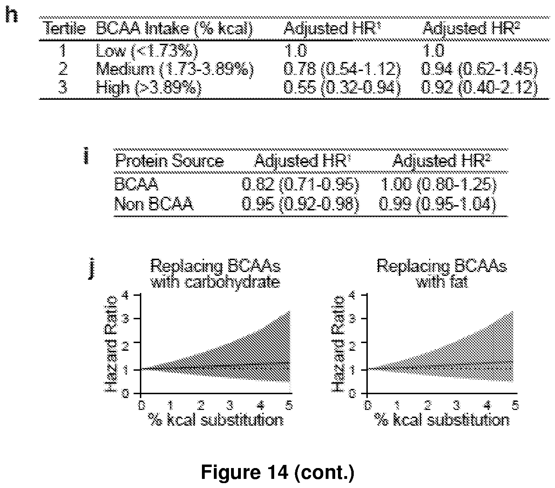

**Figure 14** shows a set of images depicting dietary BCAAs influence tumor burden in rodents and cancer mortality in humans. **Figure 14a** shows a diagram of model timeline. This diagram indicates the feeding timeline of the mice. **Figure 14b** shows a set of bar graphs depicting liver mass and non-liver lean body mass of DEN-injected and uninjected mice fed indicated diets, normalized by body weight. **Figure 14c** shows a set of photographs depicting representative histological liver analyses of DEN-injected mice fed indicated diets. **Figure 14d** shows a bar graph depicting RT-PCR analysis of BCAA catabolic enzymes from nontumor and tumor liver tissues of DEN-injected mice and normal liver tissue of uninjected mice fed indicated diets, normalized to normal liver tissue of uninjected LFD-fed mice. Order of experimental groups in bar graph detailed top right. **Figure 14e** shows a bar graph depicting average tumor sizes of mice 12 months after DEN injection fed indicated diets. Non-BCAA amino acids were adjusted proportionally to match the total protein content of low- or high-BCAA diets. **Figure 14f** shows a table depicting Population characteristics of NHANES III participants, aged 50-66 years old. Data presented as mean (with standard error). **Figure 14g** shows a table depicting change in cancer mortality risk associated with increasing BCAA or non BCAA protein content by 1% kcal in the 50-66 year old cohort. **Figure 14h** shows a table depicting analysis of cancer mortality risk of individuals >66 years-old. Hazard ratios (HR, with 95% confidence interval) based on BCAA intake, adjusted for age, sex, race, total kcal, usual dietary intake, diet change, physical activity, intentional weight loss, waist circumference, smoking, education, and prior diagnosis of cancer, diabetes and cardiovascular disease, additionally adjusted for % kcal from other macronutrients. **Figure 14i** shows a table depicting analysis of cancer mortality risk of individuals >66 years-old. Change in cancer mortality risk associated with increasing BCAA or nonBCAA protein content by 1% kcal. **Figure 14j** shows a graph depicting analysis of cancer mortality risk of individuals >66 years-old. Substitution analysis comparing change in risk when replacing BCAAs with carbohydrate or fat, with the same adjustments as HR except total kcal and % kcal from non-BCAA protein. Data are presented as hazard ratio (solid line) with 95% confidence interval (shaded area). Student's t-test, ns=not significant, *P<0.05. Data are shown as mean ± s.e.

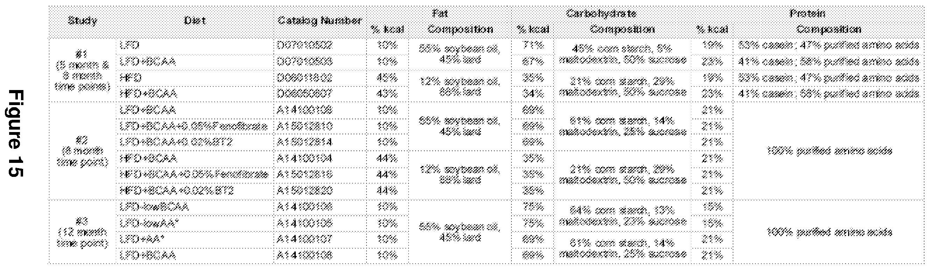

**Figure 15** shows a table depicting summary of the rodent diet composition. Summary of % of kcal, and source of the fat, carbohydrate, and protein used in the rodent studies of Figure 3. Diets with supplemented BCAAs (+BCAA) included an additional 150% of purified leucine, isoleucine, and valine over baseline levels. Diets with restricted BCAAs (lowBCAA) lowered leucine, isoleucine, and valine to 50% of baseline levels. *LFD-lowAA and LFD+AA diets were used to control for any possible effects of total protein content in LFD-lowBCAA and LFD+BCAA diets, respectively. Baseline BCAA levels were maintained, while all other amino acids were adjusted proportionally. In some figures the data were merged and reported as a combined "LFD" group for clarity.

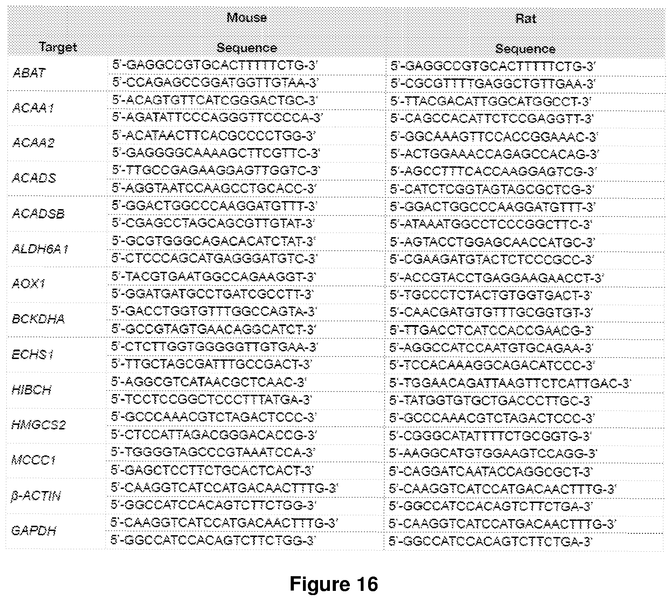

**Figure 16** shows a table depicting list of real-time PCR primer sequences. The primer sequences depicted are used for mouse and rat samples.

### DETAILED DESCRIPTION OF THE PRESENT INVENTION

Tumors display profound changes in cellular metabolism, and previous studies have highlighted the importance of these changes in supporting fundamental aspects of anabolism, including anaplerosis and redox balance. However, whether the metabolic reprogramming in tumors is a general aspect of proliferation, an unintended consequence of aberrant signalling pathways, or a central driver of the oncogenic process is unclear. The inventors have found that an accumulation of branched-chain amino acids (BCAAs) may be used to stimulate pro-oncogenic pathways in carcinogenesis. Transcriptomic analyses of human gastrointestinal cancers identified the suppression of BCAA catabolic enzymes as a common and central feature amongst cancers. The degree of suppression strongly correlated with tumor grade and stage, and was an independent predictor of clinical outcome. Using comprehensive 'multi-omic' analyses on a range of liver tumor models, it was shown that a loss of expression lead to a potent suppression of BCAA catabolic enzyme activity. Rather than entering anabolic flux as biosynthetic precursors, the BCAAs accumulated in tumors and stimulated nutrient-sensing pathways. Modulating BCAA accumulation influenced cell proliferation *in vitro* and the number and size of tumors *in vivo.* Importantly, this mechanism was unique to cancer cells and was not utilized by proliferating cells of the regenerating liver. In summary, the results reveal that metabolic reprogramming in cancer cells can be used to modulate signalling cascades that support tumor development and growth.

Thus, in a first aspect the present disclosure refers to a method of determining or predicting whether a subject has or likely to be having a proliferative disease comprising the step of measuring a level of an acylcarnitine (C5:1) of the subject. In some examples, the method may comprise the step of comparing the acylcarnitine (C5:1) level of the subject as compared to the acylcarnitine (C5:1) level of a control subject, or subjects not having said proliferative disease, wherein a decrease in the acylcarnitine (C5:1) level as compared to the level of the control subject indicates the subject is having, or is likely to have, the proliferative disease. As would be appreciated by the person skilled in the art, the final determination of the outcome or diagnosis of a subject having or likely to have a proliferative disease would be determined by a clinician and the result of the method of the present invention cannot and will not replace the role of a clinician.

The terms "decrease", "reduced", "reduction", "decrease", "removal" or "inhibit" are all used herein to mean a decrease by an amount, when compared to the "control group" or "control subject". However, for avoidance of doubt, "reduced", "reduction" or "decrease", "removal", or "inhibit" means, for example, a decrease by at least about one standard deviation as compared to the control subject, or at least about two standard deviations, or at least about three standard deviations, or by at least 1%, 2%, 3%, 4%, 5%, 8%, or 10% compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (for example, when a target metabolite is not present at all in the sample, compared to a reference sample wherein the target metabolite is present), or any decrease between 10% to 100%, compared to a reference level, or at least about a 2-fold, or at least about a 3-fold, or at least about a 4-fold, or at least about a 5-fold or at least about a 10-fold decrease, or any decrease between 2-fold and 10-fold or greater compared to a reference level. In one example, a decrease of the level of an acylcarnitine (C5:1) equivalent to a decrease of two standard deviations in a subject (when compared to the level of an acylcarnitine (C5:1) from the control group or control subject) may indicate that the subject may have or has a tumor or one or more proliferative diseases or a cancer.

As used herein, the reference to a standard deviation (also denoted as sigma, s.d., or "σ" refers to the statistical difference calculated between a control group (that is a group comprising subject who are known not to suffer from a proliferative disease) and one or more subjects to be analysed, whereby the calculation is performed using known statistical methods, for example, but not limited to, a simple t-test. In calculation, the samples used for calculation may be corrected or may be uncorrected, or may or may not take any predetermined bias into consideration. For example, in the art, the standard deviation is calculated from the mean of a normal distribution (also known as a Gauss distribution or a bell curve), calculated based on the available data. The deviation of data points from this calculated mean of the normal distribution is then termed to be a standard deviation when the data point falls within one sigma range (which is one standard deviation) on either side of the mean of the normal distribution. In the art, a negative standard deviation usually denotes a deviation area to the left of the mean (which is denoted as "0") on the x-axis of the normal distribution graph. Conversely, a positive standard deviation denotes a deviation area to the right of the mean on the x-axis of the normal distribution graph.

As used herein, the term "control subject" refers to a subject known not to have one or more diseases, which include, but are not limited to, proliferative diseases, such as cancers, metabolic diseases, fatty liver disease, hyperglycemia, other pre-disease conditions, and the like. As used herein, the term "control subject" also refers to a subject which is determined to be healthy as defined by clinical standards. The determination whether a subject is healthy or not can be performed, for example, by physical examination, blood tests, and the like. As will be appreciated by a person skilled in the art, a "control subject" can be within the same age and/or gender group as the subject.

As used herein, the term "subject" refers to an animal, mammal, human, including, without limitation, animals classed as bovine, porcine, equine, canine, lupine, feline, murine, ovine, avian, piscine, caprine, corvine, acrine, or delphine. In one example, the "subject" is a human. In one example, the "subject" is a human suspected to have, or to likely have, one or more proliferative diseases.

In one example, the method of present disclosure further comprises measuring a level of at least one branched-chain amino acid (BCAA) of the subject, and wherein an increase in the level of at least one branched-chain amino acid (BCAA) further confirms (or indicates) the subject is having, or is likely to have, the proliferative disease.

The terms "increased", "increase" or "enhance" or "activate" are all used herein to mean an increase by an amount when compared to the "control group" or "control subject". However, for the avoidance of any doubt, the terms "increased", "increase" or "enhance" or "activate" means an increase of at least about one standard deviation compared to the control subject, or at least about two standard deviations, or at least about three standard deviations, or by at least 1%, 2%, 3%, 4%, 5%, 8%, or 10% compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10% to 100% compared to a reference level, or at least about a 2-fold, or at least about a 3-fold, or at least about a 4-fold, or at least about a 5-fold or at least about a 10-fold increase, or any increase between 2-fold and 10-fold or greater compared to a reference level. In one example, an increase of the level of at least one branched-chain amino acid (BCAA) by two standard deviations in a subject (when compared to the level of at least one branched-chain amino acid (BCAA) from the control group or control subject) indicates that the subject may have or has a tumor, or one or more proliferative diseases, or a cancer.

As used herein, the term "branched-chain amino acid (BCAA)" refers to an amino acid having aliphatic side-chains with a "branch". As used herein, the term "branch" may refer to a central carbon atom bound to three or more carbon atoms. In one example, the "BCAA" is a proteinogenic or a non-proteinogenic amino acid. In one example, the "BCAA" is proteinogenic. In one example, the proteinogenic "BCAA" includes, but is not limited to, leucine, isoleucine and valine. In one example, the branched-chain amino acids of the method of the present disclosure includes, but are not limited to, leucine, isoleucine, and valine. In one example, the branched-chain amino acid referred to herein is isoleucine. In another example, the branched-chain amino acid referred to herein is leucine. In one example, the branched-chain amino acid referred to herein is valine. In another example, the branched-chain amino acids referred to herein are isoleucine and leucine. In yet another example, the branched-chain amino acids referred to herein are isoleucine and valine. In a further example, the branched-chain amino acids referred to herein are valine and leucine.

In one example, the method of the present disclosure measures the level of one branched-chain amino acid as defined herein. In one example, the method of the present disclosure measures the level of two branched-chain amino acids. In one example, the method of the present disclosure measures the level of three branched-chain amino acids.

In one example, the methods of the present disclosure further comprises measuring the level of a further amino acid, which includes, but is not limited to, phenylalanine, methionine and asparagine and wherein an increase in the level of at least one of phenylalanine, and/or methionine and/or asparagine further confirms (or indicates) that the subject has or is likely to have, the proliferative disease. In one example, the methods may further comprise measuring the level of a further amino acid, which include, but is not limited to, phenylalanine, methionine and asparagine. In another example, an increase in the level of at least one amino acid phenylalanine, and/or methionine and/or asparagine further confirms (or indicates) that the subject has or is likely to have the proliferative disease. In one example, the amino acid is phenylalanine. In another example, the amino acid is methionine. In yet another example, the amino acid is asparagine. In a further example, the amino acids are methionine and phenylalanine. In another example, the amino acids are methionine and asparagine. In yet another example, the amino acids are phenylalanine and asparagine. In one example, the methods of the present disclosure further comprise measuring the level of the amino acid which include, but is not limited to, phenylalanine, methionine and asparagine and wherein an increase in the level of phenylalanine further confirms (or indicates) that the subject has or is likely to have the proliferative disease. In one example, the methods of the present disclosure further comprises measuring the level of the amino acid which includes, but is not limited to, phenylalanine, methionine and asparagine and wherein an increase in the level of methionine further confirms (or indicates) that the subject has or is likely to have the proliferative disease. In one example, the methods of the present disclosure further comprise measuring the level of the amino acid which includes, but is not limited to, phenylalanine, methionine and asparagine and wherein an increase in the level of asparagine further confirms (or indicates) that the subject has or is likely to have the proliferative disease. In one example, the methods of the present disclosure further comprise measuring the level of the amino acid which includes, but is not limited to, phenylalanine, methionine and asparagine and wherein an increase in the level of phenylalanine and methionine further confirms (or indicates) that the subject has or is likely to have the proliferative disease. In one example, the methods of the present disclosure further comprise measuring the level of the amino acid which includes, but is not limited to, phenylalanine, methionine and asparagine and wherein an increase in the level of phenylalanine and asparagine further confirm (or indicate) that the subject has or is likely to have the proliferative disease. In one example, the methods of the present disclosure further comprise measuring the level of the amino acid which include, but is not limited to, phenylalanine, methionine and asparagine and wherein an increase in the level of methionine and asparagine further confirm (or indicate) that the subject has or is likely to have the proliferative disease. In one example, the methods of the present disclosure further comprise measuring the level of the amino acid which include, but is not limited to, phenylalanine, methionine and asparagine and wherein an increase in the level of phenylalanine and methionine and asparagine further confirms (or indicates) that the subject is having or is likely to have the proliferative disease.

In one example, the method of the present disclosure is used to determine or predict whether a subject has or is likely to have a proliferative disease. In one example, the proliferative disease is cancer. In one example, the cancer predictable or detectable by the method of present disclosure includes, but is not limited to, liver cancer (such as hepatocellular carcinoma, and cholangiocarcinoma), head and neck squamous cell carcinoma, kidney cancer (such as kidney renal clear cell carcinoma, kidney papillary cell carcinoma, and kidney chromophobe renal cell carcinoma), colon and rectum adenocarcinoma, breast carcinoma, lung carcinoma, thyroid carcinoma, stomach adenocarcinoma, esophageal carcinoma and the like.

In one example, the method of the present disclosure is used to measure a level of an acylcarnitine (C5:1) of the subject, wherein the level of acylcarnitine (C5:1) or amino acid is measured in a biological sample obtained from the subject. In one example, the biological sample for measuring the level of an acylcarnitine (C5:1) of the subject is a tissue biopsy.

Free BCAAs are typically found in the cytoplasm of a cell. A person skilled in the art would therefore appreciate that in order for a biological sample to be used in accordance with the invention as disclosed herein, it would need to contain cells. However, these cells may or may not contain BCAAs, depending on the type and state of the cell. Having said that, a biological sample obtained from a tumor, that is a tumor sample, is typically understood to comprises cancerous cells. In an example, a tumor sample that comprises too many non-cancerous cells, such as for example blood vessels, immune cells, and the like, may produce erroneous results compared to a tumor sample which comprises cancerous cells. Therefore, in one example, the biological sample is a complete, whole (that is not dissected or resected) tumor. In another example, the biological sample is a representative tumor biopsy, which can be confirmed by examining a slice obtained from a whole tumor by histology. In one example, the biological sample is substantially free of non-cancerous cells. In one example, the method comprises the step of collecting a sample suspected of containing a tumor, part of a tumor, or cancerous cells. In one example, the method comprises the step of analyzing the sample. In one example, the analyzing step comprises preparing a cell extract. As used herein, the term "substantially free" refers to an object species wherein the predominant species, for example, a particular cell type in a sample, is present. For example, on a molar basis, the predominant species is more abundant than any other individual species in the composition. In regards to biological samples, a substantially pure sample will comprise more than about 80 percent of all individual species present in the sample, or more than about 85%, about 90%, about 95%, and about 99%. Ideally, the object species is purified to essential homogeneity, meaning that any and all contaminant species cannot be detected in the composition by conventional detection methods, wherein the composition consists essentially of a single macromolecular species.

In one example, the biological sample of the method of present disclosure includes, but is not limited to, a lung tissue biopsy, a breast tissue biopsy, a colorectal tissue biopsy, an esophageal tissue biopsy, a gastric tissue biopsy, a thyroid tissue biopsy, a head or neck tissue biopsy, a kidney tissue biopsy, a liver tissue biopsy, and the like.

In one example, the level of the acylcarnitine (C5:1) and/or amino acid in a sample used for the method of the present disclosure is measured using methods/devices known in the art. In one example, the level of the acylcarnitine (C5:1) in a sample used for the method of the present disclosure is measured using methods/devices known in the art. In one example, the level of the amino acid in a sample used for the method of the present disclosure is measured using methods/devices known in the art. In one example, the level of the acylcarnitine (C5:1) and amino acid in a sample used for the method of the present disclosure is measured using methods/devices known in the art. In one example, the methods/devices include, but are not limited to, HPLC-MS/MS and Nuclear Magnetic Resonance (NMR). In one example, the comparing of the level of acylcarnitine (C5:1) and/or amino acid for the method of the present disclosure is performed using computer based analysis. In one example, the comparing of the level of acylcarnitine (C5:1) for the method of the present disclosure is performed using computer based analysis. In one example, the comparing of the level of amino acid for the method of the present disclosure is performed using computer based analysis. In one example, the comparing of the level of acylcarnitine (C5:1) and amino acid for the method of the present disclosure is performed using computer based analysis.

In one example, the method of the present disclosure may further comprising administering a pharmaceutically effective amount of a branched-chain amino acid catabolism enhancer and/or a pharmaceutically effective amount of a branched-chain α-ketoacid dehydrogenase complex (BCKDC) kinase inhibitor (BDK inhibitor) and/or an effective amount of meal replacement comprising low level of branched-chain amino acid (BCAA) into the subject in need thereof. In one example, the method of the present disclosure may further comprising administering a pharmaceutically effective amount of a branched-chain amino acid catabolism enhancer into the subject in need thereof. In one example, the method of the present disclosure may further comprising administering a pharmaceutically effective amount of a branched-chain α-ketoacid dehydrogenase complex (BCKDC) kinase inhibitor (BDK inhibitor) into the subject in need thereof. In one example, the method of the present disclosure may further comprising administering an effective amount of meal replacement comprising low level of branched-chain amino acid (BCAA) into the subject in need thereof. In one example, the method of the present disclosure may further comprising administering a pharmaceutically effective amount of a branched-chain amino acid catabolism enhancer and a pharmaceutically effective amount of a branched-chain α-ketoacid dehydrogenase complex (BCKDC) kinase inhibitor (BDK inhibitor) and an effective amount of meal replacement comprising low level of branched-chain amino acid (BCAA) into the subject in need thereof.

In one aspect the present disclosure refers to a method of determining or predicting whether a subject has or is likely to have a proliferative disease comprising: a. measuring a level of at least one branched-chain amino acid (BCAA) of the subject. In some examples, the method also comprises b. comparing the branched-chain amino acid level of the subject as compared to the branched-chain amino acid level of a control subject or subjects not having said proliferative disease, wherein the branched-chain amino acid level in excess of the branched-chain amino acid level of the control subject indicates the subject is having or is likely to have the proliferative disease. As would be appreciated by the person skilled in the art, the final determination of the outcome or diagnosis of a subject having or likely to have a proliferative disease would be determined by a clinician and the result of the method of the present invention cannot and will not replace the role of a clinician.

As used herein, the term "control subject" refers to a subject known not to have one or more diseases which include, but are not limited to, proliferative diseases, such as cancers, metabolic diseases, fatty liver disease, hyperglycemia, other pre-disease conditions, and the like. As used herein, the term "control subject" also refers to a subject which is determined to be healthy as defined by clinical standards. The determination whether a subject is healthy or not can be performed for example by, but are not limited to, physical examination, blood tests, and the like. As will be appreciated by a person skilled in the art, a "control subject" can be within the same age and/or gender group as the subject.

As used herein, the term "level in excess" or "in excess" refers to the observation that a compound, a metabolite, a peptide, a protein, and the like are present at an amount exceeding the amount generally found in a control subject. For the avoidance of any doubt, the compound, the metabolite, the peptide, the protein and the like are present, for example, in an increase of at least about one standard deviation compared to the control subject, or at least about two standard deviations, or at least about three standard deviations, or by at least 1%, 2%, 3%, 4%, 5%, 8%, or 10% compared to a reference level, or at least 20%, or at least 30%, or at least 40%, or at least 50%, or at least 60%, or at least 70%, or at least 80%, or at least 90%, or at least 100%, an increase or any increase between 10% to 100% compared to a reference level, or at least about a 2-fold, or at least about a 3-fold, or at least about a 4-fold, or at least about a 5-fold or at least about a 10-fold increase, or any increase between 2-fold and 10-fold or greater compared to a reference level. In one example, an increase of the level of at least one branched-chain amino acid (BCAA) by two standard deviations in a subject (when compared to the level of at least one branched-chain amino acid (BCAA) from the control group or control subject) indicates that the subject may have or has a tumor or one or more proliferative diseases or a cancer.

In one example, the method of present disclosure further comprises measuring a level of acylcarnitine (C5:1) of the subject, wherein a decrease in the level of acylcarnitine (C5:1) compared to the control further confirms (or indicates) that the subject has or is likely to have the proliferative disease.

In one example, the method of the present disclosure measures the level of one branched-chain amino acid as defined herein. In one example, the method of the present disclosure measures the level of two branched-chain amino acids. In one example, the method of the present disclosure measures the level of three branched-chain amino acids.

In one example, the methods of the present disclosure further comprises measuring the level of a further amino acid which include, but is not limited to, phenylalanine, methionine and asparagine and wherein an increase in the level of at least one of phenylalanine, and/or methionine and/or asparagine may further confirm (or indicate) the subject has or is likely to have the proliferative disease. In one example, the methods of the present disclosure further comprise measuring the level of the amino acid which include, but is not limited to, phenylalanine, methionine and asparagine and wherein an increase in the level of phenylalanine further confirms (or indicates) that the subject has or is likely to have the proliferative disease. In one example, the methods of the present disclosure further comprise measuring the level of the amino acid which includes, but is not limited to, phenylalanine, methionine and asparagine and wherein an increase in the level of methionine further confirms (or indicates) that the subject has or is likely to have the proliferative disease. In one example, the methods of the present disclosure further comprise measuring the level of the amino acid which includes, but is not limited to, phenylalanine, methionine and asparagine and wherein an increase in the level of asparagine further confirms (or indicates) that the subject is having or is likely to have the proliferative disease. In one example, the methods of the present disclosure further comprises measuring the level of the amino acid which includes, but is not limited to, phenylalanine, methionine and asparagine and wherein an increase in the level of phenylalanine and methionine further confirms (or indicates) that the subject is having or is likely to have the proliferative disease. In one example, the methods of the present disclosure further comprises measuring the level of the amino acid which includes, but is not limited to, phenylalanine, methionine and asparagine and wherein an increase in the levels of phenylalanine and asparagine further confirms (or indicates) that the subject has or is likely to have the proliferative disease. In one example, the methods of the present disclosure further comprises measuring the level of the amino acid which includes, but is not limited to, phenylalanine, methionine and asparagine and wherein an increase in the level of methionine and asparagine further confirms (or indicates) the subject has or is likely to have the proliferative disease. In one example, the methods of the present disclosure further comprises measuring the level of the amino acid which includes, but is not limited to, phenylalanine, methionine and asparagine and wherein an increase in the level of phenylalanine and methionine and asparagine further confirms (or indicates) that the subject has or is likely to have the proliferative disease.

In one example, the method of the present disclosure is used to determine or predict whether a subject has or likely to have a proliferative disease, wherein the proliferative disease may be cancer. In one example, the cancer predictable or detectable by the method of present disclosure includes but is not limited to, liver cancer (such as hepatocellular carcinoma, and cholangiocarcinoma), head and neck squamous cell carcinoma, kidney cancer (such as kidney renal clear cell carcinoma, kidney papillary cell carcinoma, and kidney chromophobe renal cell carcinoma), colon and rectum adenocarcinoma, breast carcinoma, lung carcinoma, thyroid carcinoma, stomach adenocarcinoma, esophageal carcinoma and the like.

In one example, the method of the present disclosure is used to measure a level of at least one branched-chain amino acid (BCAA) of the subject, wherein the level of acylcarnitine (C5:1) or amino acid is measured in a biological sample obtained from the subject. In one example, the biological sample for measuring the level of an acylcarnitine (C5:1) of the subject may be a tissue biopsy. In one example, the biological sample is substantially free of non-cancerous cells. In one example, the method comprises the step of collecting a sample suspected of containing a tumor, part of a tumour or cancerous cells.

In one example, the biological sample of the method of present disclosure includes but is not limited to a lung tissue biopsy, a breast tissue biopsy, a colorectal tissue biopsy, an esophageal tissue biopsy, a gastric tissue biopsy, a thyroid tissue biopsy, a head or neck tissue biopsy, a kidney tissue biopsy, a liver tissue biopsy, and the like.

In one example, the level of the acylcarnitine (C5:1) and/or amino acid in a sample used for the method of the present disclosure is measured using methods/devices known in the art. In one example, the level of the acylcarnitine (C5:1) in a sample used for the method of the present disclosure is measured using methods/devices known in the art. In one example, the level of the amino acid in a sample used for the method of the present disclosure is measured using methods/devices known in the art. In one example, the level of the acylcarnitine (C5:1) and amino acid in a sample used for the method of the present disclosure is measured using methods/devices known in the art. In one example, the methods/devices include, but are not limited to, HPLC-MS/MS and Nuclear Magnetic Resonance (NMR).

In one example, the comparing of the level of acylcarnitine (C5:1) and/or amino acid for the method of the present disclosure is performed using computer based analysis. In one example, the comparing of the level of acylcarnitine (C5:1) for the method of the present disclosure is performed using computer based analysis. In one example, the comparing of the level of amino acid for the method of the present disclosure is performed using computer based analysis. In one example, the comparing of the level of acylcarnitine (C5:1) and amino acid for the method of the present disclosure is performed using computer based analysis.

In one example, the method of the present disclosure further comprises administering a pharmaceutically effective amount of a branched-chain amino acid catabolism enhancer and/or a pharmaceutically effective amount of a branched-chain α-ketoacid dehydrogenase complex (BCKDC) kinase inhibitor (BDK inhibitor) and/or an effective amount of meal replacement comprising low levels of branched-chain amino acid (BCAA) to the subject in need thereof. In one example, the method of the present disclosure mfurther comprises administering a pharmaceutically effective amount of a branched-chain amino acid catabolism enhancer to the subject in need thereof. In one example, the method of the present disclosure further comprises administering a pharmaceutically effective amount of a branched-chain α-ketoacid dehydrogenase complex (BCKDC) kinase inhibitor (BDK inhibitor) to the subject in need thereof. In one example, the method of the present disclosure further comprises administering an effective amount of meal replacement comprising low level of branched-chain amino acid (BCAA) to the subject in need thereof. In one example, the method of the present disclosure further comprises administering a pharmaceutically effective amount of a branched-chain amino acid catabolism enhancer and a pharmaceutically effective amount of a branched-chain α-ketoacid dehydrogenase complex (BCKDC) kinase inhibitor (BDK inhibitor) and an effective amount of meal replacement comprising low levels of branched-chain amino acid (BCAA) to the subject in need thereof.

In one aspect, the present disclosure refers to a method of predicting the likelihood of a subject surviving cancer (prognosis of a subject) comprising: a. measuring a level of branched amino acids (BCAA) of the subject. In some examples, the method also comprises b. comparing and/or correlating the level measured in (a) to a standard level of branched amino acids, wherein the degree of deviation above the level of the standard indicates the degree of severity of the outcome.

As used herein, the term "standard level" or "level of the standard" may refer to the average level of at least one branched amino acids (BCAA) in a healthy group of subjects. "Standard level" or "level of the standard" of at least one branched amino acid (BCAA) can be determined for example by (1) collecting samples from a clinically healthy groups of people based on age and gender, (2) quantifying the BCAA levels from the healthy groups, (3) establishing a confidence interval of 95% and 99% for the average level of branched amino acids (BCAA) in a healthy group of subjects, and (4) defining abnormality for those measurements outside of the range of the confidence interval. As appreciated by a person skilled in the art, the determination of "standard level" or "level of the standard" can also be similar to previously established practices of determining liver functions using liver enzymes (which may include but are not limited to SGOT, SGPT, and the like).

As used herein, the term "degree of deviation" refers to the amount of variation present within a data population. In one example, the degree of deviation refers to the differences between the level of at least one branched amino acid (BCAA) measured from the subject and the level of at least one branched amino acid (BCAA) measured from the control subject. Thus the deviation referred to in this example is the (positive or negative) difference in value measured for a branched-chain amino acid in the subject compared to the value obtained for the same branched-chain amino acid in the control subject. The "degree of deviation" or a particular target molecule or marker, for example, a particular amino acid or, in one example, a branched-chain amino acid, can be established by collecting and accumulating data from both healthy and different cancer patient groups in order to establish a diagnosis window (which may have confidence interval of 95% or 99%). In this case, a "higher or high degree of deviation" refers to a degree of deviation of equal to or higher than three standard deviations. A "lower or low degree of deviation" refers to a degree of deviation between two to three standard deviations.

In one example, higher degree of deviation from the level of the standard is observed in (b). In an example wherein higher degree of deviation from the level of the standard is observed in (b), the outcome is poorer (which means a poor outcome, or negative, or unfavorable survival). In contrast, in another example, lower degree of deviation from the level of the standard is observed in (b). In an example wherein lower degree of deviation from the level of the standard is observed in (b), the outcome is better (which means a good prognosis, positive, or favorable survival).

In one example, the standard level of the method of the present disclosure is a predetermined level obtained from subjects known to have a good prognosis. The standard level can be determined by creating an average value based on a particular characteristic present in a group of healthy subjects. Such an average value can be generated by collecting and analyzing data from healthy patients and patients of relevant diseases (which can be further divided into groups based on diseases, gender, race, age, etc.). In one example, the average value or normal range is determined based on a group of healthy subjects based on age and gender. In another example, the average value or normal range is determined based on a group of healthy subjects based on age, gender and race). This average value or normal range can be a 95% confidence interval or a 99% confidence interval, depending on the stringency required of the diagnosis: for example, a confidence interval of 95% is used so as not to under-diagnose. A confidence interval of 99% is used, for example, to reduce potential false positive results, which are results that appear to be positive, but at actually negative for the characteristic in questions and only appear to be a positive result due to handling errors and the like. In one example, it is possible to use standard deviations as described above as a way to define low and high degree of deviations. It is of note that the terms average and mean are used interchangeably in the art. In the art of statistics, it is noted that while average and mean may refer to the same concept, the determination of the two is different. An average in statistics refers to a grouping of data points that fall around the centre of the normal distribution curve, for example. A mean is the mathematical (arithmetic) calculation of the sum of all values (for example, n) taken into consideration and divided by the total number of values (which would also be n). In other words, an average and an arithmetic mean are synonymous. However, a statistical mean can also be defined using geometric or harmonic means, and would then vary from the average as discussed above.

In one example, the method of the present disclosure measures the level of one branched-chain amino acid. In one example, the method of the present disclosure measures the level of two branched-chain amino acid. In one example, the method of the present disclosure measures the level of three branched-chain amino acids.

In one example, the method of the present disclosure is used to predict the likelihood of a subject surviving proliferative disease, wherein the proliferative disease is cancer. In one example, the cancer predictable or detectable by the method of present disclosure includes, but is not limited to, liver cancer (such as hepatocellular carcinoma, and cholangiocarcinoma), head and neck squamous cell carcinoma, kidney cancer (such as kidney renal clear cell carcinoma, kidney papillary cell carcinoma, and kidney chromophobe renal cell carcinoma), colon and rectum adenocarcinoma, breast carcinoma, lung carcinoma, thyroid carcinoma, stomach adenocarcinoma, esophageal carcinoma, and the like.

In one example, the method of the present disclosure is used to measure a level of at least one branched-chain amino acid (BCAA) of the subject, wherein the level of acylcarnitine (C5:1) or amino acid is measured in a biological sample obtained from the subject. In one example, the biological sample for measuring the level of an acylcarnitine (C5:1) of the subject is a tissue biopsy.

In one example, the biological sample of the method of present disclosure includes but is not limited to a lung tissue biopsy, a breast tissue biopsy, a colorectal tissue biopsy, an esophageal tissue biopsy, a gastric tissue biopsy, a thyroid tissue biopsy, a head or neck tissue biopsy, a kidney tissue biopsy, a liver tissue biopsy, and the like.

In one example the level of the amino acid in a sample used for the method of the present disclosure is measured using methods/devices known in the art. In one example, the methods/devices may include but are not limited to HPLC-MS/MS and Nuclear Magnetic Resonance (NMR).

In one example, the comparing of the level of amino acid for the method of the present disclosure is performed using computer based analysis.

In one example, the prognosis of a subject using the method of the present disclosure is poor or negative. In one example, the subject with poor outcomes/prognosis (negative survival) are subjects having high-grade cancer (such as having a tumor with a grade of 3 and/or 4), and/or likelihood of disease recurrence or progression, and/or not surviving more than 1, or 2, or 3, or 4, or 5 years. In one example, the subject with poor outcomes/prognosis (negative survival) are subjects having high-grade cancer (such as having a tumor with a grade of 3 and/or 4). In one example, the subjects with poor outcomes/prognosis (negative survival) are subjects in whom the likelihood of disease recurrence or progression is present. In one example, the subject with poor outcomes/prognosis (negative survival) are subjects who do not survive more than 1, or 2, or 3, or 4, or 5 years. In one example, the subject with poor outcomes/prognosis (negative survival) are subjects having high-grade cancer (such as having a tumor with a grade of 3 and/or 4), and likelihood of disease recurrence or progression. In one example, the subject with poor outcomes/prognosis (negative survival) are subjects having high-grade cancer (such as having a tumor with a grade of 3 and/or 4) and not surviving for more than 1, or 2, or 3, or 4, or 5 years. In one example, the subject with poor outcomes/prognosis (negative survival) are subjects having the likelihood of disease recurrence or progression and not surviving for more than 1, or 2, or 3, or 4, or 5 years. In one example, the subject with poor outcomes/prognosis (negative survival) are subjects having high-grade cancer (such as having a tumor with a grade of 3 and/or 4), and likelihood of disease recurrence or progression, and not surviving for more than 1, or 2, or 3, or 4, or 5 years.

As would be appreciated by a person skilled in the art, "tumor grading" or "tumor grade" or "grade of tumor" refers to the description of a tumor based on how abnormal the cells from tumor samples and the tumor tissue appear under a microscope, for example under histological analysis. The "tumor grade" or "tumor grading" or "grade of tumor" may also be an indicator of how quickly a tumor is likely to grow or spread. It is generally understood that cells from tumor samples and the organization of tumor tissue that are close (or appear similar) to normal cells and tissues are considered to be "well-differentiated", and may grow or spread at a slower rate compared to abnormal looking cells from the tumor and tumor tissues (i.e. "undifferentiated" or "poorly differentiated"). The grading system for "tumor grade" or "tumor grading" or "grade of tumor" generally comprises five different grades, which are GX (which means the grade cannot be assessed or undetermined grade), G1 (which means grade 1, well differentiated cells and/or tissues, low grade), G2 (which means grade 2, moderately differentiated cells and/or tissues, intermediate grade), G3 (which means, grade 3, poorly differentiated cells and/or tissues, high grade), and G4 (which means grade 4, undifferentiated cells and/or tissues, high grade).

In one example, poor outcome/prognosis (negative survival) indicates a reduced likelihood of survival over 7 years. In one example, poor outcome/prognosis (negative survival) indicates a reduced likelihood of survival over 6 years. In one example, poor outcome/prognosis (negative survival) indicates a reduced likelihood of survival over 5 years. In one example, poor outcome/prognosis (negative survival) indicates a reduced likelihood of survival over 4 years. In one example, poor outcome/prognosis (negative survival) indicates a reduced likelihood of survival over 3 years. In one example, poor outcome/prognosis (negative survival) indicates a reduced likelihood of survival over 2 years. In one example, poor outcome/prognosis (negative survival) indicates a reduced likelihood of survival over 1 year.

In one example, good outcome/prognosis indicates a likelihood of survival of more than 7 years, and/or recurrence-free survival (of more than 7 years). In one example, good outcome/prognosis indicates a likelihood of survival of more than 6 years, and/or recurrence-free survival (of more than 6 years). In one example, good outcome/prognosis indicates a likelihood of survival of more than 5 years, and/or recurrence-free survival (of more than 5 years). In one example, good outcome/prognosis indicates a likelihood of survival of more than 4 years, and/or recurrence-free survival (of more than 4 years). In one example, good outcome/prognosis indicates a likelihood of survival of more than 3 years, and/or recurrence-free survival (of more than 3 years). In one example, good outcome/prognosis indicates a likelihood of survival of more than 2 years, and/or recurrence-free survival (of more than 2 years). In one example, good outcome/prognosis indicates a likelihood of survival of more than 1 year, and/or recurrence-free survival (of more than 1 year).

As would be appreciated by the person skilled in the art, the final determination of the outcome or diagnosis of a cancer patient is determined by a clinician and the result of the method of the present invention cannot or will not replace the role of a clinician. In general, it would be understood that the outcome also depends on traditional variables, such as, but not limited to underlying diseases and risk factors, time of diagnosis, tumor grade, tumor stage, quality of care, approved and available treatment options and the like.

In one example, for hepatocellular carcinoma patients, it is understood that the outcome or diagnosis depends on various variables such as, for example, the geographical location of the patient, which in turn reflects underlying factors such as those factors driven by, for example, HVB/HCV, alcohol, alfatoxin, NASH/NAFLD, and the like, quality care, approved and available treatment options, and the like.

In one example, the method of the present disclosure further comprises administering a pharmaceutically effective amount of a branched-chain amino acid catabolism enhancer and/or a pharmaceutically effective amount of a branched-chain α-ketoacid dehydrogenase complex (BCKDC) kinase inhibitor (BDK inhibitor) and/or an effective amount of meal replacement comprising low level of branched-chain amino acid (BCAA) to the subject in need thereof. In one example, the method of the present disclosure further comprises administering a pharmaceutically effective amount of a branched-chain amino acid catabolism enhancer to the subject in need thereof. In one example, the method of the present disclosure further comprises administering a pharmaceutically effective amount of a branched-chain α-ketoacid dehydrogenase complex (BCKDC) kinase inhibitor (BDK inhibitor) to the subject in need thereof. In one example, the method of the present disclosure further comprises administering an effective amount of meal replacement comprising low level of branched-chain amino acid (BCAA) to the subject in need thereof. In one example, the method of the present disclosure further comprises administering a pharmaceutically effective amount of a branched-chain amino acid catabolism enhancer and a pharmaceutically effective amount of a branched-chain α-ketoacid dehydrogenase complex (BCKDC) kinase inhibitor (BDK inhibitor) and an effective amount of meal replacement comprising low level of branched-chain amino acid (BCAA) to the subject in need thereof.

In one aspect, the present disclosure refers to a method of determining or predicting whether a subject is having or likely to have a proliferative disease comprising: a. measuring a level of branched amino acids (BCAA) catabolic enzymes of the subject. In some examples, the method comprises b. comparing the level measured in (a) to the level of a branched amino acids (BCAA) catabolic enzymes of a control subject (or subjects) not having said proliferative disease, wherein a decreased branched amino acids (BCAA) catabolic enzymes level as compared to the branched amino acids (BCAA) catabolic enzymes level of the control subject indicates the subject has or is likely to have the proliferative disease. As would be appreciated by the person skilled in the art, the final determination of the outcome or diagnosis of a subject having or likely to have a proliferative disease would be determined by a clinician and the result of the method of the present invention cannot and will not replace the role of a clinician.

The terms "decrease", "reduced", "reduction", "decrease", "removal" or "inhibit" are all used herein to mean a decrease by an amount when compared to the "control group" or "control subject". However, for avoidance of doubt, "reduced", "reduction" or "decrease", "removal", or "inhibit" means a decrease by at least about one standard deviation compared to the control subject, or at least about two standard deviations, or at least about three standard deviations, or by at least 1%, 2%, 3%, 4%, 5%, 8%, or 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level as compared to a reference sample), or any decrease between 10% to 100% as compared to a reference level. In one example, a decrease of the level of at least one branched amino acids (BCAA) catabolic enzyme by two standard deviations in a subject (when compared to the level of at least one branched amino acids (BCAA) catabolic enzyme from the control group or control subject) may indicate that the subject may have or has a tumor or one or more proliferative diseases or a cancer.

As used herein, the term "control subject" refers to a subject known not to have one or more diseases which include, but are not limited to, proliferative diseases, such as cancers, metabolic diseases, fatty liver disease, hyperglycemia, other pre-disease conditions, and the like. As used herein, the term "control subject" also refers to a subject who is determined to be healthy, as defined by clinical standards. The determination whether a subject as healthy or not can be performed, for example, by physical examination, blood tests, and the like. As will be appreciated by a person skilled in the art, a "control subject" can be within the same age and/or gender group as the subject.

As used herein, the term "subject" refers to an animal, mammal, human, including, without limitation, animals classed as bovine, porcine, equine, canine, lupine, feline, murine, ovine, avian, piscine, caprine, corvine, acrine, or delphine. In one example, the "subject" is a human. In one example, the "subject" is a human having, or suspected to have or to likely have, one or more proliferative diseases.

As used herein, the term "catabolic enzyme" refers to enzymes that play a role in destructive metabolism or the breakdown of complex molecules in living organisms to form simpler ones, together with the release of energy. As such, the term "branched-chain amino acids (BCAA) catabolic enzymes" refers to enzymes that play a role in destructive metabolism or the breakdown of branched-chain amino acid (BCAA).

In one example, the branched-chain amino acids catabolic enzymes of the method of the present disclosure include, but are not limited to, ABAT (4-aminobutyrate aminotransferase), ACAA1 (acetyl-CoA acyltransferase 1), ACAA2 (acetyl-CoA acyltransferase 2), ACAD8 (acyl-CoA dehydrogenase family member 8), ACADM (acyl-CoA dehydrogenase, C-4 to C-12 straight chain), ACADS (acyl-CoA dehydrogenase, C-2 to C-3 short chain), ACADSB (acyl-CoA dehydrogenase, short/branched chain), ACAT1 (acetyl-CoA acetyltransferase 1), ACAT2 (acetyl-CoA acetyltransferase 2), ALDH1B1 (aldehyde dehydrogenase 1 family member B1), ALDH2 (aldehyde dehydrogenase 2 family (mitochondrial)), ALDH3A2 (aldehyde dehydrogenase 3 family member A2), ALDH6A1 (aldehyde dehydrogenase 6 family member A1), ALDH9A1 (aldehyde dehydrogenase 9 family member A1), AOX1 (aldehyde oxidase 1), AUH (AU RNA binding protein/enoyl-CoA hydratase), BCKDHA (branched chain keto acid dehydrogenase E1, alpha polypeptide), BCKDHB (branched chain keto acid dehydrogenase E1, beta polypeptide), DBT (dihydrolipoamide branched chain transacylase E2), DLD (dihydrolipoamide dehydrogenase), ECHS1 (enoyl-CoA hydratase, short chain, 1, mitochondrial), EHHADH (enoyl-CoA, hydratase/3-hydroxyacyl CoA dehydrogenase), HADH (hydroxyacyl-CoA dehydrogenase), HADHA (hydroxyacyl-CoA dehydrogenase/3-ketoacyl-CoA thiolase/enoyl-CoA hydratase (trifunctional protein), alpha subunit), HADHB (hydroxyacyl-CoA dehydrogenase/3-ketoacyl-CoA thiolase/enoyl-CoA hydratase (trifunctional protein), beta subunit), HIBADH (3-hydroxyisobutyrate dehydrogenase), HIBCH (3-hydroxyisobutyryl-CoA hydrolase), HMGCL (3-hydroxymethyl-3-methylglutaryl-CoA lyase), HMGCS2 (3-hydroxy-3-methylglutaryl-CoA synthase 2), HSD17B10 (hydroxysteroid (17-beta) dehydrogenase 10), IVD (isovaleryl-CoA dehydrogenase), MCCC1 (methylcrotonoyl-CoA carboxylase 1), MCCC2 (methylcrotonoyl-CoA carboxylase 2), MCEE (methylmalonyl-CoA epimerase), MUT (methylmalonyl-CoA mutase), OXCT1 (3-oxoacid CoA-transferase 1), PCCA (propionyl-CoA carboxylase alpha subunit), PCCB (propionyl-CoA carboxylase beta subunit), and the like.

The branched-chain amino acids catabolic enzymes of the method of the present disclosure may also be referred to using the following Gene ID:

| Official Symbol | Gene ID | Official Full Name |
|---|---|---|
| ABAT | 18 | 4-aminobutyrate aminotransferase |
| ACAA1 | 30 | acetyl-CoA acyltransferase 1 |
| ACAA2 | 10449 | acetyl-CoA acyltransferase 2 |
| ACAD8 | 27034 | acyl-CoA dehydrogenase family member 8 |
| ACAD M | 34 | acyl-CoA dehydrogenase, C-4 to C-12 straight chain |
| ACADS | 35 | acyl-CoA dehydrogenase, C-2 to C-3 short chain |
| ACADS B | 36 | acyl-CoA dehydrogenase, short/branched chain |
| ACAT1 | 38 | acetyl-CoA acetyltransferase 1 |
| ACAT2 | 39 | acetyl-CoA acetyltransferase 2 |
| ALDH1 B1 | 219 | aldehyde dehydrogenase 1 family member B 1 |
| ALDH2 | 217 | aldehyde dehydrogenase 2 family (mitochondrial) |
| ALDH3 A2 | 224 | aldehyde dehydrogenase 3 family member A2 |
| ALDH6 A1 | 4329 | aldehyde dehydrogenase 6 family member A1 |
| ALDH9 A1 | 223 | aldehyde dehydrogenase 9 family member A1 |
| AOX1 | 316 | aldehyde oxidase 1 |
| AUH | 549 | AU RNA binding protein/enoyl-CoA hydratase |
| BCKDH A | 593 | branched chain keto acid dehydrogenase E1, alpha polypeptide |
| BCKDH B | 594 | branched chain keto acid dehydrogenase E1, beta polypeptide |
| DBT | 1629 | dihydrolipoamide branched chain transacylase E2 |
| DLD | 1738 | dihydrolipoamide dehydrogenase |
| ECHS1 | 1892 | enoyl-CoA hydratase, short chain, 1, mitochondrial |
| EHHAD H | 1962 | enoyl-CoA, hydratase/3-hydroxyacyl CoA dehydrogenase |
| HADH | 3033 | hydroxyacyl-CoA dehydrogenase |
| HADHA | 3030 | hydroxyacyl-CoA dehydrogenase/3-ketoacyl-CoA thiolase/enoyl-CoA hydratase (trifunctional protein), alpha subunit |
| HADHB | 3032 | hydroxyacyl-CoA dehydrogenase/3-ketoacyl-CoA thiolase/enoyl-CoA hydratase (trifunctional protein), beta subunit |
| HIBAD H | 11112 | 3-hydroxyisobutyrate dehydrogenase |
| HIBCH | 26275 | 3-hydroxyisobutyryl-CoA hydrolase |
| HMGCL | 3155 | 3-hydroxymethyl-3-methylglutaryl-CoA lyase |
| HMGCS 2 | 3158 | 3-hydroxy-3-methylglutaryl-CoA synthase 2 |
| HSD17 B10 | 3028 | hydroxy steroid (17-beta) dehydrogenase 10 |
| IVD | 3712 | isovaleryl-CoA dehydrogenase |
| MCCC1 | 56922 | methylcrotonoyl-CoA carboxylase 1 |
| MCCC2 | 64087 | methylcrotonoyl-CoA carboxylase 2 |
| MCEE | 84693 | methylmalonyl-CoA epimerase |
| MUT | 4594 | methylmalonyl-CoA mutase |
| OXCT1 | 5019 | 3-oxoacid CoA-transferase 1 |
| PCCA | 5095 | propionyl-CoA carboxylase alpha subunit; and |
| PCCB | 5096 | propionyl-CoA carboxylase beta subunit. |

As shown in, for example in Figures 7a to 7c, high level of BCAA catabolic enzymes expression may be associated with efficient catabolism of BCAA and with significantly better patients outcome. Therefore, in one example, the branched amino acids catabolic enzymes of the method of the present disclosure include, but are not limited to, ACADS (acyl-CoA dehydrogenase, C-2 to C-3 short chain), ACADSB (acyl-CoA dehydrogenase, short/branched chain), and BCKDHA (branched chain keto acid dehydrogenase E1, alpha polypeptide).

In one example, the level measured in the method of the present disclosure is of branched amino acids (BCAA) catabolic enzymes activity.

In one example, the level of enzyme activity in the method of the present disclosure is be measured by Magnetic Resonance Spectroscopy (MRS). In one example, the level of enzyme activity in the method of the present disclosure isdetected by Magnetic Resonance Spectroscopy (MRS) by administering a hyperpolarized ¹³C compound. In one example, the level of enzyme activity in the method of the present disclosure is detected by Magnetic Resonance Spectroscopy (MRS) by administering ¹³C -alpha-ketoisocaproate. In one non-limiting example, the steps involved in MRS approach include, but are not limited to,: (1) preparation of a solution comprising hyperpolarized sodium [1-¹³C]2-ketoisocaproate; (2) injection of solution in point (1) to the tail end of a mouse; (3) detection of metabolite peaks corresponding to [1-¹³C]2-ketoisocaproate (i.e. the starting material), [1-¹³C]leucine (i.e. the product of BCAT enzyme activity), and [1-¹³C]bicarbonate (i.e. the byproduct of BCKDH enzyme activity); (4) quantification of the metabolite peaks of point (3) by calculating the ratio of [1-¹³C]leucine peak and [1-¹³C]bicarbonate peak to the sum of all three metabolite peaks (i.e. tCarbon); and (5) inferring the enzyme activity level of BCKDH based on the result of point (4). The Magnetic Resonance Spectroscopy of the present disclosure may differ from Magnetic Resonance Imaging (MRI) method known in the art, as MRS provides molecular information on the enzyme activities, whereas MRI gives an image or statistical map of the region of observation. The MRS approach provided herein is used to infer the level of acylcarnitine (C5:1); the level of accumulated BCAA; and the transcript level of enzymes involved in catabolism of at least one BCAA. The MRS approach may provide the enzyme activity of BCKDH, which is the first rate-limiting step of BCAA degradation. If the enzyme activity of BCKDH is lower, the BCAA degradation may be impaired/reduced which may indicate BCAA accumulation and reduced level of C5:1 acylcarnitine.

In one example, the level of enzyme measured by the method of the present disclosure is measured in a biological sample obtained from the subject. In one example, the biological sample to be measured by the method of the present disclosure is a tissue biopsy. In one example, the biological sample of the method of present disclosure includes, but is not limited to, a lung tissue biopsy, a breast tissue biopsy, a colorectal tissue biopsy, an esophageal tissue biopsy, a gastric tissue biopsy, a thyroid tissue biopsy, a head or neck tissue biopsy, a kidney tissue biopsy, a liver tissue biopsy, and the like.

In one example, the biological sample is a complete tumor. In another example, the biological sample is a representative tumor biopsy, which can be confirmed by examining a slice of the tumor by histology. In one example, the biological sample is substantially free of non-carcinoma cells.

In one example, the method comprises the step of collecting a sample suspected of containing a tumor, part of a tumor, or cancerous cells.

In one example, the measured level of branched amino acids (BCAA) catabolic enzymes of the method of present disclosure is at RNA and/or protein level(s). In one example, the measured level of branched amino acids (BCAA) catabolic enzymes of the method of present disclosure is at RNA level(s). In one example, the measured level of branched amino acids (BCAA) catabolic enzymes of the method of present disclosure may be at protein level(s). In one example, the measured level of branched amino acids (BCAA) catabolic enzymes of the method of present disclosure is at RNA and protein level(s).

In one example, the RNA level of branched amino acids (BCAA) catabolic enzymes of the method of present disclosure is measured by methods or devices which include, but is not limited to, RT-PCR, microarray, sequencing, and the like. In one example, the protein level of branched amino acids (BCAA) catabolic enzymes of the method of present disclosure is measured using methods/devices which include but is not limited to immunohistochemistry, protein array, mass spectrometry, and the like.

In one example, the comparing of the RNA and/or protein level of branched amino acids (BCAA) catabolic enzymes of the method of present disclosure is performed using computer based analysis. In one example, the comparing of the RNA level of branched amino acids (BCAA) catabolic enzymes of the method of present disclosure may be performed using computer based analysis. In one example, the comparing of the protein level of branched amino acids (BCAA) catabolic enzymes of the method of present disclosure is performed using computer based analysis. In one example, the comparing of the RNA and protein level of branched amino acids (BCAA) catabolic enzymes of the method of present disclosure may be performed using computer based analysis.

In one example, the method of the present disclosure is used to determine or predict whether a subject is having or likely to have a proliferative disease, wherein the proliferative disease is cancer. In one example, the cancer which is predictable or detectable by the method of present disclosure includes but is not limited to liver cancer (such as hepatocellular carcinoma, and cholangiocarcinoma), head and neck squamous cell carcinoma, kidney cancer (such as kidney renal clear cell carcinoma, kidney papillary cell carcinoma, and kidney chromophobe renal cell carcinoma), colon and rectum adenocarcinoma, breast carcinoma, lung carcinoma, thyroid carcinoma, stomach adenocarcinoma, esophageal carcinoma, and the like.

In one aspect, the present disclosure refers to a method of predicting the likelihood of a subject surviving cancer (prognosis of a subject) comprising: a. measuring a level of branched amino acids (BCAA) catabolic enzymes of the subject. In some examples, the method also comprises b. comparing and/or correlating the level measured in (a) to a standard level of branched amino acids catabolic enzymes, wherein the degree of deviation above the level of the standard indicates the degree of severity of the outcome.

As used herein, the term "standard level" or "level of the standard" refers to the average level of at least one branched amino acids (BCAA) catabolic enzyme in a healthy group of subjects. "Standard level" or "level of the standard" of at least one branched amino acids (BCAA) catabolic enzyme can be determined for example by (1) collecting samples from a clinically healthy groups of people based on age and gender, (2) quantifying the BCAA levels from the healthy groups, (3) establishing a confidence interval of 95% and 99% for the average level of branched amino acids (BCAA) in a healthy group of subjects, and (4) defining abnormality for those measurements outside of the range of the confidence interval. As appreciated by a person skilled in the art, the determination of "standard level" or "level of the standard" can also be similar to previously established practices of determining liver functions using liver enzymes (which may include but are not limited to SGOT, SGPT, and the like).

As used herein, the term "degree of deviation" refers to the amount of variation present within a data population. In one example, the degree of deviation refers to the differences between the level of at least one branched amino acids (BCAA) catabolic enzyme measured from the subject and the level of at least one branched amino acids (BCAA) catabolic enzyme measured from the control subject. Thus the deviation referred to in this example is the (positive or negative) difference in value measured for a branched-chain amino acid in the subject compared to the value obtained for the same branched-chain amino acid in the control subject. The "degree of deviation" or a particular target molecule or marker, for example, a particular amino acid or, in one example, a branched-chain amino acid, can be established by collecting and accumulating data from both healthy and different cancer patient groups in order to establish a diagnosis window (which may have confidence interval of 95% or 99%). In this case, a "higher or high degree of deviation" refers to a degree of deviation of equal to or higher than three standard deviations. A "lower or low degree of deviation" refers to a degree of deviation between two to three standard deviations.

In one example, higher degree of deviation from the level of the standard is observed in (b). In an example wherein higher degree of deviation from the level of the standard is observed in (b), the outcome is poorer (which means a poor outcome, negative, or unfavorable survival). In contrast, in another example, lower degree of deviation from the level of the standard may be observed in (b). In an example wherein lower degree of deviation from the level of the standard is observed in (b), the outcome may be better (which means a good prognosis, positive, or favorable survival).

As used herein, the term "catabolic enzyme" refers to enzymes that play a role in destructive metabolism or the breakdown of complex molecules in living organisms to form simpler ones, together with the release of energy. As such, the term "branched-chain amino acids (BCAA) catabolic enzymes" refers to enzymes that play a role in destructive metabolism or the breakdown of branched amino acid (BCAA).

In one example, the standard level of the method of the present disclosure may is a predetermined level obtained from subjects known to have good prognosis.

In one example, the branched amino acids catabolic enzymes of the method of the present disclosure include, but are not limited to, ABAT (4-aminobutyrate aminotransferase), ACAA1 (acetyl-CoA acyltransferase 1), ACAA2 (acetyl-CoA acyltransferase 2), ACAD8 (acyl-CoA dehydrogenase family member 8), ACADM (acyl-CoA dehydrogenase, C-4 to C-12 straight chain), ACADS (acyl-CoA dehydrogenase, C-2 to C-3 short chain), ACADSB (acyl-CoA dehydrogenase, short/branched chain), ACAT1 (acetyl-CoA acetyltransferase 1), ACAT2 (acetyl-CoA acetyltransferase 2), ALDH1B1 (aldehyde dehydrogenase 1 family member B1), ALDH2 (aldehyde dehydrogenase 2 family (mitochondrial)), ALDH3A2 (aldehyde dehydrogenase 3 family member A2), ALDH6A1 (aldehyde dehydrogenase 6 family member A1), ALDH9A1 (aldehyde dehydrogenase 9 family member A1), AOX1 (aldehyde oxidase 1), AUH (AU RNA binding protein/enoyl-CoA hydratase), BCKDHA (branched chain keto acid dehydrogenase E1, alpha polypeptide), BCKDHB (branched chain keto acid dehydrogenase E1, beta polypeptide), DBT (dihydrolipoamide branched chain transacylase E2), DLD (dihydrolipoamide dehydrogenase), ECHS1 (enoyl-CoA hydratase, short chain, 1, mitochondrial), EHHADH (enoyl-CoA, hydratase/3-hydroxyacyl CoA dehydrogenase), HADH (hydroxyacyl-CoA dehydrogenase), HADHA (hydroxyacyl-CoA dehydrogenase/3-ketoacyl-CoA thiolase/enoyl-CoA hydratase (trifunctional protein), alpha subunit), HADHB (hydroxyacyl-CoA dehydrogenase/3-ketoacyl-CoA thiolase/enoyl-CoA hydratase (trifunctional protein), beta subunit), HIBADH (3-hydroxyisobutyrate dehydrogenase), HIBCH (3-hydroxyisobutyryl-CoA hydrolase), HMGCL (3-hydroxymethyl-3-methylglutaryl-CoA lyase), HMGCS2 (3-hydroxy-3-methylglutaryl-CoA synthase 2), HSD17B10 (hydroxysteroid (17-beta) dehydrogenase 10), IVD (isovaleryl-CoA dehydrogenase), MCCC1 (methylcrotonoyl-CoA carboxylase 1), MCCC2 (methylcrotonoyl-CoA carboxylase 2), MCEE (methylmalonyl-CoA epimerase), MUT (methylmalonyl-CoA mutase), OXCT1 (3-oxoacid CoA-transferase 1), PCCA (propionyl-CoA carboxylase alpha subunit), PCCB (propionyl-CoA carboxylase beta subunit) and the like.

The branched amino acids catabolic enzymes of the method of the present disclosure may also be referred to using the following Gene ID:

| Official Symbol | Gene ID | Official Full Name |
|---|---|---|
| ABAT | 18 | 4-aminobutyrate aminotransferase |
| ACAA1 | 30 | acetyl-CoA acyltransferase 1 |
| ACAA2 | 10449 | acetyl-CoA acyltransferase 2 |
| ACAD8 | 27034 | acyl-CoA dehydrogenase family member 8 |
| ACAD M | 34 | acyl-CoA dehydrogenase, C-4 to C-12 straight chain |
| ACADS | 35 | acyl-CoA dehydrogenase, C-2 to C-3 short chain |
| ACADS B | 36 | acyl-CoA dehydrogenase, short/branched chain |
| ACAT1 | 38 | acetyl-CoA acetyltransferase 1 |
| ACAT2 | 39 | acetyl-CoA acetyltransferase 2 |
| ALDH1 B1 | 219 | aldehyde dehydrogenase 1 family member B 1 |
| ALDH2 | 217 | aldehyde dehydrogenase 2 family (mitochondrial) |
| ALDH3 A2 | 224 | aldehyde dehydrogenase 3 family member A2 |
| ALDH6 A1 | 4329 | aldehyde dehydrogenase 6 family member A1 |
| ALDH9 A1 | 223 | aldehyde dehydrogenase 9 family member A1 |
| AOX1 | 316 | aldehyde oxidase 1 |
| AUH | 549 | AU RNA binding protein/enoyl-CoA hydratase |
| BCKDH A | 593 | branched chain keto acid dehydrogenase E1, alpha polypeptide |
| BCKDH B | 594 | branched chain keto acid dehydrogenase E1, beta polypeptide |
| DBT | 1629 | dihydrolipoamide branched chain transacylase E2 |
| DLD | 1738 | dihydrolipoamide dehydrogenase |
| ECHS1 | 1892 | enoyl-CoA hydratase, short chain, 1, mitochondrial |
| EHHAD H | 1962 | enoyl-CoA, hydratase/3-hydroxyacyl CoA dehydrogenase |
| HADH | 3033 | hydroxyacyl-CoA dehydrogenase |
| HADHA | 3030 | hydroxyacyl-CoA dehydrogenase/3-ketoacyl-CoA thiolase/enoyl-CoA hydratase (trifunctional protein), alpha subunit |
| HADHB | 3032 | hydroxyacyl-CoA dehydrogenase/3-ketoacyl-CoA thiolase/enoyl-CoA hydratase (trifunctional protein), beta subunit |
| HIBAD H | 11112 | 3-hydroxyisobutyrate dehydrogenase |
| HIBCH | 26275 | 3-hydroxyisobutyryl-CoA hydrolase |
| HMGCL | 3155 | 3-hydroxymethyl-3-methylglutaryl-CoA lyase |
| HMGCS 2 | 3158 | 3-hydroxy-3-methylglutaryl-CoA synthase 2 |
| HSD17 B10 | 3028 | hydroxy steroid (17-beta) dehydrogenase 10 |
| IVD | 3712 | isovaleryl-CoA dehydrogenase |
| MCCC1 | 56922 | methylcrotonoyl-CoA carboxylase 1 |
| MCCC2 | 64087 | methylcrotonoyl-CoA carboxylase 2 |
| MCEE | 84693 | methylmalonyl-CoA epimerase |
| MUT | 4594 | methylmalonyl-CoA mutase |
| OXCT1 | 5019 | 3-oxoacid CoA-transferase 1 |
| PCCA | 5095 | propionyl-CoA carboxylase alpha subunit; and |
| PCCB | 5096 | propionyl-CoA carboxylase beta subunit. |

As shown for example in Figures 7a to 7c, high level of BCAA catabolic enzymes expression may be associated with efficient catabolism of BCAA and with significantly better patients outcome. Therefore, in one example, the branched-chain amino acids catabolic enzymes of the method of the present disclosure include, but are not limited to ACADS (acyl-CoA dehydrogenase, C-2 to C-3 short chain), ACADSB (acyl-CoA dehydrogenase, short/branched chain), and BCKDHA (branched chain keto acid dehydrogenase E1, alpha polypeptide).

In one example, the level measured in the method of the present disclosure are of branched amino acids (BCAA) catabolic enzymes activity.

In one example, the level of enzyme activity in the method of the present disclosure is measured by Magnetic Resonance Spectroscopy (MRS). In one example, the level of enzyme activity in the method of the present disclosure is detected by Magnetic Resonance Spectroscopy (MRS) by administering a hyperpolarized ¹³C compound. In one example, the level of enzyme activity in the method of the present disclosure is detected by Magnetic Resonance Spectroscopy (MRS) by administering ¹³C-alpha-ketoisocaproate. In one non-limiting example, the steps involved in MRS approach include but are not limited to: (1) preparation of a solution comprising hyperpolarized sodium [1-¹³C]2-ketoisocaproate; (2) injection of solution in point (1) to the tail end of a mouse; (3) detection of metabolite peaks corresponding to [1-¹³C]2-ketoisocaproate (i.e. the starting material), [1-¹³C]leucine (i.e. the product of BCAT enzyme activity), and [1-¹³C]bicarbonate (i.e. the byproduct of BCKDH enzyme activity); (4) quantification of the metabolite peaks of point (3) by calculating the ratio of [1-¹³C]leucine peak and [1-¹³C]bicarbonate peak to the sum of all three metabolite peaks (i.e. tCarbon); and (5) inferring the enzyme activity level of BCKDH based on the result of point (4). The Magnetic Resonance Spectroscopy of the present disclosure may differ from Magnetic Resonance Imaging (MRI) method known in the art, as MRS provides molecular information on the enzyme activities whereas MRI gives an image or statistical map of the region of observation. The MRS approach provided herein may be used to infer the level of acylcarnitine (C5:1); the level of accumulated BCAA; and the transcript level of enzymes involved in catabolism of at least one BCAA. The MRS approach may provide the enzyme activity of BCKDH, which is the first rate-limiting step of BCAA degradation. If the enzyme activity of BCKDH is lower, the BCAA degradation may be impaired/reduced which may indicate BCAA accumulation and reduced level of C5:1 acylcarnitine.

In one example, the level of enzyme measured by the method of the present disclosure is measured in a biological sample obtained from the subject. In one example, the biological sample to be measured by the method of the present disclosure is a tissue biopsy.

In one example, the biological sample of the method of present disclosure includes, but is not limited to a lung tissue biopsy, a breast tissue biopsy, a colorectal tissue biopsy, an esophageal tissue biopsy, a gastric tissue biopsy, a thyroid tissue biopsy, a head or neck tissue biopsy, a kidney tissue biopsy, a liver tissue biopsy and the like.

In one example, the biological sample is substantially free of non-carcinoma cells.

In one example, the method comprises the step of collecting a sample suspected of containing a tumor, part of a tumor, or cancerous cells.

. As used herein, the term "substantially free" refers to an object species wherein the predominant species, for example, a particular cell type in a sample, is present. For example, on a molar basis, the predominant species is more abundant than any other individual species in the composition. In regards to biological samples, a substantially pure sample will comprise more than about 80 percent of all individual species present in the sample, or more than about 85%, about 90%, about 95%, and about 99%. Ideally, the object species is purified to essential homogeneity, meaning that any and all contaminant species cannot be detected in the composition by conventional detection methods, wherein the composition consists essentially of a single macromolecular species.

In one example, the measured level of branched amino acids (BCAA) catabolic enzymes of the method of present disclosure is at RNA and/or protein level(s). In one example, the measured level of branched amino acids (BCAA) catabolic enzymes of the method of present disclosure is at RNA level(s). In one example, the measured level of branched amino acids (BCAA) catabolic enzymes of the method of present disclosure is at protein level(s). In one example, the measured level of branched amino acids (BCAA) catabolic enzymes of the method of present disclosure is at RNA and protein level(s).

In one example, the RNA level of branched amino acids (BCAA) catabolic enzymes of the method of present disclosure is measured by methods or devices which include, but are not limited to, RT-PCR, microarray, sequencing and the like.

In one example, the protein level of branched amino acids (BCAA) catabolic enzymes of the method of present disclosure is measured using methods/devices which include, but are not limited to immunohistochemistry, protein array, mass spectrometry, and the like.

In one example, the comparing of the RNA and/or protein level of branched amino acids (BCAA) catabolic enzymes of the method of present disclosure is performed using computer based analysis. In one example, the comparing of the RNA level of branched amino acids (BCAA) catabolic enzymes of the method of present disclosure is performed using computer based analysis. In one example, the comparing of the protein level of branched amino acids (BCAA) catabolic enzymes of the method of present disclosure is performed using computer based analysis. In one example, the comparing of the RNA and protein level of branched amino acids (BCAA) catabolic enzymes of the method of present disclosure is performed using computer based analysis.

In one example, the prognosis of a subject using the method of the present disclosure is poor or negative. In one example, the subject with poor outcomes/prognosis (negative survival) are subjects having high-grade cancer (such as having a tumor with a grade of 3 and/or 4), and/or likelihood of disease recurrence or progression, and/or not surviving for more than 1, or 2, or 3, or 4, or 5 years. In one example, the subject with poor outcomes/prognosis (negative survival) are subjects having high-grade cancer (such as having a tumor with a grade of 3 and/or 4). In one example, the subject with poor outcomes/prognosis (negative survival) are subjects having the likelihood of disease recurrence or progression. In one example, the subject with poor outcomes/prognosis (negative survival) are subjects who do not surviving for more than 1, or 2, or 3, or 4, or 5 years. In one example, the subject with poor outcomes/prognosis (negative survival) are subjects having high-grade cancer (such as having a tumor with a grade of 3 and/or 4), and likelihood of disease recurrence or progression. In one example, the subject with poor outcomes/prognosis (negative survival) are subjects having high-grade cancer (such as having a tumor with a grade of 3 and/or 4) and not surviving for more than 1, or 2, or 3, or 4, or 5 years. In one example, the subject with poor outcomes/prognosis (negative survival) are subjects having the likelihood of disease recurrence or progression and not surviving for more than 1, or 2, or 3, or 4, or 5 years. In one example, the subject with poor outcomes/prognosis (negative survival) are subjects having high-grade cancer (such as having a tumor with a grade of 3 and/or 4), and likelihood of disease recurrence or progression, and not surviving for more than 1, or 2, or 3, or 4, or 5 years.

As would be appreciated by a person skilled in the art, "tumor grading" or "tumor grade" or "grade of tumor" refers to the description of a tumor based on how abnormal the cells from the tumor and the tumor tissue appear under a microscope. "Tumor grade" or "tumor grading" or "grade of tumor" may also be an indicator of how quickly a tumor is likely to grow or spread. It is generally understood that cells from the tumor and organization of tumor tissue that are close to normal cells and tissues may be considered "well-differentiated" and may grow or spread at a slower rate when compared to abnormal looking cells from the tumor and tumor tissues (i.e. "undifferentiated" or "poorly differentiated"). The grading system for "tumor grade" or "tumor grading" or "grade of tumor" may generally comprise of five different grades, which are GX (i.e. grade cannot be assessed or undetermined grade), G1 (i.e. grade 1, well differentiated cells and/or tissues, low grade), G2 (i.e. grade 2, moderately differentiated cells and/or tissues, intermediate grade), G3 (i.e. grade 3, poorly differentiated cells and/or tissues, high grade), and G4 (i.e. grade 4, undifferentiated cells and/or tissues, high grade).

In one example, poor outcome/prognosis (negative survival) indicates a reduced likelihood of survival over 7 years. In one example, poor outcome/prognosis (negative survival) indicates a reduced likelihood of survival over 6 years. In one example, poor outcome/prognosis (negative survival) indicates a reduced likelihood of survival over 5 years. In one example, poor outcome/prognosis (negative survival) indicates a reduced likelihood of survival over 4 years. In one example, poor outcome/prognosis (negative survival) indicates a reduced likelihood of survival over 3 years. In one example, poor outcome/prognosis (negative survival) indicates a reduced likelihood of survival over 2 years. In one example, poor outcome/prognosis (negative survival) indicates a reduced likelihood of survival over 1 year.

In one example, good outcome/prognosis indicates a likelihood of survival of more than 7 years, and/or disease remission within 7 years. In one example, good outcome/prognosis indicates a likelihood of survival of more than 6 years, and/or disease remission within 6 years. In one example, good outcome/prognosis indicates a likelihood of survival of more than 5 years, and/or disease remission within 5 years. In one example, good outcome/prognosis indicates a likelihood of survival of more than 4 years, and/or disease remission within 4 years. In one example, good outcome/prognosis indicates a likelihood of survival of more than 3 years, and/or disease remission within 3 years. In one example, good outcome/prognosis indicates a likelihood of survival of more than 2 years, and/or disease remission within 2 years. In one example, good outcome/prognosis indicates a likelihood of survival of more than 1 year, and/or disease remission within 1 year.

As would be appreciated by the person skilled in the art, the final determination of the outcome or diagnosis of a cancer patient would be determined by a clinician and the result of the method of the present invention cannot or will not replace the role of a clinician. In general, it would be understood that the outcome would also depend on traditional variables such as underlying diseases and risk factors, time of diagnosis, tumor grade, tumor stage, quality of care, approved and available treatment options and the like.

In one example, for hepatocellular carcinoma patients, it is understood that the outcome or diagnosis depends on various variables such as location, which in turn reflects underlying factors (driven by HVB/HCV, alcohol, alfatoxin, NASH/NAFLD, and the like), quality care, approved and available treatment options, and the like.

In one example, the method of the present disclosure is used to predict the likelihood of a subject surviving proliferative disease, wherein the proliferative disease is cancer. In one example, the cancer which is predictable by the method of present disclosure includes, but is not limited to, liver cancer (such as hepatocellular carcinoma, and cholangiocarcinoma), head and neck squamous cell carcinoma, kidney cancer (such as kidney renal clear cell carcinoma, kidney papillary cell carcinoma, and kidney chromophobe renal cell carcinoma), colon and rectum adenocarcinoma, breast carcinoma, lung carcinoma, thyroid carcinoma, stomach adenocarcinoma, esophageal carcinoma and the like.

In one example, the method of the present disclosure further comprises administering a pharmaceutically effective amount of a branched-chain amino acid catabolism enhancer and/or a pharmaceutically effective amount of a branched-chain α-ketoacid dehydrogenase complex (BCKDC) kinase inhibitor (BDK inhibitor) and/or an effective amount of meal replacement comprising low level of branched-chain amino acid (BCAA) to the subject in need thereof. In one example, the method of the present disclosure further comprises administering a pharmaceutically effective amount of a branched-chain amino acid catabolism enhancer to the subject in need thereof. In one example, the method of the present disclosure further comprises administering a pharmaceutically effective amount of a branched-chain α-ketoacid dehydrogenase complex (BCKDC) kinase inhibitor (BDK inhibitor) to the subject in need thereof. In one example, the method of the present disclosure further comprises administering an effective amount of meal replacement comprising low level of branched-chain amino acid (BCAA) to the subject in need thereof. In one example, the method of the present disclosure further comprises administering a pharmaceutically effective amount of a branched-chain amino acid catabolism enhancer and a pharmaceutically effective amount of a branched-chain α-ketoacid dehydrogenase complex (BCKDC) kinase inhibitor (BDK inhibitor) and an effective amount of meal replacement comprising low level of branched-chain amino acid (BCAA) to the subject in need thereof.

In one aspect, the present disclosure refers to a kit or microarray chip for use in any of the methods as defined herein. In one example, the kit or microarray chip comprises: a. a reagent or a group of reagents for measuring a level of at least one acylcarnitine (C5:1) and/or branched-chain amino acid (BCAA) and/or a level of at least one branched-chain amino acid (BCAA) catabolic enzyme in the subject. In one example, the kit or microarray chip comprises b. a reagent or a group of reagents comprising a pre-determined level of acylcarnitine (C5:1) and/or branched-chain amino acid (BCAA) and/or branched-chain amino acid (BCAA) catabolic enzyme. In one example, the kit or microarray chip comprises c. optionally instructions for using the reagent or group of reagents in (a) and (b) to determine or predict whether a subject has or likely to have proliferative disease, wherein the pre-determined level may be determined by measured level of at least one acylcarnitine (C5:1) and/or branched-chain amino acid and/or branched-chain amino acid (BCAA) catabolic enzyme in a control subject (or subjects) not having the proliferative disease, and/or to determine the prognosis of the subject.

In one aspect, the present disclosure refers to a kit or microarray chip for use in any of the methods as defined herein. In one example, the kit or microarray chip comprises: a. a reagent or a group of reagents for measuring a level of at least one acylcarnitine (C5: 1) in the subject. In one example, the kit or microarray chip comprises b. a reagent comprising a pre-determined level of acylcarnitine (C5: 1). In one example, the kit or microarray chip comprises c. optionally instructions for using the reagent or group of reagents in (a) and (b) to determine or predict whether a subject has or likely to have proliferative disease, wherein the pre-determined level may be determined by measured level of at least one acylcarnitine (C5:1) in a control subject (or subjects) not having the proliferative disease, and/or to determine the prognosis of the subject.

In one aspect, the present disclosure refers to a kit or microarray chip for use in any of the methods as defined herein. In one example, the kit or microarray chip comprises: a. a reagent or a group of reagents for measuring a level of at least one branched-chain amino acid (BCAA) in the subject. In one example, the kit or microarray chip comprises b. a reagent or a group of reagents comprising a pre-determined level of at least one branched-chain amino acid (BCAA). In one example, the kit or microarray chip comprises c. optionally instructions for using the reagent or a group of reagents in (a) and (b) to determine or predict whether a subject has or likely to have proliferative disease, wherein the pre-determined level may be determined by measured level of at least one branched-chain amino acid in a control subject (or subjects) not having the proliferative disease, and/or to determine the prognosis of the subject.

In one aspect, the present disclosure refers to a kit or microarray chip for use in any of the methods as defined herein. In one example, the kit or microarray chip comprises: a. a reagent or a group of reagents for measuring a level of at least one branched-chain amino acid (BCAA) catabolic enzyme in the subject. In one example, the kit or microarray chip comprises b. a reagent or a group of reagents comprising a pre-determined level of branched-chain amino acid (BCAA) catabolic enzyme. In one example, the kit or microarray chip comprises c. optionally instructions for using the reagent or group of reagents in (a) and (b) to determine or predict whether a subject has or likely to have proliferative disease, wherein the pre-determined level may be determined by measured level of at least one branched-chain amino acid (BCAA) catabolic enzyme in a control subject (or subjects) not having the proliferative disease, and/or to determine the prognosis of the subject.

In one aspect, the present disclosure refers to a kit or microarray chip for use in any of the methods as defined herein. In one example, the kit or microarray chip comprises: a. a reagent or a group of reagents for measuring a level of at least one acylcarnitine (C5:1) and branched-chain amino acid (BCAA) and a level of at least one branched-chain amino acid (BCAA) catabolic enzyme in the subject. In one example, the kit or microarray chip comprises b. a reagent or a group of reagents comprising a pre-determined level of acylcarnitine (C5:1) and branched-chain amino acid (BCAA) and branched-chain amino acid (BCAA) catabolic enzyme. In one example, the kit or microarray chip comprises c. optionally instructions for using the reagent or group of reagents in (a) and (b) to determine or predict whether a subject has or likely to have proliferative disease, wherein the pre-determined level are determined by measured level of at least one acylcarnitine (C5:1) and branched-chain amino acid and branched-chain amino acid (BCAA) catabolic enzyme in a control subject (or subjects) not having the proliferative disease, and/or to determine the prognosis of the subject.

In one example, the kit or microarray kit or microarray chip as defined herein contains an array of one or more samples from one or more diseased tissues. The molecules on the array are, but is not limited to, polynucleotides, polypeptides or antibody molecules as described herein. The kit optionally also includes a detectable label or a labelled compound or agent capable of detecting expression of a gene product in a biological sample, and the necessary reagents for labelling the sample and affecting hybridization to complementary sequences on the array. The kit optionally also includes means for determining the amount of transcript in the sample, such as a colorimetric chart or device.

More than one array may be included in the kit, wherein each array corresponds to a tissue afflicted with different diseases and wherein each array contains a plurality of samples corresponding to a tissue afflicted with a disease. The compound or agent can be packaged in a suitable container. The kit can further include instructions for using the kit to detect protein or nucleic acid.

In one aspect, the present disclosure refers to a method for manufacturing a microarray chip or protein array chip for determining or predicting whether a subject is having or likely to have a proliferative disease. In one example, the method comprises a. immobilizing reagents on the chip that allow the measurement of the level of at least one acylcarnitine (C5:1) and/or branched-chain amino acid (BCAA) and/or branched-chain amino acid (BCAA) catabolic enzyme in the subject. In one example, the method comprises b. optionally instructions on determining or predicting whether a subject has or likely to have proliferative disease.

In one aspect, the present disclosure refers to a method for manufacturing a microarray chip or protein array chip for determining or predicting whether a subject is having or likely to have a proliferative disease. In one example, the method comprises a. immobilizing reagents on the chip that allow the measurement of the level of at least one acylcarnitine (C5:1) in the subject. In one example, the method comprises b. optionally instructions on determining or predicting whether a subject has or likely to have proliferative disease.

In one aspect, the present disclosure refers to a method for manufacturing a microarray chip or protein array chip for determining or predicting whether a subject is having or likely to have a proliferative disease. In one example, the method comprises a. immobilizing reagents on the chip that allow the measurement of the level of at least one branched-chain amino acid (BCAA) in the subject. In one example, the method comprises b. optionally instructions on determining or predicting whether a subject has or likely to have proliferative disease.

In one aspect, the present disclosure refers to a method for manufacturing a microarray chip or protein array chip for determining or predicting whether a subject is having or likely to have a proliferative disease. In one example, the method comprises a. immobilizing reagents on the chip that allow the measurement of the level of at least one branched-chain amino acid (BCAA) catabolic enzyme in the subject. In one example, the method comprises b. optionally instructions on determining or predicting whether a subject has or likely to have proliferative disease.

In one aspect, the present disclosure refers to a method for manufacturing a microarray chip or protein array chip for determining or predicting whether a subject is having or likely to have a proliferative disease. In one example, the method comprises a. immobilizing reagents on the chip that allow the measurement of the level of at least one acylcarnitine (C5:1) and branched-chain amino acid (BCAA) and branched-chain amino acid (BCAA) catabolic enzyme in the subject. In one example, the method comprises b. optionally instructions on determining or predicting whether a subject has or likely to have proliferative disease.

In one aspect, the present disclosure refers to a method for manufacturing a microarray chip or protein array chip for determining the outcome of a proliferative disease in a subject. In one example, the method comprises a. immobilizing reagents on the chip that allow the measurement of the level of at least one acylcarnitine (C5:1) and/or branched-chain amino acid (BCAA) and/or branched-chain amino acid (BCAA) catabolic enzyme in the subject. In one example, the method comprises b. optionally instructions on determining what outcome a subject has.

In one aspect, the present disclosure refers to a method for manufacturing a microarray chip or protein array chip for determining the outcome of a proliferative disease in a subject. In one example, the method comprises a. immobilizing reagents on the chip that allow the measurement of the level of at least one acylcarnitine (C5:1). In one example, the method comprises b. optionally instructions on determining what outcome a subject has.

In one aspect, the present disclosure refers to a method for manufacturing a microarray chip or protein array chip for determining the outcome of a proliferative disease in a subject. In one example, the method comprises a. immobilizing reagents on the chip that allow the measurement of the level of at least one branched-chain amino acid (BCAA) in the subject. In one example, the method comprises b. optionally instructions on determining what outcome a subject has.

In one aspect, the present disclosure refers to a method for manufacturing a microarray chip or protein array chip for determining the outcome of a proliferative disease in a subject. In one example, the method comprises a. immobilizing reagents on the chip that allow the measurement of the level of at least one branched-chain amino acid (BCAA) catabolic enzyme in the subject. In one example, the method comprises b. optionally instructions on determining what outcome a subject has.

In one aspect, the present disclosure refers to a method for manufacturing a microarray chip or protein array chip for determining the outcome of a proliferative disease in a subject. In one example, the method comprises a. immobilizing reagents on the chip that allow the measurement of the level of at least one acylcarnitine (C5:1) and branched-chain amino acid (BCAA) and branched-chain amino acid (BCAA) catabolic enzyme in the subject. In one example, the method comprises b. optionally instructions on determining what outcome a subject has.

A person skilled in the art would appreciate that the kit or microarray kit or microarray chip of the present disclosure can be manufactured with any method or technique known in the art. In one example, the kit or microarray kit or microarray chip as defined herein contains an array of one or more samples from one or more diseased tissues. The molecules on the array are, but are not limited to, polynucleotides, polypeptides or antibody molecules as described herein. The kit optionally also includes a detectable label or a labelled compound or agent capable of detecting expression of a gene product in a biological sample and the necessary reagents for labelling the sample and affecting hybridization to complementary sequences on the array. The kit optionally also includes means for determining the amount of transcript in the sample, such as a colorimetric chart or device.

More than one array may be included in the kit, wherein each array corresponds to a tissue afflicted with different diseases, and wherein each array contains a plurality of samples corresponding to a tissue afflicted with a disease. The compound or agent can be packaged in a suitable container. The kit can further include instructions for using the kit to detect protein or nucleic acid.

In one aspect, the present disclosure refers to a method of treating or preventing a proliferative disease in a subject in need thereof. In one example, the proliferative disease is characterized and/or diagnosed by: a decrease in a level of an acylcarnitine (C5:1) (when compared to a healthy control subject or group of control subjects); and/or an accumulation of at least one branched-chain amino acid (BCAA) (when compared to a healthy control subject or group of control subjects); and/or a suppression of transcripts-level of enzymes involved in the catabolism of at least one branched-chain amino acid (BCAA) (when compared to a healthy control subject or group of control subjects); and/or a suppression of enzyme activity involved in the catabolism of at least one branched-chain amino acid (BCAA) (when compared to a healthy control subject or group of control subjects). In one example, said method comprises administering a branched-chain amino acid catabolism enhancer and/or a branched-chain α-ketoacid dehydrogenase complex (BCKDC) kinase inhibitor (BDK inhibitor).

In one aspect, the present disclosure refers to a method of treating or preventing a proliferative disease in a subject in need thereof. In one example, the proliferative disease is characterized and/or diagnosed by: a decrease in a level of an acylcarnitine (C5:1) (when compared to a healthy control subject or group of control subjects). In one example, said method comprises administering a branched-chain amino acid catabolism enhancer and/or a branched-chain α-ketoacid dehydrogenase complex (BCKDC) kinase inhibitor (BDK inhibitor).

In one aspect, the present disclosure refers to a method of treating or preventing a proliferative disease in a subject in need thereof. In one example, the proliferative disease is characterized and/or diagnosed by: an accumulation of at least one branched-chain amino acid (BCAA) (when compared to a healthy control subject or group of control subjects). In one example, said method comprises administering a branched-chain amino acid catabolism enhancer and/or a branched-chain α-ketoacid dehydrogenase complex (BCKDC) kinase inhibitor (BDK inhibitor).

In one aspect, the present disclosure refers to a method of treating or preventing a proliferative disease in a subject in need thereof. In one example, the proliferative disease is characterized and/or diagnosed by: a suppression of transcripts-level of enzymes involved in the catabolism of at least one branched-chain amino acid (BCAA) (when compared to a healthy control subject or group of control subjects). In one example, said method comprises administering a branched-chain amino acid catabolism enhancer and/or a branched-chain α-ketoacid dehydrogenase complex (BCKDC) kinase inhibitor (BDK inhibitor).

In one aspect, the present disclosure refers to a method of treating or preventing a proliferative disease in a subject in need thereof. In one example, the proliferative disease is characterized and/or diagnosed by: a suppression of enzyme activity involved in the catabolism of at least one branched-chain amino acid (BCAA) (when compared to a healthy control subject or group of control subjects). In one example, said method comprises administering a branched-chain amino acid catabolism enhancer and/or a branched-chain α-ketoacid dehydrogenase complex (BCKDC) kinase inhibitor (BDK inhibitor).

In one aspect, the present disclosure refers to a method of treating or preventing a proliferative disease in a subject in need thereof. In one example, the proliferative disease is characterized and/or diagnosed by: a decrease in a level of an acylcarnitine (C5:1) (when compared to a healthy control subject or group of control subjects); and an accumulation of at least one branched-chain amino acid (BCAA) (when compared to a healthy control subject or group of control subjects); and a suppression of transcripts-level of enzymes involved in the catabolism of at least one branched-chain amino acid (BCAA) (when compared to a healthy control subject or group of control subjects); and a suppression of enzyme activity involved in the catabolism of at least one branched-chain amino acid (BCAA) (when compared to a healthy control subject or group of control subjects). In one example, said method comprises administering a branched-chain amino acid catabolism enhancer and/or a branched-chain α-ketoacid dehydrogenase complex (BCKDC) kinase inhibitor (BDK inhibitor).

As used herein, the term "catabolic enzyme" refers to enzymes that play a role in destructive metabolism or the breakdown of complex molecules in living organisms to form simpler ones, together with the release of energy. As such, the term "branched amino acids (BCAA) catabolic enzymes" refers to enzymes that play a role in destructive metabolism or the breakdown of branched amino acid (BCAA).

In one example, the method of the present disclosure detects an increased accumulation in one branched-chain amino acids level. In one example, the method of the present disclosure detects an increased accumulation in two branched-chain amino acids levels. In one example, the method of the present disclosure detects an increased accumulation in three branched-chain amino acids levels.

In one example, the method of the present disclosure detects that the proliferative disease further comprises the characteristics of an accumulation of at least one amino acid, which includes, but is not limited to, phenylalanine, methionine and asparagine. In one example, the method of the present disclosure detects that the proliferative disease further comprises the characteristics of an accumulation of at least two amino acids, which include, but are not limited to, phenylalanine, methionine and asparagine. In one example, the method of the present disclosure detects that the proliferative disease further comprises the characteristics of an accumulation of at least three amino acids, which include, but are not limited to, phenylalanine, methionine and asparagine.

In one example, the method of the present disclosure further comprises administering a branched-chain amino acid catabolism enhancer and/or a branched-chain α-ketoacid dehydrogenase complex (BCKDC) kinase inhibitor (BDK inhibitor). In one example, the method of the present disclosure further comprises administering a branched-chain amino acid catabolism enhancer. In one example, the method of the present disclosure further comprises administering a branched-chain α-ketoacid dehydrogenase complex (BCKDC) kinase inhibitor (BDK inhibitor). In one example, the method of the present disclosure further comprises administering a branched-chain amino acid catabolism enhancer and a branched-chain α-ketoacid dehydrogenase complex (BCKDC) kinase inhibitor (BDK inhibitor).

In one example, the branched-chain amino acid catabolism enhancer used in the method of the present disclosure may be a peroxisome proliferator-activated receptor-alpha (PPARa) agonist.

In one example, the PPARa agonist used in the method of the present disclosure includes but is not limited to propan-2-yl 2-[4-(4-chlorobenzoyl)phenoxy]-2-methylpropanoate (fenofibrate), 2-[4-[2-[(4-chlorobenzoyl)amino]ethyl]phenoxy]-2-methylpropanoic acid (bezafibrate), 2-[4-[2-[4-cyclohexylbutyl(cyclohexylcarbamoyl)amino]ethyl]phenyl]sulfanyl-2-methylpropanoic acid (GW7647), and the like.

In one example, the BDK inhibitor used in the method of the present disclosure includes, but is not limited to, natural and synthetic BDK inhibitors. Thus, in one example, the BDK inhibitor is, but is not limited to, propan-2-yl 2-[4-(4-chlorobenzoyl)phenoxy]-2-methylpropanoate (fenofibrate), 2-[4-[2-[(4-chlorobenzoyl)amino]ethyl]phenoxy]-2-methylpropanoic acid (bezafibrate)), 3,6-dichloro-1-benzothiophene-2-carboxylate (BT2), and the like.

In one example, the method of the present disclosure is used to treat or prevent a proliferative disease, wherein the proliferative disease is cancer. In one example, the cancer which is treatable or preventable by the method of present disclosure includes, but is not limited to, liver cancer (such as hepatocellular carcinoma, and cholangiocarcinoma), head and neck squamous cell carcinoma, kidney cancer (such as kidney renal clear cell carcinoma, kidney papillary cell carcinoma, and kidney chromophobe renal cell carcinoma), colon and rectum adenocarcinoma, breast carcinoma, lung carcinoma, thyroid carcinoma, stomach adenocarcinoma, esophageal carcinoma, and the like.

In one aspect, the present disclosure refers to a method of treating or preventing a proliferative disease in a subject in need thereof. In one example, the proliferative disease is characterized and/or diagnosed by: a decrease in a level of an acylcarnitine (C5:1) (when compared to a healthy control subject or group of control subjects); and/or an accumulation of at least one branched-chain amino acid (BCAA) (when compared to a healthy control subject or group of control subjects); and/or a suppression of transcripts-level of enzymes involved in the catabolism of at least one branched-chain amino acid (BCAA) (when compared to a healthy control subject or group of control subjects); and/or a suppression of enzyme activity involved in the catabolism of at least one branched-chain amino acid (BCAA) (when compared to a healthy control subject or group of control subjects). In one example, said method comprises administering a meal replacement comprising low level of branched-chain amino acid (BCAA) to the subject in need thereof.

In one aspect, the present disclosure refers to a method of treating or preventing a proliferative disease in a subject in need thereof. In one example, the proliferative disease is characterized and/or diagnosed by: a decrease in a level of an acylcarnitine (C5:1) (when compared to a healthy control subject or group of control subjects). In one example, said method comprises administering a meal replacement comprising low levels of branched-chain amino acid (BCAA) to the subject in need thereof.

In one aspect, the present disclosure refers to a method of treating or preventing a proliferative disease in a subject in need thereof. In one example, the proliferative disease is characterized and/or diagnosed by: an accumulation of at least one branched-chain amino acid (BCAA) (when compared to a healthy control subject or group of control subjects). In one example, said method comprises administering a meal replacement comprising low levels of branched-chain amino acid (BCAA) to the subject in need thereof.

In one aspect, the present disclosure refers to a method of treating or preventing a proliferative disease in a subject in need thereof. In one example, the proliferative disease is characterized and/or diagnosed by: a suppression of transcripts-level of enzyme involved in the catabolism of at least one branched-chain amino acid (BCAA) (when compared to a healthy control subject or group of control subjects). In one example, said method comprises administering a meal replacement comprising low levels of branched-chain amino acid (BCAA) to the subject in need thereof.

In one aspect, the present disclosure refers to a method of treating or preventing a proliferative disease in a subject in need thereof. In one example, the proliferative disease is characterized and/or diagnosed by: a suppression of enzyme activity involved in the catabolism of at least one branched-chain amino acid (BCAA) (when compared to a healthy control subject or group of control subjects). In one example, said method comprises administering a meal replacement comprising low levels of branched-chain amino acid (BCAA) to the subject in need thereof.

In one aspect, the present disclosure refers to a method of treating or preventing a proliferative disease in a subject in need thereof. In one example, the proliferative disease is characterized and/or diagnosed by: a decrease in a level of an acylcarnitine (C5:1) (when compared to a healthy control subject or group of control subjects); and an accumulation of at least one branched-chain amino acid (BCAA) (when compared to a healthy control subject or group of control subjects); and a suppression of transcripts-level of enzyme involved in the catabolism of at least one branched-chain amino acid (BCAA) (when compared to a healthy control subject or group of control subjects); and a suppression of enzyme activity involved in the catabolism of at least one branched-chain amino acid (BCAA) (when compared to a healthy control subject or group of control subjects). In one example, said method comprises administering a meal replacement comprising low level of branched-chain amino acid (BCAA) to the subject in need thereof.

As used herein, the term "catabolic enzyme" refers to enzymes that play a role in destructive metabolism or the breakdown of complex molecules in living organisms to form simpler ones, together with the release of energy. As such, the term "branched-chain amino acids (BCAA) catabolic enzymes" refers to enzymes that play a role in destructive metabolism or the breakdown of branched-chain amino acids (BCAA).

In one example, the method of the present disclosure detects an increased accumulation in one branched-chain amino acids level. In one example, the method of the present disclosure detects that an increased accumulation in two branched-chain amino acids levels. In one example, the method of the present disclosure detects an increased accumulation in three branched-chain amino acids levels.

In one example, the method of the present disclosure detects that the proliferative disease further comprises the characteristics of an accumulation of at least one amino acid, which includes but, is not limited to, phenylalanine, methionine and asparagine. In one example, the method of the present disclosure detects that the proliferative disease further comprises the characteristics of an accumulation of at least two amino acids, which include, but are not limited to phenylalanine, methionine and asparagine. In one example, the method of the present disclosure detects that the proliferative disease further comprises the characteristics of an accumulation of at least three amino acids, which include, but are not limited to phenylalanine, methionine and asparagine.

In one example, the method of the present disclosure further comprises administering a branched-chain amino acid catabolism enhancer and/or a branched-chain α-ketoacid dehydrogenase complex (BCKDC) kinase inhibitor (BDK inhibitor). In one example, the method of the present disclosure further comprises administering a branched-chain amino acid catabolism enhancer. In one example, the method of the present disclosure further comprises administering a branched-chain α-ketoacid dehydrogenase complex (BCKDC) kinase inhibitor (BDK inhibitor). In one example, the method of the present disclosure further comprises administering a branched-chain amino acid catabolism enhancer and a branched-chain α-ketoacid dehydrogenase complex (BCKDC) kinase inhibitor (BDK inhibitor).

In one example, the branched-chain amino acid catabolism enhancer used in the method of the present disclosure is a peroxisome proliferator-activated receptor-alpha (PPARa) agonist.

In one example, the PPARa agonist used in the method of the present disclosure includes, but is not limited to, propan-2-yl 2-[4-(4-chlorobenzoyl)phenoxy]-2-methylpropanoate (fenofibrate), 2-[4-[2-[(4-chlorobenzoyl)amino]ethyl]phenoxy]-2-methylpropanoic acid (bezafibrate), 2-[4-[2-[4-cyclohexylbutyl(cyclohexylcarbamoyl)amino]ethyl]phenyl]sulfanyl-2-methylpropanoic acid (GW7647), and the like.

In one example, the method of the present disclosure is used to treat or prevent a proliferative disease, wherein the proliferative disease is cancer. In one example, the cancer which is treatable or preventable by the method of present disclosure includes, but is not limited to, liver cancer (such as hepatocellular carcinoma, and cholangiocarcinoma), head and neck squamous cell carcinoma, kidney cancer (such as kidney renal clear cell carcinoma, kidney papillary cell carcinoma, and kidney chromophobe renal cell carcinoma), colon and rectum adenocarcinoma, breast carcinoma, lung carcinoma, thyroid carcinoma, stomach adenocarcinoma, esophageal carcinoma, and the like.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

### EXPERIMENTAL SECTION

### Methods

**Animals and statistics.** All animal studies were approved by the Institutional Animal Care and Use Committee at A*STAR. Animals were fed ad libitum and maintained in a specific pathogen free facility with constant ambient temperature and a 12-hour light cycle. C57BL/6 mice for breeding were obtained from Biological Resource Center, A*STAR. 15 day-old pups were injected i.p. with a single 50 mg/kg dose of diethylnitrosamine (DEN), then given a single 2 mg/kg dose of TCPOBOP i.p. 30 days later. To ensure sufficient tissue for transcriptomic, metabolomics, and proteomic analyses in Fig. 2, DEN tumors were collected at 8 months post injection and DEN non-tumor tissues were collected prior to tumor formation at 5 months. Normal liver tissues were collected from age-matched mice without DEN injection. ACI rats were obtained from Harlan (Dublin, VA) and used in all rat studies. For the regenerating liver model, 10-12 week-old rats were anesthetized and two-thirds of the liver was removed as previously described. Liver tissue was harvested 24 hours later, at a time when hepatocyte proliferation is at its highest. For the Morris Hepatoma model, 10 week-old rats were anesthetized, 1 million MH3924a cells were injected directly into the liver, and tumors were harvested two weeks later. Control rat liver was harvested from age-matched rats. For rat DEN model, DEN was given to 10 week-old rats via drinking water containing 100 mg/L DEN for up to 4 months, and control rat livers from age-matched rats were analyzed. During sacrifice, animals were anesthetized and blood was collected by cardiac puncture and death was confirmed by cervical dislocation. Livers were resected, measured by electronic calipers and snap frozen in liquid nitrogen. For mice on special diets, animals were randomly assigned to experimental groups upon weaning. Sample sizes were estimated based on prior experience with the DEN model with a power analysis, including a power level of 80% and confidence interval of 95%. Special diets were purchased from Research Diets, Inc. (New Brunswick, NJ) and their composition is summarized in Table 1 (fig. 15). Body weights were measured weekly and prior to sacrifice lean and fat mass were measured by an EchoMRI Body Composition Analyzer. After sacrifice, tissues were assigned random ID numbers and analyses were performed blinded to experimental group information. Homoscedastic two-tailed t-test values are shown unless otherwise noted. Standard error of the mean (s.e.m.) are shown for all quantitative data, except when smaller than data point symbols (cell proliferation growth curves) or for clarity (heat maps and dot plots). Samples were considered for exclusion only if identified by a Grubbs' test with an alpha value below 0.01.

**Transcriptomic and metabolomic data.** RNA was extracted using Trizol (Life Technologies) and/or purified on RNAeasy columns (Qiagen), then analyzed for purity using RNA pico chips run on an Agilent 2100 bioanalyzer. Only samples with a RIN > 7 and 28s:18s ratio >1.0 were used in analysis. Samples were sequenced by Beijing Genomics Institute (BGI, Hong Kong) using the paired-end sequencing method (91 bp) with approximately 40 million reads per sample. RNA-Seq data are deposited in the Gene Expression Omnibus under accession number GSE75677. Gene lists were analyzed using DAVID (david.ncifcrf.gov) and Ingenuity Pathway Analysis (Qiagen), and heat maps were generated with GenePattern (www.broadinstitute.org/cancer/software/genepattern). RT-PCR was run on an Applied Biosystems StepOnePlus with Power SYBR Green (Life Technologies) and the sequences and the SEQ ID NO of the RT PCR Primer used herein are listed in Table 1 at the end of this section. Amino acids were quantified by HPLC-MS/MS using purified standards (Sigma). Acylcarnitine measurements were made by flow injection tandem mass spectrometry using sample preparation methods described previously. The data were acquired using a Waters Acquity^{™} UPLC system equipped with a TQ (triple quadrupole) detector and a data system controlled by MassLynx 4.1 operating system (Waters, Milford, MA). Tissue BCKDH activity was determined as previously described. Frozen liver samples were pulverized in liquid nitrogen, then 200 mg of tissue was homogenized in 1 mL of ice cold homogenization buffer (30 mM KPi pH7.5, 3 mM EDTA, 5 mM DTT, 1 mM α-ketoisovalerate, 3% FBS, 5% Triton X-100, 1 µM Leupeptin) using a QIAGEN TissueLyser II set at a frequency of 15/s for 1 minute. Homogenized samples were centrifuged for 10 minutes at 10,000 × g and the supernatant was collected. 50 µL of supernatant was added to 300 µL of assay buffer (50 mM HEPES pH 7.5, 30 mM KPi pH7.5, 0.4 mM CoA, 3 mM NAD+, 5% FBS, 2 mM Thiamine Pyrophosphate, 2 mM MgCl₂, 7.8 µM [1-¹⁴C]α-ketoisovalerate) in a polystyrene test tube containing a raised 1 M NaOH CO₂ trapping system. The tubes were capped and placed in a shaking water bath set at 37°C for 30 min. Tubes were then placed on ice and the reaction mixture was acidified by injection of 100 µl of 70% perchloric acid followed by shaking on an orbital shaker at room temperature for 1 hour. The ¹⁴CO₂ contained in the 1 M NaOH trap was counted in a liquid scintillation counter. For magnetic resonance spectroscopy studies, approximately 48 mg of [1-¹³C] 2-ketoisocaproic acid (Sigma #750832), doped with 15 mM trityl-radical (OXO63, GE Healthcare) and 3 µl of gadoterate meglumine (10 mM, Dotarem^{®}, Guerbet), was hyperpolarized in a polarizer, with 60 min of microwave irradiation. The sample was subsequently dissolved in a pressurized and heated alkaline solution, containing 100 mg/L EDTA to yield a solution of 80 mM hyperpolarized sodium [1-¹³C]2-ketoisocaproate with a polarization of 30%, T1 of 25 seconds and physiological temperature and pH. Rats were positioned in a 9.4 T horizontal bore MR scanner interfaced to a Avance III console (Bruker Biospec), and inserted into a dual-tuned (¹H/¹³C) rat abdominal coil (20 mm diameter). Correct positioning was confirmed by the acquisition of a coronal proton FLASH image (TE/TR, 8.0/100.0 ms; matrix size, 192 × 192; FOV, 50 × 36 mm; slice thickness, 2.0 mm; excitation flip angle, 30°). A respiratory-gated shim was used to reduce the proton linewidth to approximately 230 Hz. Immediately before injection, a respiratory-gated ¹³C MR pulse-acquire spectroscopy sequence was initiated. 2.0-2.5 mL (0.5 mmol /kg body weight) of hyperpolarized 2-ketoisocaproate was intravenously injected over 10 s into the anesthetized rat. Thirty individual liver spectra were acquired over 1 min after injection (TR, 2 s; excitation flip angle, 25°; sweep width, 8,000 Hz; acquired points, 2,048; frequency centered on the ketoisocaproate resonance). Liver ¹³C MR spectra were analyzed using the AMARES algorithm as implemented in the jMRUI software package. Spectra were baseline and DC offset-corrected based on the last half of acquired points. To quantify hepatic metabolism, the spectra were summed over the first 30 s upon 2-ketoisocaproate arrival. Metabolite peaks corresponding to [1-^{e}C]2-ketoisocaproate (172.6ppm) and its metabolic derivatives [1-¹³C]leucine (176.8ppm) and [1-¹³C]bicarbonate (160.8 ppm) were fitted with prior knowledge assuming a Lorentzian line shape, peak frequencies, relative phases, and linewidths. For each animal, tCarbon is defined as the sum of all these three metabolite peaks. The normalized ratios [1-¹³C]leucine/tCarbon and [1-¹³C]bicarbonate/tCarbon were computed for statistical analysis.

**Cell Culture.** Cell lines were obtained [Huh7 from Japanese Collection of Research Bioresources; Hep3B, SNU182, SNU387, SNU398, and SNU449 from ATCC; and Morris Hepatoma 3924a from German Cancer Research Center Tumor Collection] and not further verified. Only cell stocks that had been verified mycoplasma-negative within the prior 9 months were used. All cell lines were maintained in RPMI 1640 with 10% FBS and 1% Pen Strep (Gibco) and grown in a 37°C humidified incubator with 5% CO₂. Real-time cell growth measurements were taken on an xCELLigence RTCA SP (Acea) at 15-minute intervals. For compound treatment growth curves, cells were seeded in quadruplicate at 1,250, 2,500 or 5,000 cells per well and normalized 12 hours later (time 0 hours), immediately prior to changing half of the media to add compounds or vehicle. Proliferation rates were calculated over a 48-hour period beginning 2 hours after addition of BT2 (3,6-dichlorobenzo[b]thiophene-2-carboxylic acid; Matrix Scientific), Fenofibrate (Sigma), GW7647 (Sigma), Rapamycin (Tocris), Torin 1 (Tocris) or vehicle (DMSO). For immunoblots, cells were seeded in 10 cm dishes and allowed to grow for at least 24 hours. At approximately 80% confluence the media was changed to include indicated amounts of compound or vehicle (DMSO) and whole cell lysates were harvested 2 hours later. For inducible shRNA knockdowns, interfering RNA for target sequences were inserted into the pTRIPZ vector (Dharmacon; BCKDK shRNA1: CGCCTGTGTGAGCACAAGTAT, and shRNA2: GGACCCAGACAGATGGACTTA) or Tet-pLKO-puro (Addgene plasmid #21915; BCKDHA shRNA1: CCGGTCCTTCTACATGACCAACTATCTCGAGATAGT TGGTCATGTAGAAGGATTTTTG, and shRNA2: CCG GGC AGT CAC GAA AGA AGG TCA TCT CGA GAT GAC CTT CTT TCG TGA CTG CTT TTT G). The resulting constructs, along with lentiviral packaging vectors, were transfected into HEK293T cells with Lipofectamine 2000 (Invitrogen) following manufacturer's protocol. Supernatants containing lentivirus were collected 24 hours later, passed through 0.2µm filters, and added to recipient cell lines for 24 hours along with 8µg/mL polybrene (Sigma). Cells were then incubated in selection media (1-3µg/mL puromycin, depending on cell line sensitivity) for 5-7 days. For growth curves, cells were maintained in 250µg/mL doxycycline for 5 days, then seeded into wells with (+Dox) or without (-Dox) doxycycline in quadruplicate at 625, 1,250, 2,500 or 5,000 cells per well. Wells were normalized 12 hours later (Day 0) and allowed to grow without media change or addition of additional doxycycline (so that the average concentration was 125µg/mL). Proliferation rates were calculated over a 7-day period (Day 0 to Day 7). For immunoblots, cells were seeded in 10 or 15 cm dishes, and were split or received media change every 3-4 days to maintain a doxycycline concentration of 250µg/mL. After 10 days, cells were harvested as whole cell lysates, or as purified mitochondrial fractions using an isolation kit (Thermo Fisher). Results shown are one of at least three independent experiments. For CRISPR-Cas9-induced mutagenesis, previously published methods were followed. Briefly, sgRNAs were computationally identified (http://www.genome-engineering.org/crispr) and inserted into the lentiCRISPRv2 vector (Addgene plasmid #52961; *BCKDK* target the sequence GTCGGCCATCGACGCGGCAG). Lentivirus was produced as detailed above, and Hep3B cells were transduced and underwent with 1µg/mL puromycin selection for 7 days. Single-cell colonies were generated and 80 clones were examined for mutations by sequencing 15-20 PCR products of the target region. LentiCRISPRv2 empty vector-transduced Hep3B cells were used as controls, which behaved similarly to parental Hep3B cells and non-frameshift BCKDK mutant Hep3B clones. For growth curves, 1250 cells from each clone were plated in quadruplicate, normalized 6 hours later, and allowed to grow for 5 days. For immunoblots, cells were seeded into 10cm or 15cm dishes and harvested 5 days later as whole cell lysates or purified mitochondrial fractions, when the cells were approximately 80% confluent.

**Immunoblots and immunohistochemistry.** Antibodies were obtained from Cell Signaling [Cleaved Caspase 3 (9664), Cox IV (4844), p-Histone H2A.X (9718), S6 (2317), p-S6 (4858), S6K (9202), p-S6K (9234)], Santa Cruz [Bckdha (sc-67200), Bckdhb (sc160974), Gapdh (sc-32233)], Bethyl [p-Bckdha (A304-672A)], Sigma [Tubulin (T5168), Acads (HPA022271)], and Abcam [Acadsb (ab99951), Bckdk (ab125389 and ab151297), Ki67 (ab15580)]. All samples were harvested in RIPA buffer with protease inhibitors (Roche). For immunoblots, approximately 10 µg protein samples were run by SDS-PAGE, transferred to PVDF membranes using iBlot2 (Life Technologies), blocked with 5% milk and incubated with primary antibodies in 5% BSA. Membranes were then incubated with α-mouse/rabbit-HRP secondary antibodies (GE) and developed with ECL prime (GE). For immunohistochemistry, liver specimens were fixed with 10% neutral buffered formalin and 70% ethanol and embedded in paraffin. Sections were cut at 7 µm, deparaffinized, subjected to citrate buffer antigen retrieval, and exposed to hydrogen peroxide to quench endogenous peroxidase prior to incubation with primary antibodies. Vectastain ABC kit and ImmPACT DAB (Vector Laboratories) were used for chromagen development, then counterstained with Harris hematoxylin.

**Human expression data.** RNA-Seq raw count data were downloaded from The Cancer Genome Atlas (http://cancergenome.nih.gov) and differentially expressed genes were quantified with the DESeq2 package in R. For multi-cancer analyses, genes with an unadjusted P-value <0.001 were considered significant, and for single-cancer analyses genes P-values<2.44×10⁻⁶ (meeting Bonferroni correction criteria) were considered significant. Gene lists were analyzed using DAVID (david.ncifcrf.gov). The survMisc package in R and Cutoff Finder (http://molpath.charite.de/cutoff) were used to identify appropriate cutoff values for splitting patients into high and low expression groups. Combined expression indexes were screened, developed, and analyzed using a method similar to the Steepest Decent. Kaplan-Meier survival estimate curves and associated statistics were generated with the Survival package in R. Adjustments in hazard ratios include age at initial pathologic diagnosis, gender (male/female) tumor stage (1-4), tumor grade (1-4), radiation therapy (yes/no/unspecified), pharmaceutical therapy (yes/no/unspecified), additional therapies (additional pharmaceutical therapy, additional radiation therapy, or additional surgery procedure yes/no/unspecified), and tumor subtype (colon adenocarcinoma or rectum adenocarcinoma for colorectal adenocarcinoma; adenocarcinoma diffuse type, intestinal adenocarcinoma tubular type, intestinal adenocarcinoma mucinous type, intestinal adenocarcinoma papillary type, adenocarcinoma signet ring type, adenocarcinoma not otherwise specified, or intestinal adenocarcinoma not otherwise specified for stomach adenocarcinoma). Heat maps were generated using GenePattern (www.broadinstitute.org/cancer/software/genepattern). Oncomine (www.oncomine.org) data were analyzed with filters [data type: mRNA, gene rank threshold: all; fold change threshold: 1.5; p-value threshold: 0.05]. Biopsy immunohistochemical micrographs from male patients were downloaded from The Human Protein Atlas (http://www.proteinatlas.org).

**Human dietary analysis.** NHANES III (1988-1994) is a US nationally-representative population-based survey of non-institutionalized individuals over 2 years of age. The study population included 6779 non-pregnant, non-lactating adults with reliable energy intake (400-7000 kcal), ages 50-90 with mortality follow-up from the date of participation (1988-1994) through December 31, 2011. Data from the structured household interview and mobile examination center (MEC) physical examination were included in the present analysis. Variables deemed important in the literature were evaluated for potential confounding, and adjusted for in final analyses. Age, sex, race/ethnicity, years of education, cigarette smoking, leisure-time physical activity, dietary intake, dietary behaviors, and doctor-diagnosed medical history were self-reported. Waist circumference was measured by trained personnel to the nearest 0.1cm at the iliac crest. Dummy variables were created for smoking status (current, former, never) based on responses to the questions "Have you smoked at least 100 cigarettes during your entire life?" and "Do you smoke cigarettes now?" Participants were coded as 'never' smokers if they had not smoked at least 100 cigarettes and did not currently smoke cigarettes; 'former' smokers had smoked at least 100 cigarettes but did not currently smoke, and 'current' smokers responded affirmatively to both questions. Three dummy variables were created for race-ethnicity (Non-Hispanic white, Non-Hispanic black, and Mexican-American). Three dummy variables were also created for years of education (less than high school education, high school graduate, and college graduate). Self-reported history (y/n) of doctor diagnosed diabetes, cancer, and cardiovascular disease were included as binary variables in all models. Dietary behaviors including intentional weight loss in the last year (y/n), dietary changes in the last year (y/n), and whether intake on the dietary recall was less than, more than, or comparable to their usual day were included as covariates. Total kilocalories, percent kilocalories from fat, carbohydrate, and non-BCAA protein were computed from the 24-hour recall used to assess dietary intake. Total leisure-time physical activity was estimated by a physical activity questionnaire, which asked participants to report the frequency (over the past 30 days) of engaging in nine activities and up to four additional other activities not queried directly in the survey. Weekly frequencies of each activity were multiplied by a validated intensity rating in metabolic equivalents (MET) and summed for each individual. MET values used in NHANES III were defined by the Compendium of Physical Activities. In this cohort, the observation that BCAAs comprised 17.3% of total protein was calculated. Given dietary recommendations for protein intake at 10-35% of total kcal, the threshold for low BCAA intake group was set at 1.73% (10% protein/total kcal × 17.3% BCAA/total protein kcal). Few individuals consumed very high protein diets (>35% of total kcal), and therefore the threshold for high BCAA intake was set as above the mean of recommended protein intake (22.5% protein/total kcal × 17.3%BCAA/total protein kcal). Cox Proportional Hazard Models adjusted for covariates were used to evaluate the association between BCAA intake as a percent of total kilocalories and cancer mortality stratified by age group (50-66 and 66 and older). The effect of replacing kilocalories from BCAA with carbohydrates and fat was estimated by examining the continuous multivariable-adjusted association between BCAA intake and cancer mortality while simultaneously including these macronutrients in the model. Effects of interchanging different macronutrients were estimated by computing the differences between linear coefficients and their corresponding covariance matrix to obtain HRs and 95% CIs. It was not possible to estimate the effect of replacing BCAA with non-BCAA protein due to multicollinearity (r=0.975), but in sensitivity analyses where non-BCAA protein intake was modeled as the primary exposure variable, associations with cancer mortality were attenuated. All analyses were conducted with SAS 9.4 software (SAS Institute).

### Result and Discussion

In an attempt to identify novel targetable pathways in oncogenesis, differential expression analysis was performed on RNA-seq data published by The Cancer Genome Atlas (TCGA). First, the transcriptomic profiles of human liver, stomach, colorectal, and esophageal cancers were compared to their respective normal tissues, as these gastrointestinal cancers are all among the top 6 leading causes of cancer-related death. Overall, relative to respective normal tissues, 1082 genes were significantly upregulated and 419 genes were significantly downregulated across all 4 cancers (Fig. 1a). KEGG pathway analysis of these 1501 genes identified processes quintessential to cancer such as cell cycle, DNA replication, mismatch repair, and homologous recombination. Surprisingly, it also identified the metabolic pathway of branched-chain amino acid (BCAA) catabolism (Fig. 1b). Similar results were obtained when analyzing the gene sets with Ingenuity Pathway Analysis (IPA; Fig. 5a). Approximately 40 enzymes are involved in BCAA catabolism, and the transcripts were broadly suppressed in tumors (Fig. 1c). Oncomine analysis confirmed that these expression changes were consistent across multiple validated cohorts (Fig. 5b). In addition, immunostained biopsies profiled by The Human Protein Atlas provided evidence that these enzymes (i.e. BCAA catabolic enzymes) were downregulated at the protein level in gastrointestinal tumors (Figs. 6a-c).

Surprisingly, transcript levels of the BCAA catabolic enzymes were not only suppressed in tumors when compared to adjacent normal tissue, but the degree of suppression was correlated with multiple indicators of disease progression and tumor aggressiveness, including stage, grade, vascular invasion, lymph node invasion, and distant metastasis (Fig. 1d and 5c). Moreover, enzyme expression levels were also strongly associated with clinical outcome, even when adjusting for patient and tumor characteristics (Fig. 7). Maintaining high expression of these enzymes, therefore allowing efficient catabolism of BCAAs, was associated with significantly better patient outcome. Surveying the prognostic utility of aggregating multiple (up to 5) genes identified the combined expression index of *BCKDHA, ACADS,* and *ACADSB* as the most robust predictor of patient survival (Fig. 1e). Of note, these enzymes are critically involved in the first two irreversible steps of BCAA catabolism. The bifurcation in patient survival was most dramatic in patients with high-grade tumors, in which patients with high enzyme expression maintained a survival rate above 85% throughout the study duration, while survival of patients with lower expression quickly dropped below 35% within 5 years (Figs. 5d).

Somatic copy number variation (CNV) loss of the BCAA catabolic enzymes affected an estimated 22% of tumors, depending on gene and cancer type, and led to the most potent decreases in mRNA expression (Fig. 8a, b). However, tumors with normal CNVs still displayed decreased gene expression relative to normal tissues, indicating additional regulatory mechanisms are likely involved. Analysis of the datasets failed to find any significant difference in promoter methylation of the BCAA catabolic enzymes, or associations with changes in microRNA expression (data not shown). In contrast, IPA upstream analysis highlighted the transcription factors PPARα, KLF15, HNF4α, and p53 as likely regulators of all significant, differentially expressed genes, and in particular, the BCAA catabolic enzymes (Fig. 8c-e). ENCODE data confirmed that these transcription factors broadly bind to the promoters the BCAA catabolic genes (Fig. 8f). Furthermore, a significant proportion of tumors had mutations in these transcription factors or CNV loss leading to decreased transcription factor expression (Fig. 8g-i). Tumors with transcription factor mutation or CNV loss also displayed lower levels of BCAA catabolic enzyme expression (Fig. 1f and 8j). In line with primary human samples, numerous tumorigenic cell lines had significantly lower BCAA catabolic enzyme expression at both the RNA and protein level relative to the nontumorigenic HepG2 cell line (Fig. 1g,h). This was associated with mutation or changes in expression of the transcription factors identified by the IPA analyses (Fig. 1h).

Finally, the differential expression analysis was performed on all individual cancer subtypes profiled by TCGA with at least five adjacent normal samples for appropriate comparison. Overall, approximately 70% of cancers displayed significant suppression of at least half of the BCAA catabolic pathway (Fig. 5e). In fact, enzymes expression was rarely significantly upregulated, leading to a broad net suppression across the profiled cancers (Fig. 1i). All together, these data demonstrate that loss of BCAA catabolic enzymes is observed in multiple cancer types, associated with CNV and transcription factor changes, and correlates with tumor aggressiveness and patient survival.

KEGG pathway analysis of differentially expressed genes among individual cancers revealed that suppression of BCAA catabolic enzymes was particularly robust in liver cancers, ranking as the top pathway for both hepatocellular carcinoma (HCC) and cholangiocarcinoma (Fig. 1j). Therefore, to further explore the role of BCAA enzyme suppression in tumorigenesis, RNA-seq and metabolomic analyses were performed on tumor and nontumor samples collected from multiple *in vivo* liver cancer animal models. Tissues were harvested from animals administered diethylnitrosamine (DEN), as this compound generates endogenous tumors resembling poor-prognosis HCC by causing multiple stochastic DNA mutations in the liver. A syngenic orthotopic Morris Hepatoma 3924a model was also employed to represent aggressive, fast-growing tumors. KEGG analysis of all significant, differentially-expressed genes confirmed that BCAA catabolism ranked as the top pathway (Fig. 9a). Importantly, BCAA catabolism remained the most significant pathway even after omitting genes differentially expressed in proliferating hepatocytes of the regenerating liver (Fig. 2a-c and 9b-g). An initial non-targeted screen of over 200 metabolites identified five that had accumulated in liver tumors with high significance, and among these were all three BCAAs (Fig. 9h). Subsequent targeted analyses confirmed that the BCAAs and only three other amino acids (phenylalanine, methionine, and asparagine) had accumulated in both tumor models but not regenerating tissue (Fig. 2d). Finally, targeted analysis of acylcarnitines (the derivatives of fatty acids and some amino acids targeted for oxidation) identified C5:1 as the only one significantly different in all tumor models analyzed (Fig. 2e). This acylcarnitine derives from downstream metabolites of leucine and isoleucine catabolism, and its lower abundance in tumors is consistent with a reduced BCAA catabolic flux. Integration of transcriptomic and metabolomic data highlights that an overt consequence of the broad suppression of enzymes involved in BCAA catabolism is the accumulation of BCAAs in tumors.

To further explore changes in BCAA catabolism, the investigation was focused on putative rate-limiting steps. The accumulation of BCAAs and reduction of the C5:1 acylcarnitine indicated that the catabolic enzymes acting between these metabolites, namely, BCKDHA, ACADS, and ACADSB are critical. Indeed, the branched-chain ketoacid dehydrogenase (BCKD) complex is the first irreversible, and in many cases rate-limiting step in BCAA catabolism. Inhibition of the complex's activity is achieved through suppression of total protein levels, as well as phosphorylation of the BCKDHA subunit by the kinase BCKDK. While Morris Hepatoma tumors had dramatic reductions in total protein levels of all BCAA catabolic enzymes assayed, DEN-induced tumors displayed more overt changes in BCKDHA phosphorylation (Fig. 2f). Relative to normal (DEN-free) liver tissues, the phospho/total BCKDHA ratios were elevated in pre-tumor tissues, and further increased in tumors (Fig. 2g and 9i). *Ex vivo* BCKDH enzyme activity assays confirmed that regardless of the primary method of suppression, all tumor tissues tested had a dramatically reduced catabolic capacity (Figs. 3g and 9j). Importantly, phospho/total BCKDHA ratios, BCKDH activity, and BCKDK expression remained unchanged in regenerating tissues (Figs. 2g,h). Based on the concept of the *ex vivo* BCKDH assay, a hyperpolarized ¹³C magnetic resonance spectroscopy method to quantify enzyme activity *in vivo* was developed (Fig. 10a,b). Using this platform, a significantly reduced BCKDH activity in rats bearing DEN-induced tumors was detected (Fig. 2j), indicating that this method can be used to noninvasively monitor enzyme activity in live subjects. Taken together, these data demonstrate that BCAA catabolic enzyme activity is overtly suppressed in tumors, and suggest that the BCKDH complex is an important, rate-limiting step.

Next, to examine the potential functional consequences of BCAA accumulation, multiple strategies to manipulate the BCAA catabolic enzyme activity across normal liver and tumorigenic HCC cell lines were used. In order to recapitulate the loss of BCAA catabolic enzyme activity in tumors, a key subunit of the BCKDH complex in the immortalized hepatocyte cell line AML12 was targetted. Knocking down BCKDHA with inducible shRNAs led to a clear enhancement of proliferation rates as well as an increased cell density at confluency (Fig. 2i). Conversely, restoring BCAA catabolism by overexpressing BCKDHA, ACADS, or ACADSB inhibited proliferation of the HCC cell line Hep3B (Fig. 2j). Although a similar restoration of enzyme expression may be possible in clinical settings, given the relative difficulty of gene therapy, additional potential approaches were examined. BCKDK, the kinase that inhibits BCAA catabolism by hyperphosphorylating BCKDHA, was observed to be overexpressed in pre-tumor and tumor tissues of the animal models (Fig. 3a), as well as in human HCC relative to normal liver tissues (Fig. 3b). Moreover, CRISPR-Cas9-mediated knockout of BCKDK significantly reduced the growth of Hep3B cells (Fig. 3c and 10c), suggesting it can be targeted to effectively reactivate BCAA catabolism and cancer cell proliferation. A number of natural and synthetic compounds are reported to inhibit BCKDK. Thus, a panel of HCC cell lines was treated with two BDKDK inhibitors: BT2, a recently developed compound with high specificity, and Fenofibrate, a fibrate that has been safely used for decades for non-cancer-related indications. Accordingly, promoting BCAA catabolism through BT2 or Fenofibrate treatment led to a potent dose-dependent decrease in cell proliferation (Figs. 3d,e and 10d,e). Importantly, the growth suppressive effects were observed within the average serum concentrations of commonly prescribed doses of Fenofibrate.

While BCAAs are reported to have pleiotropic effects, the most potent and extensively-characterized is the stimulation of the mechanistic target of rapamycin (mTOR). Activation of the mTOR pathway potently enhances cell growth and tumorigenesis, including liver cancer in humans and rodent models. Thus, it was hypothesized that BCAA accumulation would influence cell proliferation, at least in part, by modulating mTOR activity. As expected, all HCC cell lines examined had active mTOR pathways under basal growth conditions, and were at least partially sensitive to the inhibitors rapamycin and Torin 1 (Figs. 10f-h). Mechanistically, mTOR integrates signals from growth factors and nutrient abundance, and removal of either input is sufficient to block its activity. While growth factors regulate mTOR activity through tuberous sclerosis complex (TSC)-Rheb interaction, nutrients control mTOR subcellular localization, with nutrient insufficiency causing dispersion of mTOR away from the lysosome. In line with previous analyses performed in 293T cells, the HCC cell lines displayed clusters of mTOR that colocalized with the lysosomal marker LAMP2 under standard growth conditions (Fig. 3f). Moreover, removal of all amino acids for 60 minutes led to a dispersion of mTOR, inhibiting its activity (Fig 3f,g). Importantly, enhancing BCAA catabolism through BT2 or Fenofibrate treatment led to a similar dispersion of mTOR, and broad loss of mTOR activity across the HCC cell lines (Fig. 3c, f-h and Fig.10i,j). Collectively, these data demonstrate that BCAA catabolic enzymes can regulate cancer cell proliferation and influence mTOR activity by modulating nutrient sufficiency signals.

Next, the hypothesis whether a BCAA-mediated stimulation of pro-tumorigenic pathways was also utilized by developing tumors *in vivo* was explored. As essential amino acids, BCAAs are derived exclusively from dietary sources. Therefore, DEN-injected mice were fed diets with normal or high levels of BCAAs (Fig. 11a). Given the significant overlap of BCAA and fatty acid catabolic enzymes, and influence of fatty acids on BCAA accumulation, the effects of diets with either standard low (LFD) or high (HFD) fat content were first explored. Five months after DEN injection, at a time when no tumors developed in mice fed a LFD, mice fed a diet with high fat and/or BCAAs had tumor incidence of 20-40% (Fig. 11b). In addition, the liver masses of DEN-injected mice fed BCAA-supplemented diets were elevated, while this was not observed in uninjected mice, indicating that BCAAs specifically influence precancerous tissues (Figs. 11c and 12a,b). By 8 months post-injection, diets supplemented with BCAAs led to a dramatic increase in tumor number and tumor size (Figs. 4a,b). The BCAAs were efficiently taken up by the liver and had significantly accumulated in both tumor and nontumor tissues at both time points, while this was not observed for other amino acids (Figs. 4c, 11d and 13). Interestingly, the BCAA content of nontumor liver tissue positively correlated with tumor multiplicity (Fig. 4d). BCAA supplementation did not enhance DNA damage, fibrosis, or cell death leading to compensatory proliferation (Figs. 11e, 12c, and 14c), established factors influencing liver tumorigenesis. Rather, in agreement with *in vitro* data, the mTOR pathway was hyperactivated in liver tissues of BCAA-supplemented groups (Figs. 4e,f and 11d). Normal liver tissues of uninjected mice responded to diets high in BCAAs or fat by enhancing the expression of BCAA catabolic enzymes (Fig. 12d). In contrast, BCAA catabolic enzymes were significantly suppressed in nontumor tissues of DEN-injected mice, and further suppressed in tumors, and largely failed to increase in response to high BCAAs or fat (Figs. 12d,e and 14d). Moreover, administration of the BCKDK inhibitors fenofibrate or BT2 at low doses was able to reverse the tumor promoting effects of BCAA-supplemented diets (Fig. 4g,h).

In a follow-up cohort, the hypothesis whether restricting BCAAs in the diet by 50% could limit tumor burden was also investigated (Fig. 14a). All DEN-injected mice fed a low-BCAA diet survived the study duration, while this was true for only 70% of mice fed normal or supplemented levels of BCAAs (Fig. 4i). Moreover, at 12 months post-injection, mice fed low-BCAA diets had significantly smaller tumors and reduced liver BCAA content (Fig. 4j,k). Importantly, normal lean body mass and liver function was maintained (Figs. 14b-d). The effects of the high- and low-BCAA diets were not due to differences in protein intake, as the tumor burden of mice fed diets adjusted for total protein content were similar (Fig. 14e). Overall, these data show that BCAA accumulation is critical for the development and growth of liver tumors *in vivo,* and that dietary interventions influence tumor progression and overall survival.

Finally, to determine if dietary intake of BCAAs correlated with cancer mortality in a human population, the NHANES III dataset with linked mortality data was analyzed. Individuals 50-66 years old in the highest tertile of BCAA intake had a 200% increased risk of death from cancer relative to the lowest tertile, even when adjusting for known confounders, as well as percent kilocalories from fat, carbohydrate, and non-BCAA protein intake (Figs. 4m and 14f). When evaluated as continuous substitutive variables, isocalorically replacing 3% of energy from BCAAs with either carbohydrate or fat decreased cancer mortality risk by more than 50% (Fig. 4n). While the effect of substituting BCAAs with non-BCAA protein could not be accurately evaluated due to high collinearity between the two variables (r=0.975), non-BCAA protein intake was associated with only a modest increase in cancer mortality risk (Fig. 14g). Of note, these associations did not hold true for more elderly populations that have higher basal protein requirements (Fig. 14h-j), and additional factors, such as the microbiome, are known to influence systemic BCAA levels.

In summary, comprehensive transcriptomic and metabolomic analyses of human cancers and animal tumors and regenerating tissues identified a mechanism specifically utilized by cancer cells to enhance the accumulation of BCAAs (Fig. 4o).

### Tables

**Table 1: List of PCR primers used**

| **Primer sequences used for Mouse Sample** | | |
|---|---|---|
| **Target** | **Sequence** | |
| ABAT | 5'-GAGGCCGTGCACTTTTTCTG-3' | SEQ ID NO: 1 |
| | 5'-CCAGAGCCGGATGGTTGTAA-3' | SEQ ID NO: 2 |
| ACAA1 | 5'-ACAGTGTTCATCGGGACTGC-3' | SEQ ID NO: 3 |
| | 5'-AGATATTCCCAGGGTTCCCCA-3' | SEQ ID NO: 4 |
| ACAA2 | 5'-ACATAACTTCACGCCCCTGG-3' | SEQ ID NO: 5 |
| | 5'-GAGGGGCAAAAGCTTCGTTC-3' | SEQ ID NO: 6 |
| ACADS | 5'-TTGCCGAGAAGGAGTTGGTC-3' | SEQ ID NO: 7 |
| | 5'-AGGTAATCCAAGCCTGCACC-3' | SEQ ID NO: 8 |
| ACADSB | 5'-GGACTGGCCCAAGGATGTTT-3' | SEQ ID NO: 9 |
| | 5'-CGAGCCTAGCAGCGTTGTAT-3' | SEQ ID NO: 10 |
| ALDH6A1 | 5'-GCGTGGGCAGACACATCTAT-3' | SEQ ID NO: 11 |
| | 5'-CTCCCAGCATGAGGGATGTC-3' | SEQ ID NO: 12 |
| AOX1 | 5'-TACGTGAATGGCCAGAAGGT-3' | SEQ ID NO: 13 |
| | 5'-GGATGATGCCTGATCGCCTT-3' | SEQ ID NO: 14 |
| BCKDHA | 5'-GACCTGGTGTTTGGCCAGTA-3' | SEQ ID NO: 15 |
| | 5'-GCCGTAGTGAACAGGCATCT-3' | SEQ ID NO: 16 |
| ECHS1 | 5'-CTCTTGGTGGGGGTTGTGAA-3' | SEQ ID NO: 17 |
| | 5'-TTGCTAGCGATTTGCCGACT-3' | SEQ ID NO: 18 |
| HIBCH | 5'-AGGCGTCATAACGCTCAAC-3' | SEQ ID NO: 19 |
| | 5'-TCCTCCGGCTCCCTTTATGA-3' | SEQ ID NO: 20 |
| HMGCS2 | 5'-GCCCAAACGTCTAGACTCCC-3' | SEQ ID NO: 21 |
| | 5'-CTCCATTAGACGGGACACCG-3' | SEQ ID NO: 22 |
| MCCC1 | 5'-TGGGGTAGCCCGTAAATCCA-3' | SEQ ID NO: 23 |
| | 5'-GAGCTCCTTCTGCACTCACT-3' | SEQ ID NO: 24 |
| β-ACTIN | 5'-CAAGGTCATCCATGACAACTTTG-3' | SEQ ID NO: 25 |
| | 5'-GGCCATCCACAGTCTTCTGG-3' | SEQ ID NO: 26 |
| GAPDH | 5'-CAAGGTCATCCATGACAACTTTG-3' | SEQ ID NO: 27 |
| | 5'-GGCCATCCACAGTCTTCTGG-3' | SEQ ID NO: 28 |
| | | |

| **Primer sequences used for Rat Sample** | | |
|---|---|---|
| **Target** | **Sequence** | |
| ABAT | 5'-GAGGCCGTGCACTTTTTCTG-3' | SEQ ID NO: 29 |
| | 5'-CGCGTTTTGAGGCTGTTGAA-3' | SEQ ID NO: 30 |
| ACAA1 | 5'-TTACGACATTGGCATGGCCT-3' | SEQ ID NO: 31 |
| | 5'-CAGCCACATTCTCCGAGGTT-3' | SEQ ID NO: 32 |
| ACAA2 | 5'-GGCAAAGTTCCACCGGAAAC-3' | SEQ ID NO: 33 |
| | 5'-ACTGGAAACCAGAGCCACAG-3' | SEQ ID NO: 34 |
| ACADS | 5'-AGCCTTTCACCAAGGAGTCG-3' | SEQ ID NO: 35 |
| | 5'-CATCTCGGTAGTAGCGCTCG-3' | SEQ ID NO: 36 |
| ACADSB | 5'-GGACTGGCCCAAGGATGTTT-3' | SEQ ID NO: 37 |
| | 5'-ATAAATGGCCTCCCGGCTTC-3' | SEQ ID NO: 38 |
| ALDH6A1 | 5'-AGTACCTGGAGCAACCATGC-3' | SEQ ID NO: 39 |
| | 5'-CGAAGATGTACTCTCCCGCC-3' | SEQ ID NO: 40 |
| AOX1 | 5'-ACCGTACCTGAGGAAGAACCT-3' | SEQ ID NO: 41 |
| | 5'-TGCCCTCTACTGTGGTGACT-3' | SEQ ID NO: 42 |
| BCKDHA | 5'-CAACGATGTGTTTGCGGTGT-3' | SEQ ID NO: 43 |
| | 5'-TTGACCTCATCCACCGAACG-3' | SEQ ID NO: 44 |
| ECHS1 | 5'-AGGCCATCCAATGTGCAGAA-3' | SEQ ID NO: 45 |
| | 5'-TCCACAAAGGCAGACATCCC-3' | SEQ ID NO: 46 |
| HIBCH | 5'-TGGAACAGATTAAGTTCTCATTGAC-3' | SEQ ID NO: 47 |
| | 5'-TATGGTGTGCTGACCCTTGC-3' | SEQ ID NO: 48 |
| HMGCS2 | 5'-GCCCAAACGTCTAGACTCCC-3' | SEQ ID NO: 49 |
| | 5'-CGGGCATATTTTCTGCGGTG-3' | SEQ ID NO: 50 |
| MCCC1 | 5'-AAGGCATGTGGAAGTCCAGG-3' | SEQ ID NO: 51 |
| | 5'-CAGGATCAATACCAGGCGCT-3' | SEQ ID NO: 52 |
| β-ACTIN | 5'-CAAGGTCATCCATGACAACTTTG-3' | SEQ ID NO: 53 |
| | 5'-GGCCATCCACAGTCTTCTGA-3' | SEQ ID NO: 54 |
| GAPDH | 5'-CAAGGTCATCCATGACAACTTTG-3' | SEQ ID NO: 55 |
| | 5'-GGCCATCCACAGTCTTCTGA-3' | SEQ ID NO: 56 |

## Claims

1. A branched-chain amino acid catabolism enhancer and/or a branched-chain α-ketoacid dehydrogenase complex (BCKDC) kinase inhibitor (BDK inhibitor); or a meal replacement comprising low level of branched-chain amino acid (BCAA) for use in treating or preventing a proliferative disease in a subject in need thereof, wherein the branched-chain amino acid catabolism enhancer is a peroxisome proliferator-activated receptor-alpha (PPARa) agonist, wherein the PPARa agonist is at least one selected from the group consisting of propan-2-yl 2-[4-(4-chlorobenzoyl)phenoxy]-2-methylpropanoate (fenofibrate), 2-[4-[2-[(4-chlorobenzoyl)amino]ethyl]phenoxy]-2-methylpropanoic acid (bezafibrate), and 2-[4-[2-[4-cyclohexylbutyl(cyclohexylcarbamoyl)amino]ethyl]phenyl]sulfanyl-2-methylpropanoic acid (GW7647), wherein the BDK inhibitor is at least one selected from the group consisting of peroxisome proliferator-activated receptor-alpha (PPARa) agonist (such as propan-2-yl 2-[4-(4-chlorobenzoyl)phenoxy]-2-methylpropanoate (fenofibrate), 2-[4-[2-[(4-chlorobenzoyl)amino]ethyl]phenoxy]-2-methylpropanoic acid (bezafibrate)), and 3,6-dichloro-1-benzothiophene-2-carboxylate (BT2), and wherein the proliferative disease is characterized and/or diagnosed by at least one selected from the group consisting of:
a suppression of transcripts-level of enzymes involved in the catabolism of at least one branched-chain amino acid (BCAA) when compared to a healthy control subject or group of control subjects; and
a decrease in a level of an acylcarnitine (C5:1) when compared to a healthy control subject or group of control subjects;
wherein the enzymes involved in the catabolism of at least one branched-chain amino acid (BCAA) are selected from the group consisting of branched chain keto acid dehydrogenase E1 alpha polypeptide (BCKDHA), acyl-CoA dehydrogenase C-2 to C-3 short chain (ACADS), and acyl-CoA dehydrogenase short/branched chain (ACADSB),
wherein the branched-chain amino acids are leucine, isoleucine, and valine; and
wherein the proliferative disease is selected from the group consisting of liver cancer, head and neck squamous cell carcinoma, kidney cancer, colon and rectum adenocarcinoma, breast carcinoma, lung carcinoma, thyroid carcinoma, stomach adenocarcinoma, and esophageal carcinoma.

2. The branched-chain amino acid catabolism enhancer and/or the branched-chain α-ketoacid dehydrogenase complex (BCKDC) kinase inhibitor (BDK inhibitor) or meal replacement for use of claim 1, wherein the accumulation of at least one branched-chain amino acid when compared to a healthy control subject or group of control subjects is observed in one, or two, or three branched-chain amino acids level.

3. The branched-chain amino acid catabolism enhancer and/or the branched-chain α-ketoacid dehydrogenase complex (BCKDC) kinase inhibitor (BDK inhibitor) or meal replacement for use of claim 1, wherein the proliferative disease further comprises the characteristics of an accumulation of at least one amino acid when compared to a healthy control subject or group of control subjects selected from the group consisting of phenylalanine, methionine and asparagine.

4. The branched-chain amino acid catabolism enhancer and/or the branched-chain α-ketoacid dehydrogenase complex (BCKDC) kinase inhibitor (BDK inhibitor) or meal replacement for use of any one of claims 1 to 3, wherein the method further comprises administering a branched-chain amino acid catabolism enhancer and/or a branched-chain α-ketoacid dehydrogenase complex (BCKDC) kinase inhibitor (BDK inhibitor), wherein the branched-chain amino acid catabolism enhancer is a peroxisome proliferator-activated receptor-alpha (PPARa) agonist, wherein the PPARa agonist is at least one selected from the group consisting of propan-2-yl 2-[4-(4-chlorobenzoyl)phenoxy]-2-methylpropanoate (fenofibrate), 2-[4-[2-[(4-chlorobenzoyl)amino]ethyl]phenoxy]-2-methylpropanoic acid (bezafibrate), and 2-[4-[2-[4-cyclohexylbutyl(cyclohexylcarbamoyl)amino]ethyl]phenyl]sulfanyl-2-methylpropanoic acid (GW7647), wherein the BDK inhibitor is at least one selected from the group consisting of peroxisome proliferator-activated receptor-alpha (PPARa) agonist (such as propan-2-yl 2-[4-(4-chlorobenzoyl)phenoxy]-2-methylpropanoate (fenofibrate), 2-[4-[2-[(4-chlorobenzoyl)amino]ethyl]phenoxy]-2-methylpropanoic acid (bezafibrate)), and 3,6-dichloro-1-benzothiophene-2-carboxylate (BT2).

## Patentansprüche

1. Verzweigtkettiger Aminosäurekatabolismus-Enhancer und/oder verzweigtkettiger α-Ketosäure-Dehydrogenase-Komplex-(BCKDC)-Kinase-Inhibitor (BDK-Inhibitor); oder Mahlzeitenersatz, umfassend einen niedrigen Spiegel an verzweigtkettiger Aminosäure (BCAA), zur Verwendung bei der Behandlung oder Prävention einer proliferativen Krankheit bei einem Probanden, der dieser bedarf, wobei der verzweigtkettige Aminosäurekatabolismus-Enhancer ein Peroxisom-Proliferator-aktivierter Rezeptor-Alpha(PPARa)-Agonist ist, wobei es sich bei dem PPARa-Agonisten um mindestens eines bzw. eine handelt, das bzw. die aus der Gruppe bestehend aus 2-[4-(4-Chlorbenzoyl)phenoxy]-2-methylpropansäurepropan-2-ylester (Fenofibrat), 2-[4-[2-[(4-Chlorbenzoyl)amino]ethyl]phenoxy]-2-methylpropansäure (Bezafibrat) und 2-[4-[2-[4-Cyclohexylbutyl(cyclohexylcarbamoyl)amino]ethyl]phenyl] sulfanyl-2-methylpropansäure (GW7647) ausgewählt ist, wobei es sich bei dem BDK-Inhibitor um mindestens einen handelt, der aus der Gruppe bestehend aus einem Peroxisom-Proliferator-aktivierten Rezeptor-Alpha(PPARa)-Agonisten (wie 2-[4-(4-Chlorbenzoyl)phenoxy]-2-methylpropansäurepropan-2-ylester (Fenofibrat), 2-[4-[2-[(4-Chlorbenzoyl)amino]ethyl]phenoxy]-2-methylpropansäure (Bezafibrat)) und 3,6-Dichlor-1-benzothiophen-2-carboxylat (BT2) ausgewählt ist, und wobei die proliferative Krankheit durch mindestens eine gekennzeichnet und/oder diagnostiziert ist, die ausgewählt ist aus der Gruppe bestehend aus:
einer Suppression der Transkriptebene von Enzymen, die an dem Katabolismus von mindestens einer verzweigtkettigen Aminosäure (BCAA) beteiligt sind, im Vergleich zu einem gesunden Kontrollprobanden oder einer gesunden Gruppe von Kontrollprobanden; und
einer Verringerung eines Spiegels eines Acylcarnitins (C5:1) im Vergleich zu einem gesunden Kontrollprobanden oder einer gesunden Gruppe von Kontrollprobanden;
wobei die Enzyme, die an dem Katabolismus von mindestens einer verzweigtkettigen Aminosäure (BCAA) beteiligt sind, aus der Gruppe bestehend aus verzweigtkettigem Ketosäure-Dehydrogenase-E1-Alpha-Polypeptid (BCKDHA), C2- bis C3-kurzkettiger Acyl-CoA-Dehydrogenase (ACADS) und kurz-/verzweigtkettiger Acyl-CoA-Dehydrogenase (ACADSB) ausgewählt sind,
wobei die verzweigtkettigen Aminosäuren Leucin, Isoleucin und Valin sind; und
wobei die proliferative Krankheit aus der Gruppe bestehend aus Leberkrebs, Kopf-Hals-Plattenepithelkarzinom, Nierenkrebs, Dickdarm- und Mastdarmadenokarzinom, Brustkarzinom, Lungenkarzinom, Schilddrüsenkarzinom, Magenadenokarzinom und Speiseröhrenkarzinom ausgewählt ist.

2. Verzweigtkettiger Aminosäurekatabolismus-Enhancer und/oder verzweigtkettiger α-Ketosäure-Dehydrogenase-Komplex-(BCKDC)-Kinase-Inhibitor (BDK-Inhibitor) oder Mahlzeitenersatz zur Verwendung nach Anspruch 1, wobei die Akkumulation von mindestens einer verzweigtkettigen Aminosäure im Vergleich zu einem gesunden Kontrollprobanden oder einer gesunden Gruppe von Kontrollprobanden auf einer Ebene von einer oder zwei oder drei verzweigtkettigen Aminosäuren beobachtet wird.

3. Verzweigtkettiger Aminosäurekatabolismus-Enhancer und/oder verzweigtkettiger α-Ketosäure-Dehydrogenase-Komplex-(BCKDC)-Kinase-Inhibitor (BDK-Inhibitor) oder Mahlzeitenersatz zur Verwendung nach Anspruch 1, wobei die proliferative Krankheit weiterhin die Charakteristika einer Akkumulation von mindestens einer Aminosäure im Vergleich zu einem gesunden Kontrollprobanden oder einer gesunden Gruppe von Kontrollprobanden, die aus der Gruppe bestehend aus Phenylalanin, Methionin und Asparagin ausgewählt ist, umfasst.

4. Verzweigtkettiger Aminosäurekatabolismus-Enhancer und/oder verzweigtkettiger α-Ketosäure-Dehydrogenase-Komplex-(BCKDC)-Kinase-Inhibitor (BDK-Inhibitor) oder Mahlzeitenersatz zur Verwendung nach einem der Ansprüche 1 bis 3, wobei das Verfahren weiterhin ein Verabreichen eines verzweigtkettigen Aminosäurekatabolismus-Enhancers und/oder verzweigtkettigen α-Ketosäure-Dehydrogenase-Komplex-(BCKDC)-Kinase-Inhibitors (BDK-Inhibitors) umfasst, wobei der verzweigtkettige Aminosäurekatabolismus-Enhancer ein Peroxisom-Proliferator-aktivierter Rezeptor-Alpha(PPARa)-Agonist ist, wobei es sich bei dem PPARa-Agonisten um mindestens eines bzw. eine handelt, das bzw. die aus der Gruppe bestehend aus 2-[4-(4-Chlorbenzoyl)phenoxy]-2-methylpropansäurepropan-2-ylester (Fenofibrat), 2-[4-[2-[(4-Chlorbenzoyl)amino]ethyl]phenoxy]-2-methylpropansäure (Bezafibrat) und 2-[4-[2-[4-Cyclohexylbutyl(cyclohexylcarbamoyl)amino]ethyl]phenyl] sulfanyl-2-methylpropansäure (GW7647) ausgewählt ist, wobei es sich bei dem BDK-Inhibitor um mindestens einen handelt, der aus der Gruppe bestehend aus einem Peroxisom-Proliferator-aktivierten Rezeptor-Alpha (PPARa)-Agonisten (wie 2-[4-(4-Chlorbenzoyl)phenoxy]-2-methylpropansäurepropan-2-ylester (Fenofibrat), 2-[4-[2-[(4-Chlorbenzoyl)amino]ethyl]phenoxy]-2-methylpropansäure (Bezafibrat)) und 3,6-Dichlor-l-benzothiophen-2-carboxylat (BT2) ausgewählt ist.

## Revendications

1. Amplificateur du catabolisme d'acides aminés à chaîne ramifiée et/ou inhibiteur de kinase complexe de déshydrogénase d'alpha-cétoacides à chaîne ramifiée (BCKDC) (inhibiteur de BDK) ; ou substitut de repas comprenant un faible niveau d'acides aminés à chaîne ramifiée (BCAA), pour une utilisation dans le traitement ou la prévention d'une maladie proliférative chez un sujet en ayant besoin, dans lequel l'amplificateur du catabolisme d'acides aminés à chaîne ramifiée est un agoniste de récepteur alpha activé par les proliférateurs de peroxysomes (PPARa), dans lequel l'agoniste de PPARa est au moins l'un choisi dans le groupe constitué par le 2-[4-(4-chlorobenzoyl)phénoxy]-2-méthylpropanoate de propan-2-yle (fénofibrate), l'acide 2-[4-[2-[(4-chlorobenzoyl)amino]éthyl]phénoxy]-2-méthylpropanoïque (bézafibrate), et l'acide 2-[4-[2-[4-cyclohexylbutyl(cyclohexylcarbamoyl)amino]éthyl]-phényl]sulfanyl-2-méthylpropanoïque (GW7647), dans lequel l'inhibiteur de BDK est au moins l'un choisi dans le groupe constitué par un agoniste de récepteur alpha activé par les proliférateurs de peroxysomes (PPARa) (tel que le 2-[4-(4-chlorobenzoyl)phénoxy]-2-méthylpropanoate de propan-2-yle (fénofibrate), l'acide 2-[4-[2-[(4-chlorobenzoyl)amino]éthyl]phénoxy]-2-méthylpropanoïque (bézafibrate), et un 3,6-dichloro-1-benzothiophène-2-carboxylate (BT2), et dans lequel la maladie proliférative est **caractérisée** et/ou diagnostiquée par au moins l'un choisi dans le groupe constitué par :
la suppression de niveaux de transcription d'enzymes impliquées dans le catabolisme d'au moins un acide aminé à chaîne ramifiée (BCAA) en comparaison avec un sujet témoin en bonne santé ou un groupe de sujets témoins ; et
une diminution du niveau d'une acylcarnitine (C5:1) en comparaison avec un sujet témoin en bonne santé ou un groupe de sujets témoins ;
dans lequel les enzymes impliquées dans le catabolisme d'au moins un acide aminé à chaîne ramifiée (BCAA) sont choisies dans le groupe constitué par le peptide alpha de la déshydrogénase des cétoacides à chaîne ramifiée E1 (BCKDHA), une chaîne courte en C-2 à C-3 d'acyl-CoA déshydrogénase (ACADS), et une chaîne courte/ramifiée d'acyl-CoA déshydrogénase (ACADSB),
dans lequel les acides aminés à chaîne ramifiée sont la leucine, l'isoleucine et la valine ; et
dans lequel la maladie proliférative est choisie dans le groupe constitué par un cancer du foie, un carcinome à cellules squameuses de la tête et du cou, un cancer rénal, un adénocarcinome du côlon et du rectum, un carcinome mammaire, un carcinome pulmonaire, un carcinome de la thyroïde, un adénocarcinome de l'estomac, et un carcinome de l'œsophage.

2. Amplificateur du catabolisme d'acides aminés à chaîne ramifiée et/ou inhibiteur de kinase complexe de déshydrogénase d'alpha-cétoacides à chaîne ramifiée (BCKDC) (inhibiteur de BDK) ou substitut de repas pour une utilisation selon la revendication 1, dans lequel l'accumulation d'au moins un acide aminé à chaîne ramifiée en comparaison avec un sujet témoin en bonne santé ou un groupe de sujets témoins est observée au niveau d'un, ou de deux, ou de trois acides aminés à chaîne ramifiée.

3. Amplificateur du catabolisme d'acides aminés à chaîne ramifiée et/ou inhibiteur de kinase complexe de déshydrogénase d'alpha-cétoacides à chaîne ramifiée (BCKDC) (inhibiteur de BDK) ou substitut de repas pour une utilisation selon la revendication 1, dans lequel la maladie proliférative comprend en outre les caractéristiques d'une accumulation d'au moins un acide aminé, en comparaison avec un sujet témoin en bonne santé ou un groupe de sujets témoins, choisi dans le groupe constitué par la phénylalanine, la méthionine et l'asparagine.

4. Amplificateur du catabolisme d'acides aminés à chaîne ramifiée et/ou inhibiteur de kinase complexe de déshydrogénase d'alpha-cétoacides à chaîne ramifiée (BCKDC) (inhibiteur de BDK) ou substitut de repas pour une utilisation selon l'une quelconque des revendications 1 à 3, dans lequel la méthode comprend en outre l'administration d'un amplificateur du catabolisme d'acides aminés à chaîne ramifiée et/ou d'un inhibiteur de kinase complexe de déshydrogénase d'alpha-cétoacides à chaîne ramifiée (BCKDC) (inhibiteur de BDK), dans lequel l'amplificateur du catabolisme d'acides aminés à chaîne ramifiée est un agoniste de récepteur alpha activé par les proliférateurs de peroxysomes (PPARa), dans lequel l'agoniste de PPARa est au moins l'un choisi dans le groupe constitué par le 2-[4-(4-chlorobenzoyl)phénoxy]-2-méthylpropanoate de propan-2-yle (fénofibrate), l'acide 2-[4-[2-[(4-chlorobenzoyl)amino]éthyl]phénoxy]-2-méthylpropanoïque (bézafibrate), et l'acide 2-[4-[2-[4-cyclohexylbutyl(cyclohexylcarbamoyl)amino]éthyl]-phényl]sulfanyl-2-méthylpropanoïque (GW7647), dans lequel l'inhibiteur de BDK est au moins l'un choisi dans le groupe constitué par un agoniste de récepteur alpha activé par les proliférateurs de peroxysomes (PPARa) (tel que le 2-[4-(4-chlorobenzoyl)phénoxy]-2-méthylpropanoate de propan-2-yle (fénofibrate), l'acide 2-[4-[2-[(4-chlorobenzoyl)amino]éthyl]phénoxy]-2-méthylpropanoïque (bézafibrate), et un 3,6-dichloro-1-benzothiophène-2-carboxylate (BT2).
